(19) Europäisches Patentamt
European Patent Office
Office européen des brevets

(11) EP 3 494 999 A1

(12) **EUROPEAN PATENT APPLICATION**

(43) Date of publication:
**12.06.2019 Bulletin 2019/24**

(51) Int Cl.:
***A61K 51/04*** *(2006.01)*

(21) Application number: **17206516.1**

(22) Date of filing: **11.12.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicant: **Technische Universität München
80333 München (DE)**

(72) Inventor: **The designation of the inventor has not yet been filed**

(74) Representative: **Vossius & Partner
Patentanwälte Rechtsanwälte mbB
Siebertstrasse 3
81675 München (DE)**

(54) **PSMA LIGANDS FOR IMAGING AND ENDORADIOTHERAPY**

(57) The present disclosure relates to imaging and endoradiotherapy of diseases involving prostate-specific membrane antigen (PSMA). Provided are compounds which bind or inhibit PSMA and furthermore carry at least one moiety which is amenable to radiolabeling. Provided are also medical uses of such compounds.

EP 3 494 999 A1

## Description

The present disclosure relates to imaging and endoradiotherapy of diseases involving prostate-specific membrane antigen (PSMA). Provided are compounds which bind or inhibit PSMA and furthermore carry at least one moiety which is amenable to radiolabeling. Provided are also medical uses of such compounds.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Prostate Cancer (PCa) remained over the last decades the most common malignant disease in men with high incidence for poor survival rates. Due to its overexpression in prostate cancer (Silver, D.A., et al., Prostate-specific membrane antigen expression in normal and malignant human tissues. Clinical Cancer Research, 1997. 3(1): p. 81-85), prostate-specific membrane antigen (PSMA) or glutamate carboxypeptidase II (GCP II) proved its eligibility as excellent target for the development of highly sensitive radiolabeled agents for endoradiotherapy and imaging of PCa (Afshar-Oromieh, A., et al., The diagnostic value of PET/CT imaging with the 68Ga-labelled PSMA ligand HBED-CC in the diagnosis of recurrent prostate cancer. European journal of nuclear medicine and molecular imaging, 2015. 42(2): p. 197-209; Benešová, M., et al., Preclinical Evaluation of a Tailor-Made DOTA-Conjugated PSMA Inhibitor with Optimized Linker Moiety for Imaging and Endoradiotherapy of Prostate Cancer. Journal of Nuclear Medicine, 2015. 56(6): p. 914-920; Robu, S., et al., Preclinical evaluation and first patient application of 99mTc-PSMA-I&S for SPECT imaging and radioguided surgery in prostate cancer. Journal of Nuclear Medicine, 2016: p. jnumed. 116.178939; Weineisen, M., et al., Development and first in human evaluation of PSMA I&T-A ligand for diagnostic imaging and endoradiotherapy of prostate cancer. Journal of Nuclear Medicine, 2014. 55(supplement 1): p. 1083-1083; Rowe, S., et al., PET imaging of prostate-specific membrane antigen in prostate cancer: current state of the art and future challenges. Prostate cancer and prostatic diseases, 2016; Maurer, T., et al., Current use of PSMA-PET in prostate cancer management. Nature Reviews Urology, 2016). Prostate-specific membrane antigen is an extracellular hydrolase whose catalytic center comprises two zinc(II) ions with a bridging hydroxido ligand. It is highly upregulated in metastatic and hormone-refractory prostate carcinomas, but its physiologic expression has also been reported in kidneys, salivary glands, small intestine, brain and, to a low extent, also in healthy prostate tissue. In the intestine, PSMA facilitates absorption of folate by conversion of pteroylpoly-γ-glutamate to the pteroylglutamate (folate). In the brain, it hydrolyses N-acetyl-Laspartyl- L-glutamate (NAAG) to N-acetyl-L-aspartate and glutamate. The enzymatic function of PSMA in normal and diseased prostate has yet not been clarified.

PSMA targeting molecules usually comprise a binding unit that encompasses a zinc-binding group (such as urea (Zhou, J., et al., NAAG peptidase inhibitors and their potential for diagnosis and therapy. Nature Reviews Drug Discovery, 2005. 4(12): p. 1015-1026), phosphinate or phosphoramidate) connected to a P1' glutamate moiety, which warrants high affinity and specificity to PSMA and is typically further connected to an effector functionality (Machulkin, A.E., et al., Small-molecule PSMA ligands. Current state, SAR and perspectives. Journal of drug targeting, 2016: p. 1-15). The effector part is more flexible and to some extent tolerant towards structural modifications. The entrance tunnel of PSMA accommodates two other prominent structural features, which are important for ligand binding. The first one is an arginine patch, a positively charged area at the wall of the entrance funnel and the structural explanation for the preference of negatively charged functionalities at the P1 position of PSMA. As a consequence thereof, art-established PSMA ligands present moieties bearing a negative charge in order to ensure a favorable interaction with the above mentioned arginine patch.

Upon binding the concerted repositioning of the arginine side chains can lead to the opening of an S1 hydrophobic accessory pocket, the second important structure, that has been shown to accommodate an iodo-benzyl group of several urea based inhibitors, thus contributing to their high affinity for PSMA (Barinka, C., et al., Interactions between Human Glutamate Carboxypeptidase // and Urea-Based Inhibitors: Structural Characterization. Journal of medicinal chemistry, 2008. 51(24): p. 7737-7743).

Zhang et al. discovered a remote binding site of PSMA, which can be employed for bidentate binding mode (Zhang, A.X., et al., A remote arene-binding site on prostate specific membrane antigen revealed by antibody-recruiting small molecules. Journal of the American Chemical Society, 2010. 132(36): p. 12711-12716). The so called arene-binding site is a simple structural motif shaped by the side chains of Arg463, Arg511 and Trp541, and is part of the PSMA entrance lid. The engagement of the arene-binding site by a distal inhibitor moiety can result in a substantial increase in the inhibitor affinity for PSMA due to avidity effects. PSMA I&T (see Figure 1) was developed with the intention to interact this way with PSMA, albeit no crystal structure analysis of binding mode is available. A necessary feature according to Zhang et al. is a linker unit (suberic acid in the case of PSMA I&T) which facilitates an open conformation of the entrance lid of PSMA and thereby enabling the accessibility of the arene-binding site. It was further shown that the structural composition of the linker has a significant impact on the tumor-targeting and biologic activity as well as on imaging contrast and pharmacokinetics (Liu, T., et al., Spacer length effects on in vitro imaging and surface accessibility

of fluorescent inhibitors of prostate specific membrane antigen. Bioorganic & medicinal chemistry letters, 2011. 21(23): p. 7013-7016), properties which are crucial for both high imaging quality and efficient targeted endoradiotherapy.

Two categories of PSMA targeting inhibitors are currently used in clinical settings. On the one side are tracer with chelating units for radionuclide complexation as PSMA I&T or related compounds (Kiess, A.P., et al., Prostate-specific membrane antigen as a target for cancer imaging and therapy. The quarterly journal of nuclear medicine and molecular imaging: official publication of the Italian Association of Nuclear Medicine (AIMN)[and] the International Association of Radiopharmacology (IAR),[and] Section of the Society of... 2015. 59(3): p. 241). On the other side are small molecules, comprising a targeting unit and effector molecules. Depending on the used radionuclide/halogen, the radiolabeled PSMA inhibitors may be used for imaging or endoradiotherapy. Among small molecule inhibitors with chelators for imaging, the most often used agents for selective PSMA imaging are PSMA HBED-CC (Eder, M., et al., 68Ga-complex lipophilicity and the targeting property of a urea-based PSMA inhibitor for PET imaging. Bioconjugate chemistry, 2012. 23(4): p. 688-697), PSMA-617 (Benešová, M., et al., Preclinical Evaluation of a Tailor-Made DOTA-Conjugated PSMA Inhibitor with Optimized Linker Moiety for Imaging and Endoradiotherapy of Prostate Cancer. Journal of Nuclear Medicine, 2015. 56(6): p. 914-920) and PSMA I&T (Weineisen, M., et al., Development and first in human evaluation of PSMA I&T-A ligand for diagnostic imaging and endoradiotherapy of prostate cancer. Journal of Nuclear Medicine, 2014. 55(supplement 1): p. 1083-1083). PSMA HBED-CC, or PSMA-11 was one the first PSMA inhibitors and is currently used for imaging since therapeutic applications are not possible with the chelator HBED-CC. However, due to the unique physical characteristics and the advantages of 18F for PET imaging, like the longer half-life, the low positron energy, which results in higher image resolution and the possibility for largescale production in a cyclotron, several groups have focused on the development of $^{18}$F-labeled urea-based Inhibitors for PCa imaging. The $^{18}$F-labeled urea-based PSMA inhibitor [$^{18}$F]DCFPyl demonstrated promising results in detection of primary and metastatic PCa (Rowe, S.P., et al., PSMA-Based [18F] DCFPyL PET/CT Is Superior to Conventional Imaging for Lesion Detection in Patients with Metastatic Prostate Cancer. Molecular Imaging and Biology, 2016: p. 1-9) and superiority to [$^{68}$Ga]PSMA-HBED-CC in a comparative study (Dietlein, M., et al., Comparison of [18F] DCFPyL and [68Ga] Ga-PSMA-HBED-CC for PSMA-PET imaging in patients with relapsed prostate cancer. Molecular Imaging and Biology, 2015. 17(4): p. 575-584).

PSMA DKFZ 617 (Benešová, M., et al., Preclinical Evaluation of a Tailor-Made DOTA-Conjugated PSMA Inhibitor with Optimized Linker Moiety for Imaging and Endoradiotherapy of Prostate Cancer. Journal of Nuclear Medicine, 2015. 56(6): p. 914-920, Becker, A., et al., Nephro-and hepatotoxicity after radioligand therapy of metastatic castrate-resistant prostate cancer with 177Lu-PSMA-617. Journal of Nuclear Medicine, 2016. 57(supplement 2): p. 1430-1430;Rahbar, K., et al., Response and tolerability of a single dose of 177Lu-PSMA-617 in patients with metastatic castration-resistant prostate cancer: a multicenter retrospective analysis. Journal of Nuclear Medicine, 2016: p. jnumed. 116.173757) and PSMA I&T (Weineisen, M., et al., Development and first in human evaluation of PSMA I&T-A ligand for diagnostic imaging and endoradiotherapy of prostate cancer. Journal of Nuclear Medicine, 2014. 55(supplement 1): p. 1083-1083, Eiber, M., et al., Systemic radioligand therapy with 177Lu-PSMA I&T in patients with metastatic castration-resistant prostate cancer. Journal of Nuclear Medicine, 2016. 57(supplement 2): p. 61-61; Schottelius, M., et al., [111In] PSMA-I&T: expanding the spectrum of PSMA-I&T applications towards SPECT and radioguided surgery. EJNMMI research, 2015. 5(1): p. 1) are applied in clinical settings for palliative treatment of prostate cancer patients. The chelating unit DOTA and the related DOTAGA, allow not only imaging but also therapeutical applications, since the scope for possible radiometal chelation encompasses $^{111}$In, $^{177}$Lu, $^{90}$Y and $^{213}$Bi amongst others. [$^{111}$In]PSMA I&T was already clinically implemented for radioguided surgery to assist the surgeon during excision of the malignant tissue (Schottelius, M., et al., [111In] PSMA-I&T: expanding the spectrum of PSMA-I&T applications towards SPECT and radioguided surgery. EJNMMI research, 2015. 5(1): p. 1). Likewise, the recent developed and clinically tested PSMA Inhibitor PSMA I&S (imaging and surgery) demonstrated highly encouraging results (Robu, S., et al., Preclinical evaluation and first patient application of 99mTc-PSMA-I&S for SPECT imaging and radioguided surgery in prostate cancer. Journal of Nuclear Medicine, 2016: p. jnumed. 116.178939).

Endoradiotherapeutic approaches with [$^{177}$Lu]PSMA I&T demonstrated efficiency, tolerability and high safety potential in patients receiving up to four cycles with 7.4 GBq. The obtained dosimetric values for organ radiation revealed, that especially the kidneys and the salivary glands receive the highest dose after tumor lesions. Similar radiation values were shown for PSMA DKFZ 617 and [$^{18}$F]DCFPyL (Rowe, S.P., et al., PSMA-Based [18F] DCFPyL PET/CT Is Superior to Conventional Imaging for Lesion Detection in Patients with Metastatic Prostate Cancer. Molecular Imaging and Biology, 2016: p. 1-9; Delker, A., et al., Dosimetry for 177Lu-DKFZ-PSMA-617: a new radiopharmaceutical for the treatment of metastatic prostate cancer. European journal of nuclear medicine and molecular imaging, 2016. 43(1): p. 42-51; Kabasakal, L., et al., Pre-therapeutic dosimetry of normal organs and tissues of 177Lu-PSMA-617 prostate-specific membrane antigen (PSMA) inhibitor in patients with castration-resistant prostate cancer. European journal of nuclear medicine and molecular imaging, 2015. 42(13): p. 1976-1983; Yadav, M.P., et al., 177Lu-DKFZ-PSMA-617 therapy in metastatic castration resistant prostate cancer: safety, efficacy, and quality of life assessment. European Journal of Nuclear Medicine and Molecular Imaging, 2016: p. 1-11). These elevated numbers are explainable by the physiologic expression of PSMA (Silver, D.A., et al., Prostate-specific membrane antigen expression in normal and malignant human tissues. Clinical

Cancer Research, 1997. 3(1): p. 81-85) and the renal excretion of the radiolabeled compound. The occasional occurring renal and hematological toxicities after administration are usually reversible, although is legitimate concern about chronic toxicity especially in patients with overall long survival rates, like in PCa patients. Therefore, a suitable concept is needed to reduce the unwanted radiation exposure.

A conceivable means for reducing the mentioned unwanted radiation exposure to kidneys include means to improve the renal clearance and/or reducing the renal retention. Having said that, there is currently no promising ligand with a specific modification that is able to reduce radiation exposure of the kidneys significantly.

In view of the above, the technical problem underlying the present invention can be seen in the provision of means and methods to improve internalization and/or clearance of PSMA targeted radiolabeled diagnostics and therapeutics. More generally, the technical problem can be seen in the provision of improved PSMA ligands.

This technical problem is solved by the subject-matter summarized in the attached claims and explained in further detail below.

In particular, the present invention provides in a first aspect a compound of formula (I), or a pharmaceutically acceptable salt thereof,

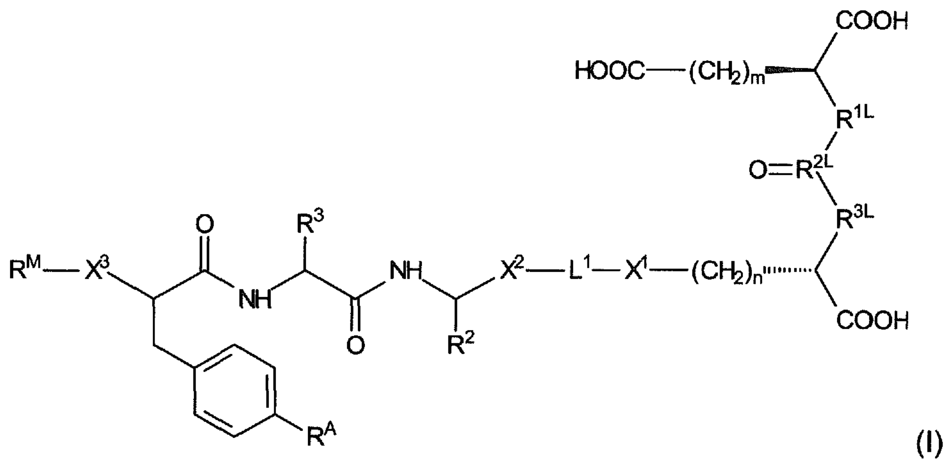

(I)

wherein:

- m is an integer of 2 to 6, preferably 2 to 4, more preferably 2;
- n is an integer of 2 to 6, preferably 2 to 4, more preferably 4;
- $R^{1L}$ is $CH_2$, NH or O, preferably NH;
- $R^{2L}$ is C or P(OH), preferably C;
- $R^{3L}$ is $CH_2$, NH or O, preferably NH;
- $X^1$ is selected from an amide bond, an ether bond, a thioether bond, an ester bond, a thioester bond, a urea bridge, an amine bond, and a group of the formula $-C(O)-NH-CH(R^1)-C(O)-NH-$, wherein $R^1$ is a group $-CH_2$-phenyl which is optionally substituted on its aromatic ring with a substituent selected from -OH and -I; and is preferably an amide bond;
- $L^1$ is a divalent linking group with a structure selected from an oligoamide, an oligoether, an oligothioether, an oligoester, an oligothioester, an oligourea, an oligo(ether-amide), an oligo(thioether-amide), an oligo(ester-amide), an oligo(thioester-amide), oligo(urea-amide), an oligo(ether-thioether), an oligo(ether-ester), an oligo(ether-thioester), an oligo ether-urea), an oligo(thioether-ester), an oligo(thioether-thioester), an oligo(thioether-urea), an oligo(ester-thioester), an oligo(ester-urea), and an oligo(thioester-urea), preferably with a structure selected from and oligoamide and an oligo(ester-amide);
- $X^2$ is selected from an amide bond, an ether bond, a thioether bond, an ester bond, a thioester bond, a urea bridge, and an amine bond, and is preferably an amide bond;
- $R^2$ is an aryl group or an aralkyl group, which aryl group or aralkyl group may be substituted on its aromatic ring with a substituent selected from halogen, preferably I, and -OH;
- $R^3$ is an aryl group or an aralkyl group, which aryl group or aralkyl group may be substituted on its aromatic ring with a substituent selected from halogen, preferably I, and -OH;
- $R^A$ is $-NH_2$;
- $X^3$ is selected from an amide bond, an ether bond, a thioether bond, an ester bond, a thioester bond, a urea bridge, an amine bond, and a group of the formula

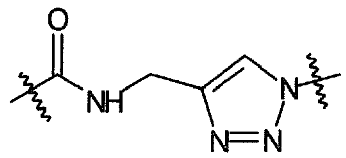

,

wherein the marked bond at the carbonyl group attaches $X^3$ to $R^M$ and the other marked bond attaches $X^3$ to the remainder of the compound of formula (I); and is preferably an amide bond;

$R^M$ is a marker group which comprises a chelating group optionally containing a chelated non-radioactive or radioactive cation.

As can be seen from the formula depicting the compound of the first aspect of the present invention, said compounds differ from PSMA I&T, which could be viewed as parent compound (displayed in Example 2), in that the peptidic portion is expanded. In particular, a basic amino acid, preferably 4-amino-phenylalanine is inserted, preferably in proximity to the chelating moiety.

The term "peptidic portion", in the context of the present invention, refers to the part of the compound which comprises $R^2$, $R^3$ and 4-amino-Phe, wherein 4-amino-Phe provides for the above-mentioned expansion of the peptidic portion.

As noted in the background section herein above, the structural features of the ligand binding sites of PSMA are known in the art. Owing to the presence of an arginine patch, there is an established preference for negatively charged groups on that part of PSMA ligands which is expected to end up in the neighborhood of said arginine patch upon binding of the urea type head group to the catalytic center of PSMA.

Yet, the present inventors introduced the unnatural amino acid 4-amino-phenylalanine in said part of the PSMA binding molecule.

It turns out that the compounds of the present invention, i.e. compounds with a p-substituted aromatic amino acid such as 4-amino-phenylalanine, exhibit similar and in several instances surprisingly improved affinity for PSMA as compared to the parent compound PSMA I&T.

Furthermore, and to some extent depending on the stereochemical configuration of the 4-amino-phenylalanine moiety, internalization rate, tumor uptake and clearance within the kidneys is significantly improved as compared to known PSMA binders such as PSMA I&T.

As noted above, salts of the compounds of the invention including compounds of formula (I) (and including their preferred embodiments) are also suitable for use in the context of the invention. It will be understood that these salts are generally pharmaceutically acceptable salt forms of these compounds which may be formed, e.g., by protonation of an atom carrying an electron lone pair which is susceptible to protonation, such as an amino group, with an inorganic or organic acid, or as a salt of a carboxylic acid group with a physiologically acceptable cation as they are well known in the art. Exemplary base addition salts comprise, for example, alkali metal salts such as sodium or potassium salts; alkaline-earth metal salts such as calcium or magnesium salts; ammonium salts; aliphatic amine salts such as trimethylamine, triethylamine, dicyclohexylamine, ethanolamine, diethanolamine, triethanolamine, procaine salts, meglumine salts, diethanol amine salts or ethylenediamine salts; aralkyl amine salts such as N,N-dibenzylethylenediamine salts, benetamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts; and basic amino acid salts such as arginine salts or lysine salts. Exemplary acid addition salts comprise, for example, mineral acid salts such as hydrochloride, hydrobromide, hydroiodide, sulfate salts, nitrate salts, phosphate salts (such as, e.g., phosphate, hydrogenphosphate, or dihydrogenphosphate salts), carbonate salts, hydrogencarbonate salts or perchlorate salts; organic acid salts such as acetate, propionate, butyrate, pentanoate, hexanoate, heptanoate, octanoate, cyclopentanepropionate, undecanoate, lactate, maleate, oxalate, fumarate, tartrate, malate, citrate, nicotinate, benzoate, salicylate or ascorbate salts; sulfonate salts such as methanesulfonate, ethanesulfonate, 2-hydroxyethanesulfonate, benzenesulfonate, p-toluenesulfonate (tosylate), 2-naphthalenesulfonate, 3-phenylsulfonate, or camphorsulfonate salts; and acidic amino acid salts such as aspartate or glutamate salts.

Further examples of pharmaceutically acceptable salts include, but are not limited to, acetate, adipate, alginate, ascorbate, aspartate, benzenesulfonate, benzoate, bicarbonate, bisulfate, bitartrate, borate, bromide, butyrate, calcium edetate, camphorate, camphorsulfonate, camsylate, carbonate, chloride, citrate, clavulanate, cyclopentanepropionate, digluconate, dihydrochloride, dodecylsulfate, edetate, edisylate, estolate, esylate, ethanesulfonate, formate, fumarate, gluceptate, glucoheptonate, gluconate, glutamate, glycerophosphate, glycolylarsanilate, hemisulfate, heptanoate, hexanoate, hexylresorcinate, hydrabamine, hydrobromide, hydrochloride, hydroiodide, 2-hydroxyethanesulfonate, hydroxynaphthoate, iodide, isothionate, lactate, lactobionate, laurate, lauryl sulfate, malate, maleate, malonate, mandelate,

mesylate, methanesulfonate, methylsulfate, mucate, 2-naphthalenesulfonate, napsylate, nicotinate, nitrate, N-methylglucamine ammonium salt, oleate, oxalate, pamoate (embonate), palmitate, pantothenate, pectinate, persulfate, 3-phenylpropionate, phosphate/diphosphate, picrate, pivalate, polygalacturonate, propionate, salicylate, stearate, sulfate, subacetate, succinate, tannate, tartrate, teoclate, tosylate, triethiodide, undecanoate, valerate, and the like (see, for example, S. M. Berge et al., "Pharmaceutical Salts", J. Pharm. Sci., 66, pp. 1-19 (1977)).

It is understood that throughout the present specification the term "compound" encompasses solvates, polymorphs, prodrugs, codrugs, cocrystals, tautomers, racemates, enantiomers, or diastereomers or mixtures thereof unless mentioned otherwise.

When the compounds of the present invention are provided in crystalline form, the structure can contain solvent molecules. The solvents are typically pharmaceutically acceptable solvents and include, among others, water (hydrates) or organic solvents. Examples of possible solvates include ethanolates and iso-propanolates.

The term "codrug" refers to two or more therapeutic compounds bonded via a covalent chemical bond. A detailed definition can be found, e.g., in N. Das et al., European Journal of Pharmaceutical Sciences, 41, 2010, 571-588.

The term "cocrystal" refers to a multiple component crystal in which all components are solid under ambient conditions when in their pure form. These components co-exist as a stoichiometric or non-stoichometric ratio of a target molecule or ion (i.e., compound of the present invention) and one or more neutral molecular cocrystal formers. A detailed discussion can be found, for example, in Ning Shan et al., Drug Discovery Today, 13(9/10), 2008, 440-446 and in D. J. Good et al., Cryst. Growth Des., 9(5), 2009, 2252-2264.

The compounds of the present invention can also be provided in the form of a prodrug, namely a compound which is metabolized in vivo to the active metabolite. Suitable prodrugs are, for instance, esters. Specific examples of suitable groups are given, among others, in US 2007/0072831 in paragraphs [0082] to [0118] under the headings prodrugs and protecting groups.

To the extent compounds of the invention exhibit a pH-dependent charged state, it is understood that all possible charged states are embraced. A preferred pH range in this regard is from 0 to 14.

To the extent a compound according to the invention bears a net charge, it is understood that the compound is provided in electroneutral form. This is achieved by one or more counterions, preferred counterions being defined in relation to the term "salt" herein above.

In formula (I), m is an integer of 2 to 6. Preferably, m is 2 to 4, more preferably 2. $R^{1L}$ is $CH_2$, NH or O, preferably NH. $R^{2L}$ is C or P(OH), preferably C. $R^{3L}$ is $CH_2$, NH or O, preferably NH. Thus, compounds of formula (I) or their salts are also preferred wherein m is 2, $R^{1L}$ is NH, $R^{2L}$ is C, and $R^{3L}$ is NH.

n is an integer of 2 to 6, preferably 2 to 4, more preferably 2 or 4.

Thus, compounds of formula (I) or their salts are particularly preferred wherein m is 2, n is 2 or 4, $R^{1L}$ is NH, $R^{2L}$ is C, and $R^{3L}$ is NH. More preferred are compounds of formula (I) or their salts wherein m is 2, n is 4, $R^{1L}$ is NH, $R^{2L}$ is C, and $R^{3L}$ is NH.

$X^1$ in formula (I) is selected from an amide bond (i.e. -C(O)-NH-), an ether bond (i.e. -O-), a thioether bond (i.e. -S-), an ester bond (i.e. -C(O)-O-, a thioester bond (i.e. -C(S)-O- or -C(O)-S-), a urea bridge (i.e. -NH-C(O)-NH-), an amine bond (i.e. -NH-), and a group of the formula -C(O)-NH-CH($R^1$)-C(O)-NH-. Preferred as $X^1$ is the amide bond and the group of the formula -C(O)-NH-CH($R^1$)-C(O)-NH-. More preferred as $X^1$ is the amide bond with the nitrogen atom of -C(O)-NH- being attached to the group $-(CH_2)_n-$ in formula (I), wherein n is 4. Also more preferred as $X^1$ is the amide bond with the carbon atom of -C(O)-NH- being attached to the group $-(CH_2)_n-$ in formula (I), wherein n is 2.

In the above group of the formula -C(O)-NH-CH($R^1$)-C(O)-NH- as $X^1$, $R^1$ is a group $-CH_2$-phenyl, which is optionally substituted on its aromatic ring, i.e. the phenyl ring, with a substituent selected from -OH and -I. It will be understood that this includes the possibility that one or more than one, e.g. two or three, substituent(s) selected from -OH and -I is (are) present. However, preferably the phenyl ring is non-substituted, or is substituted with a single substituent -OH, a single substituent -I, or the combination of one substituent -OH and one substituent -I.

$L^1$ in formula (I) is is a divalent linking group with a structure selected from an oligoamide, an oligoether, an oligothioether, an oligoester, an oligothioester, an oligourea, an oligo(ether-amide), an oligo(thioether-amide), an oligo(ester-amide), an oligo(thioester-amide), oligo(urea-amide), an oligo(ether-thioether), an oligo(ether-ester), an oligo(ether-thioester), an oligo ether-urea), an oligo(thioether-ester), an oligo(thioether-thioester), an oligo(thioether-urea), an oligo(ester-thioester), an oligo(ester-urea), and an oligo(thioester-urea), preferably with a structure selected from and oligoamide and an oligo(ester-amide).

The term "oligo" as used in the definition of $L^1$ in the terms oligoamide, oligoether, oligothioether, oligoester, oligothioester, oligourea, oligo(ether-amide), oligo(thioether-amide), oligo(ester-amide), oligo(thioester-amide), oligo(urea-amide), oligo(ether-thioether), oligo(ether-ester), oligo(ether-thioester), oligo (ether-urea), oligo(thioether-ester), oligo(thioether-thioester), oligo(thioether-urea), oligo(ester-thioester), oligo(ester-urea), and oligo(thioester-urea) is preferably to be understood as referring to a group wherein 2 to 20, more preferably wherein 2 to 10 subunits are linked by the type of bonds specified in the same terms. As will be understood by the skilled reader, where two different types of bonds are indicated in brackets, both types of bonds are contained in the concerned group (e.g. in "oligo (ester-amide)", ester bonds and amide bonds are contained).

It is preferred that $L^1$ is a divalent linking group with a structure selected from an oligo(ester-amide) which comprises a total of 2 to 5, more preferably a total of 2 to 3, and most preferably a total of 2 bonds selected from amide and ester bonds within its backbone, and an oligoamide which comprises a total of 1 to 5, more preferably a total of 1 to 3, and most preferably a total of 1 or 2 bonds selected from amide and ester bonds within its backbone. In a particularly preferred embodiment, $L^1$ represents a divalent linking group with an oligoamide structure which comprises one or two amide bonds within its backbone.

In formula (I), $X^2$ is selected from an amide bond, an ether bond, a thioether bond, an ester bond, a thioester bond, a urea bridge, and an amine bond, and is preferably an amide bond. It is more preferred that the nitrogen atom of the amide bond -C(O)-NH- is attached to $L^1$. Thus as will be understood from the above, it is generally preferred for formula (I) that $X^1$ is selected from an amide bond and a group of the formula -C(O)-NH-CH($R^1$)-C(O)-NH-, wherein $R^1$ is a group -$CH_2$-phenyl which is optionally substituted on its aromatic ring with a substituent selected from -OH and -I, more preferably wherein $R^1$ is an amide bond and most preferably an amide bond with the nitrogen atom of -C(O)-NH- being attached to the group -$(CH_2)_n$- in formula (I), and $X^2$ is an amide bond, more preferably an amide bond. It is more preferred that the nitrogen atom of the amide bond -C(O)-NH- is attached to $L^1$.

Thus, as will be understood from the above, compounds and salts of formula (I) are particularly preferred in the context of the present invention, wherein the moiety -$X^2$-$L^1$-$X^1$-has a structure selected from the formulae (L-1) to (L-3):

*-C(O)-NH-$R^4$-NH-C(O)-$R^5$-C(O)-NH- (L-1),

*-C(O)-NH-$R^6$-NH-C(O)-$R^7$-C(O)-NH-$R^8$-NH-C(O)- (L-2),

*-C(O)-NH-$R^9$-NH-C(O)-$R^{10}$-C(O)-NH-CH($R^1$)-C(O)-NH- (L-3);

wherein the bond marked with * in the formulae (L-1), (L-2) and (L-3), respectively, is attached to the carbon atom carrying $R^2$ in formula (I). $R^4$ to $R^{10}$ are independently selected from C2 to C10 alkanediyl, preferably linear C2 to C10 alkanediyl, which alkanediyl groups may each be substituted by one or more substitutents independently selected from -OH, -$OCH_3$, -COOH, -$COOCH_3$, -$NH_2$, and -NHC(NH)$NH_2$, preferably by one or more substituents selected from -COOH and -$NH_2$, and most preferably by one or more -COOH. It is also preferred that the alkanediyl groups are each unsubstituted, or that the number of substituents or preferred substituents is limited to one per alkendiyl group. $R^1$ is a group - $CH_2$-phenyl which is optionally substituted on its aromatic ring with a substituent selected from -OH and -I.

Moreover, it is preferred for $R^4$ to $R^{10}$ in the formulae (L-1), (L-2) and (L-3), that
the total number of carbon atoms in $R^4$ and $R^5$ is 6 to 20, more preferably 6 to 16, without carbon atoms contained in optional substituents,
the total number of carbon atoms in $R^6$, $R^7$ and $R^8$ is 6 to 20, more preferably 6 to 16, without carbon atoms contained in optional substituents, and
the total number of carbon atoms in $R^9$ and $R^{10}$ is 4 to 16, more preferably 4 to 12, without carbon atoms contained in optional substituents.

It is still more preferred that the moiety -$X^2$-$L^1$-$X^1$- in formula (I) has a structure selected from formulae (L-4) to (L-6):

*-C(O)-NH-CH(COOH)-$R^{11}$-NH-C(O)-$R^{12}$-C(O)-NH- (L-4),

*-C(O)-NH-CH(COOH)-$R^{13}$-NH-C(O)-$R^{14}$-C(O)-NH-$R^{15}$-CH(COOH)-NH-C(O)- (L-5),

*-C(O)-NH-CH(COOH)-$R^{16}$-NH-C(O)-$R^{17}$-C(O)-NH-CH($R^1$)-C(O)-NH- (L-6);

wherein the bond marked with * is attached to the carbon atom carrying $R^2$ in formula (I). $R^{11}$, $R^{13}$ and $R^{16}$ are independently selected from linear C2 to C6 alkanediyl, preferably a linear C3 to C6 alkanediyl. $R^{12}$ is a linear C2 to C10 alkanediyl, preferably a linear C4 to C8 alkanediyl. $R^{14}$, $R^{15}$ and $R^{17}$ are independently selected from linear C2 to C6 alkanediyl, and $R^1$ is a group -$CH_2$-phenyl which is optionally substituted on its aromatic ring with a substituent selected from -OH and -I.

For the structures selected from formulae (L-4) to (L-6), it is preferred that
the total number of carbon atoms in $R^{11}$ and $R^{12}$ is 6 to 16, more preferably 6 to 14,
the total number of carbon atoms in $R^{13}$, $R^{14}$ and $R^{15}$ is 6 to 16, more preferably 6 to 14, and
the total number of carbon atoms in $R^{16}$ and $R^{17}$ is 4 to 12, more preferably 4 to 10.

In formula (I), $R^2$ is an optionally substituted aryl group or an optionally substituted aralkyl group, preferably an optionally substituted aralkyl group. As will be understood, the term "aralkyl group" as used herein refers to an alkyl

group wherein a hydrogen atom is replaced by an aryl group as a substituent. Preferably, the aralkyl group is a group wherein one aryl group is bound to an alkanediyl group. The aryl group or aralkyl group represented by $R^2$ may be substituted on its aromatic ring with a substituent selected from halogen, preferably I, and -OH. The aryl and the aryl portion of the aralkyl group are preferably selected from phenyl and naphthyl, such as 2-naphthyl. The alkanediyl portion of the aralkyl group is preferably a C1-C4 alkanediyl group, more preferably a $-CH^2-$ group. Thus, $R^2$ is more preferably selected from optionally substituted $-CH_2$-phenyl and optionally substituted $-CH_2$-naphtyl, in particular $-CH_2$-(2-naphtyl). It is most preferably optionally substituted $-CH_2$-phenyl, and optionally substituted $-CH_2$-(2-naphtyl).

The optionally substituted aryl group and the aryl portion of the optionally substituted aralkyl group, including their preferred embodiments, may be substituted with a substituent selected from halogen, preferably I, and -OH. It will be also be understood that this includes the possibility that one or more than one, e.g. 2 or 3, substituent(s) selected from halogen, preferably I, and -OH is (are) present. However, it is preferred that $R^2$ is non-substituted.

Thus, the most preferred options for $R^2$ are non-substituted $-CH_2$-phenyl, and non-substituted $-CH_2$-(2-naphtyl).

In formula (I), $R^3$ is an optionally substituted aryl group or an optionally substituted aralkyl group, preferably an optionally substituted aralkyl group. The aryl group or aralkyl group may be substituted on its aromatic ring with a substituent selected from halogen, preferably I, and -OH. The aryl and the aryl portion of the aralkyl group are preferably selected from phenyl and naphthyl, such as 2-naphthyl. It is more preferred that the aryl and the aryl portion of the aralkyl are phenyl. The alkanediyl portion of the aralkyl group is preferably a C1-C4 alkanediyl group, more preferably a $-CH_2-$ group. Thus, $R^3$ is more preferably optionally substituted $-CH_2$-phenyl.

The optionally substituted aryl group and the aryl portion of the optionally substituted aralkyl group, including their preferred embodiments, may be substituted with a substituent selected from halogen, preferably I, and -OH. It will be also be understood that this includes the possibility that one or more than one, e.g. 2 or 3, substituent(s) selected from halogen, preferably I, and -OH is (are) present. Preferably, $R^3$ is substituted with a one substituent that is -OH, or with a combination of one substituent -OH and one substituent -I, and more preferably with a combination of one substituent -I and one substituent -OH. Thus, it is more preferred that $R^3$ is $-CH_2$-phenyl substituted with one substituent that is -OH, or with a combination of one substituent -OH and one substituent -I. Most preferably, the substituent -OH is present in the para-position of the phenyl ring relative to the $-CH_2-$ group.

It is particularly preferred that, in formula (I), $R^2$ is a group of the formula

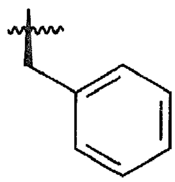

or

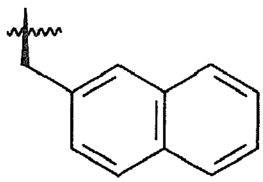

and $R^3$ is a group of the formula

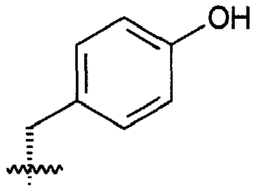

or

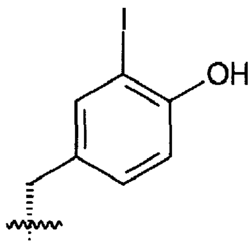

wherein 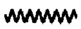 marks the bond which attaches $R^2$ and $R^3$, respectively, to the remainder of the compound of formula (I).

It is even further preferred that $R^2$ is a group of the formula

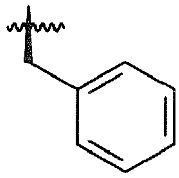

and $R^3$ is a group of the formula

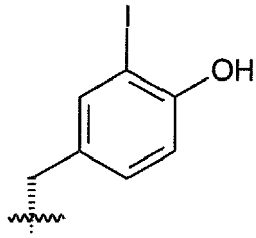

wherein 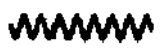 marks the bond which attaches $R^2$ and $R^3$, respectively, to the remainder of the compound, or that $R^2$ is a group of the formula

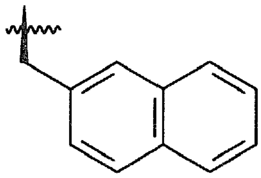

and $R^3$ is a group of the formula

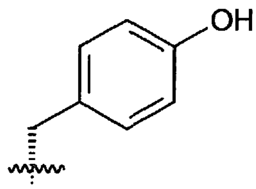

wherein 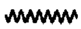 marks the bond which attaches $R^2$ and $R^3$, respectively, to the remainder of the compound.

$X^3$ is selected from an amide bond, an ether bond, a thioether bond, an ester bond, a thioester bond, a urea bridge, an amine bond, and a group of the formula

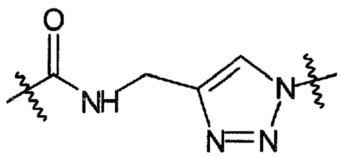

,

wherein the marked bond at the carbonyl group attaches $X^4$ to $R^M$ and the other marked bond attaches $X^3$ to the remainder of the compound.

Preferably, $X^3$ is selected from an amide bond, and a group of the formula

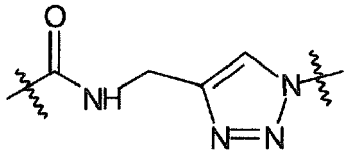

,

wherein the marked bond at the carbonyl group attaches $X^3$ to $R^M$ and the other marked bond attaches $X^3$ to the remainder of the molecule. In a more preferred embodiment, $X^3$ is an amide bond, even more preferred an amide bond -C(O)-NH- with the carbon atom attached to $R^M$.

$R^M$ is a marker group which comprises a chelating group optionally containing a chelated non-radioactive or radioactive cation.

As will be understood by the skilled reader, the above definition according to which $R^M$ comprises the chelating group encompasses the case that $R^M$ is a chelating group; in this case the chelating group group is typically directly bound to $X^3$,
and the case that $R^M$ comprises, together with the chelating group, e.g. a further linker moiety; in this case the chelating group can be indirectly bound via this further linker moiety to $X^3$.

The chelating group provided by $R^M$ is suitable to form a chelate with a radioactive or non-radioactive cation. Suitable chelating groups for diverse cations are well known in the art, and can be used in the context of the present invention.

The chelating group optionally containing a chelated non-radioactive or radioactive cation is preferably selected from a chelating group comprising at least one of

(i) a macrocyclic ring structure with 8 to 20 ring atoms of which 2 or more, preferably 3 or more, are selected from oxygen atoms, sulfur atoms and nitrogen atoms; and
(ii) an acyclic, open chain chelating structure with 8 to 20 main chain atoms of which 2 or more, preferably 3 or more are heteroatoms selected from oxygen atoms, sulfur atoms and nitrogen atoms.

An exemplary chelating group, and thus also an exemplary group $R^M$, is a residue of a chelating agent selected from bis(carboxymethyl)-1,4,8,11-tetraazabicyclo[6.6.2]hexadecane (CBTE2a), cyclohexyl-1,2-diaminetetraacetic acid (CDTA), 4-(1,4,8,11-tetraazacyclotetradec-1-yl)-methylbenzoic acid (CPTA), N'-[5-[acetyl(hydroxy)amino]pentyl]-N-[5-[[4-[5-aminopentyl-(hydroxy)amino]-4-oxobutanoyl]amino]pentyl]-N-hydroxybutandiamide (DFO), 4,11-bis(carboxymethyl)-1,4,8,11-tetraazabicyclo[6.6.2]hexadecan (DO2A) 1,4,7,10-tetraazacyclododecan-N,N',N",N'"-tetraacetic acid (DOTA), 2-[1,4,7,10-tetraazacyclododecane-4,7,10-triacetic acid]-pentanedioic acid (DOTAGA), N,N'-dipyridoxylethylendiamine-N,N'-diacetate-5,5'-bis(phosphat) (DPDP), diethylenetriaminepentaacetic acid (DTPA), ethylenediamine-N,N'-tetraacetic acid (EDTA), ethyleneglykol-O,O-bis(2-aminoethyl)-N,N,N',N'-tetraacetic acid (EGTA), N,N-bis(hydroxybenzyl)-ethylenediamine-N,N'-diacetic acid (HBED), hydroxyethyldiaminetriacetic acid (HEDTA), 1-(p-nitrobenzyl)-1,4,7,10-tetraazacyclodecan-4,7,10-triacetate (HP-DOA3), 6-hydrazinyl-N-methylpyridine-3-carboxamide (HYNIC), 1,4,7-triazacyclononan-1-succinic acid-4,7-diacetic acid (NODASA), 1-(1-carboxy-3-carboxypropyl)-4,7-(carbooxy)-1,4,7-triazacyclononane (NODAGA), 1,4,7-triazacyclononanetriacetic acid (NOTA), 4,11-bis(carboxymethyl)-1,4,8,11-tetraazabicyclo[6.6.2]hexadecane (TE2A), 1,4,8,11-tetraazacyclododecane-1,4,8,11-tetraacetic acid (TETA), terpyridin-bis(methyleneamintetraacetic acid (TMT), 1,4,7,10-tetraazacyclotridecan-N,N',N",N'"-tetraacetic acid (TRITA), triethylenetetraaminehexaacetic acid (TTHA), *N*,*N*'-bis[(6-carboxy-2-pyridyl)methyl]-4,13-diaza-18-crown-6 ($H_2$macropa), and 4-amino-4-{2-[(3-hydroxy-1,6-dimethyl-4-oxo-1,4-dihydro-pyridin-2-ylmethyl)-carbamoyl]-ethyl} heptanedioic acid bis-[(3-hydroxy-1,6-dimethyl-4-oxo-1,4-dihydro-pyridin- 2-ylmethyl)-amide] (THP); which residue is provided by covalently binding a carboxyl group contained in the chelating agent to the remainder of the compound via an ester or an amide bond, preferably an amide bond. It will be understood by the skilled reader that, in formula (I), this ester or amide bond can in this case be represented by or encompassed by $X^3$.

Among these chelating agents, DOTA and DOTAGA are preferred.

Thus, it is further preferred that $R^M$-$X^3$- is a group of the formula

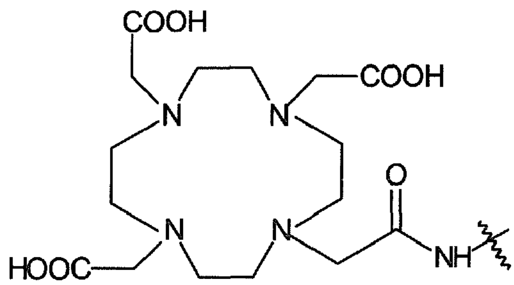

(M-1),

or

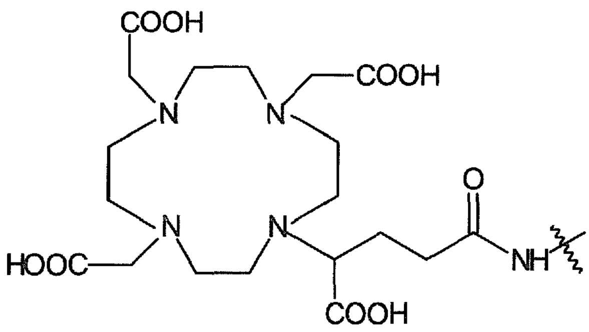

(M-2)

wherein the bond marked with  is attached to the remainder of the compound of formula (I), and wherein the chelating group may contain a chelated non-radioactive or radioactive cation.

Exemplary radioactive cations that are optionally chelated by the chelating group are selected from the cations of $^{44}Sc$, $^{47}Sc$, $^{51}Cr$, $^{52m}Mn$, $^{58}Co$, $^{52}Fe$, $^{56}Ni$, $^{57}Ni$, $^{62}Cu$, $^{64}Cu$, $^{67}Cu$, $^{66}Ga$, $^{68}Ga$, $^{67}Ga$, $^{89}Zr$, $^{90}Y$, $^{89}Y$, $^{94m}Tc$, $^{99m}Tc$, $^{97}Ru$, $^{105}Rh$, $^{109}Pd$, $^{111}Ag$,$^{110m}In$, $^{111}In$, $^{113m}In$, $^{114m}In$, $^{117m}Sn$, $^{121}Sn$, $^{127}Te$, $^{142}Pr$, $^{143}Pr$, $^{149}Pm$, $^{151}Pm$, $^{149}Tb$, $^{153}Sm$, $^{157}Gd$, $^{161}Tb$, $^{166}Ho$, $^{165}Dy$, $^{169}Er$, $^{169}Yb$, $^{175}Yb$, $^{172}Tm$, $^{177}Lu$, $^{186}Re$, $^{188}Re$, $^{191}pt$, $^{197}Hg$, $^{198}Au$, $^{199}Au$, $^{212}Pb$, $^{203}Pb$, $^{211}At$, $^{212}Bi$, $^{213}Bi$, $^{223}Ra$, $^{225}Ac$, and $^{227}Th$, or a cationic molecule comprising $^{18}F$, such as $^{18}F$-$[AlF]^{2+}$.

Preferred chelated cations are selected from the cations of $^{44}Sc$, $^{47}Sc$, $^{64}Cu$, $^{67}Cu$, $^{68}Ga$, $^{90}Y$, $^{111}In$, $^{161}Tb$, $^{166}Ho$, $^{177}Lu$, $^{188}Re$, $^{212}Pb$, $^{212}Bi$, $^{213}Bi$, $^{225}Ac$, and $^{227}Th$, or a cationic molecule comprising $^{18}F$.

Preferred in the context of the present invention are the compounds of the following formulae (Ia) or (Ib), or a pharmaceutically acceptable salt thereof:

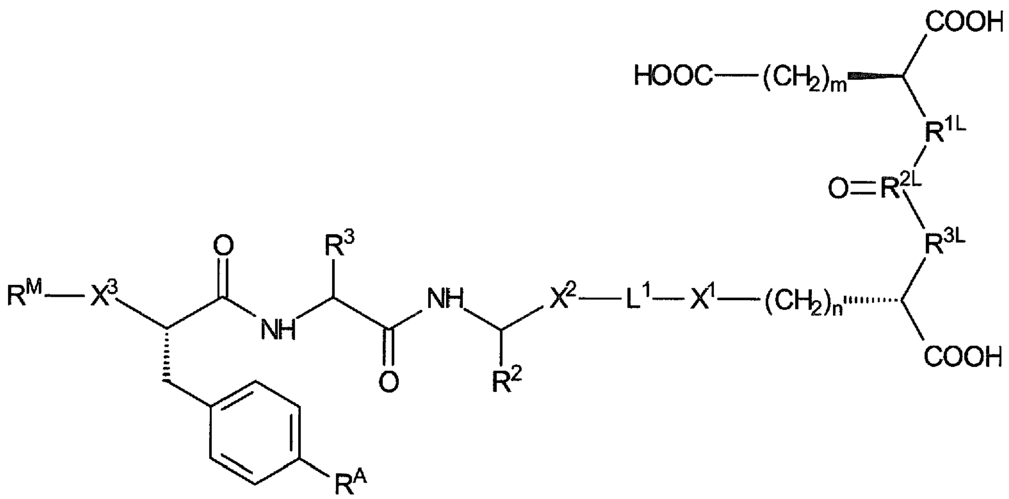

(Ia)

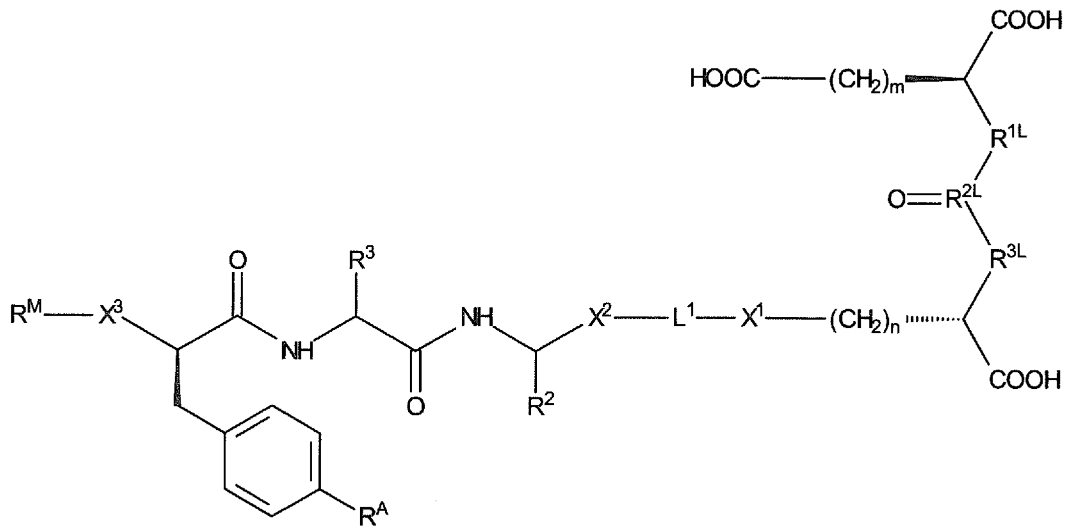

(Ib)

wherein $R^{1L}$, $R^{2L}$, $R^{3L}$, m, n, $X^1$, $L^1$, $X^2$, $R^2$, $R^3$, $R^A$, $X^3$ and $R^M$ are defined as are defined as above, including their preferred meanings.

In a further aspect, the present invention relates to a pharmaceutical or diagnostic composition comprising or consisting of one or more compounds or salts in accordance with the present invention.

In a further aspect, the present invention provides a compound or salt in accordance with the invention for use in a method of diagnosing and/or treating

(a) cancer including prostate cancer; or
(b) neoangiogenesis/angiogenesis.

Compounds in accordance with the invention and comprising D-4-amino-phenylalanine are characterized by accelerated kidney clearance. In terms of evidence, the inventors found accelerated kidney clearance of [$^{68}$Ga] PSMA-2 *in vivo* in SCID mice (for compound designation see the examples enclosed herewith).

Furthermore, a particularly high degree of human serum albumin binding has been found for compounds in accordance with this preferred embodiment. More specifically, the introduction of D-4-amino-phenylalanine into the PSMA ligand with the peptide scaffold: DOTAGA-(4-amino)f-y-nal-k(Glut-(I-f)-KuE resulted in the so far highest human serum albumin binding for this set of compounds (> 97%) and resulted in high tumor uptake after 24h in LNCaP xenograft bearing SCID mice.

Compounds of the invention comprising L-4-amino-phenylalanine are characterized by particularly increased affinity to PSMA and/or particularly increased internalization rate. This has been demonstrated for [$^{177}$Lu] PSMA-3 (for compound designations see the examples enclosed herewith). Increased tumor uptake by about 200% has been observed.

More specifically, the introduction of L-4-amino-phenylalanine into the PSMA ligand with the peptide scaffold: DOTAGA-(4-amino)F-y-nal-k-Sub-KuE (PSMA-6) resulted in the highest affinity so far measured in our laboratory and is around 7 fold higher than [Lu]PSMA I&T and more than 3 fold higher than [Lu]PSMA DKFZ 617.

Preferred embodiments include the compound PSMA-11.

In a further preferred embodiment, said compound has the formula (Ic) or (Id), or is a pharmaceutically acceptable salt thereof:

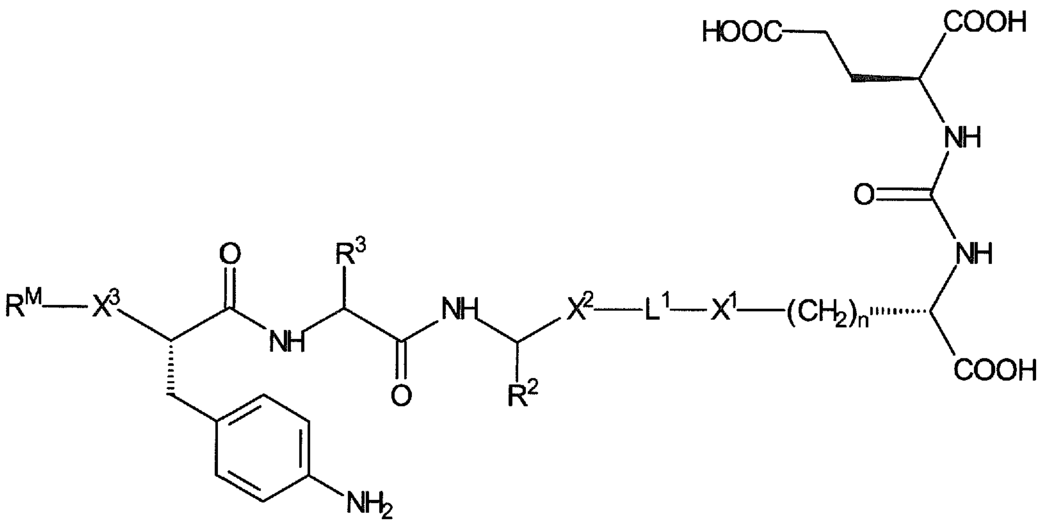

(Ic)

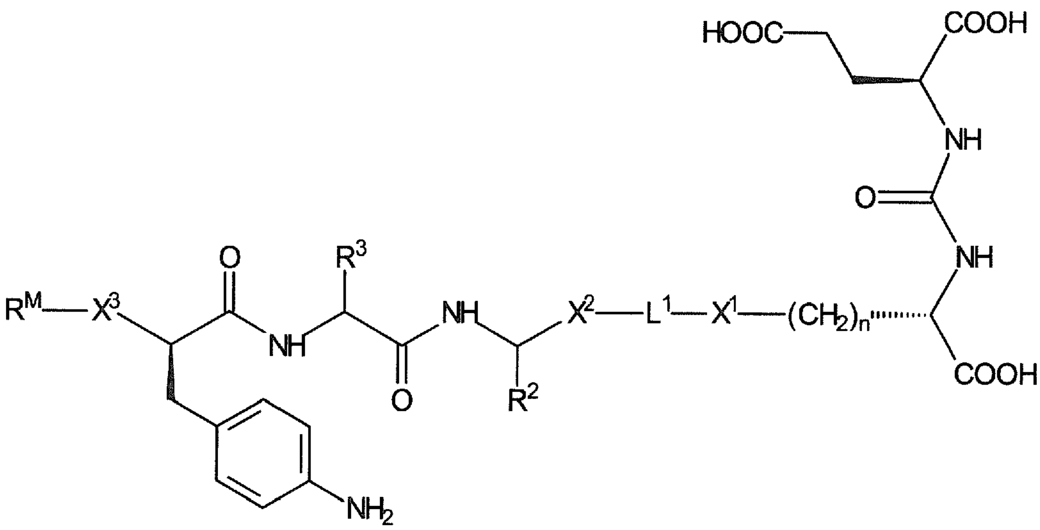

(Id)

wherein n, $X^1$, $L^1$, $X^2$, $R^2$, $R^3$, $X^3$ and $R^M$ are as defined above, including their preferred embodiments.

In a further preferred embodiment, said compound has the formula (Ie) or (If), or is a pharmaceutically acceptable salt thereof:

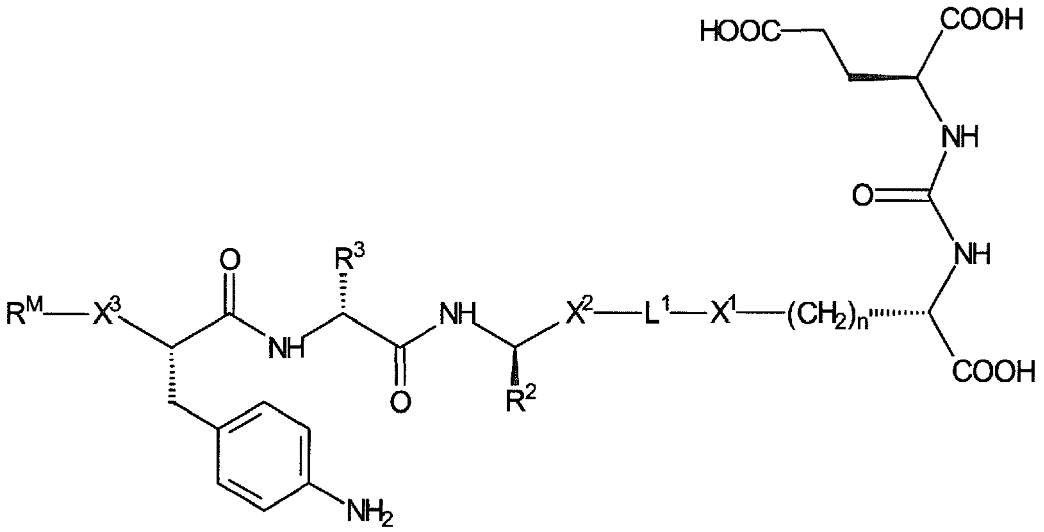

(Ie)

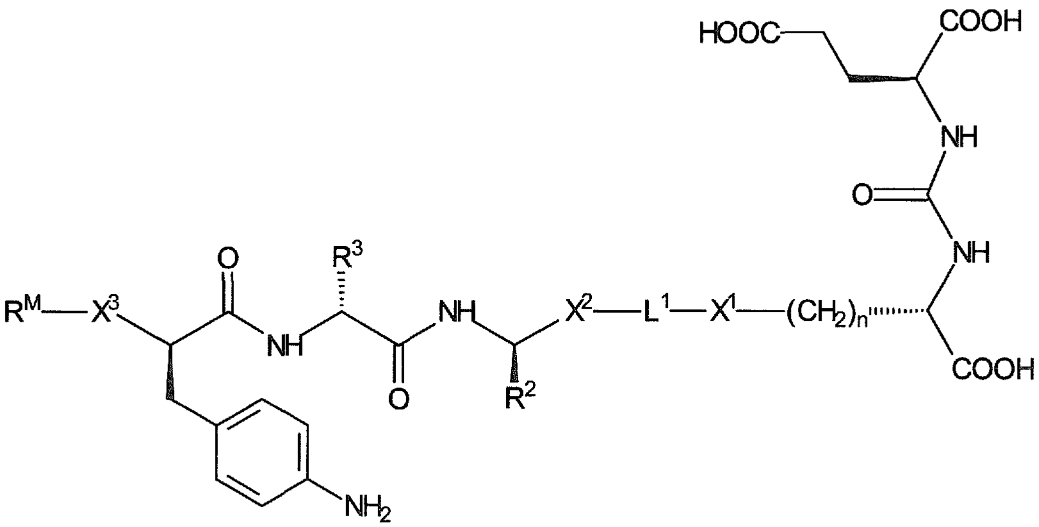

(If)

wherein n, $X^1$, $L^1$, $X^2$, $R^2$, $R^3$, $X^3$ and $R^M$ are as defined above, including their preferred embodiments.

In a further preferred embodiment, said compound has the formula (Ig) or (Ih), or is a pharmaceutically acceptable salt thereof:

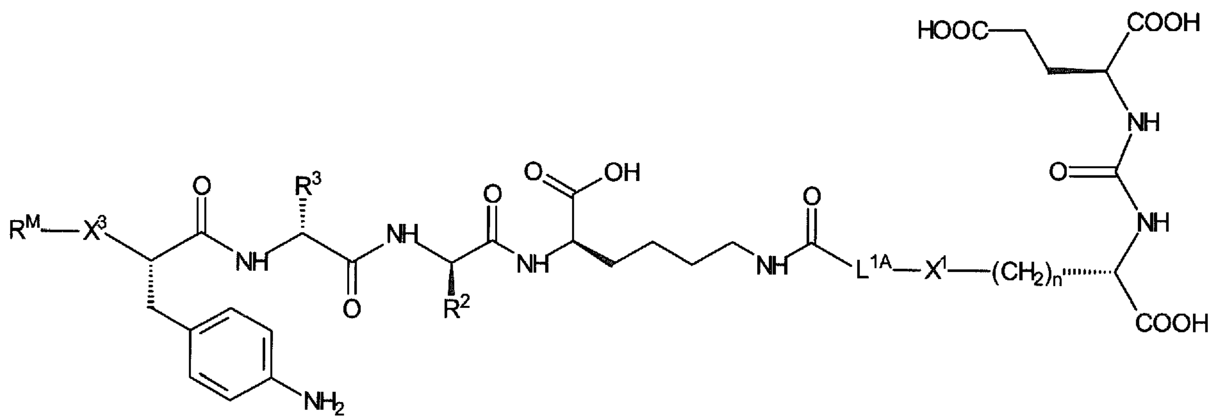

(Ig)

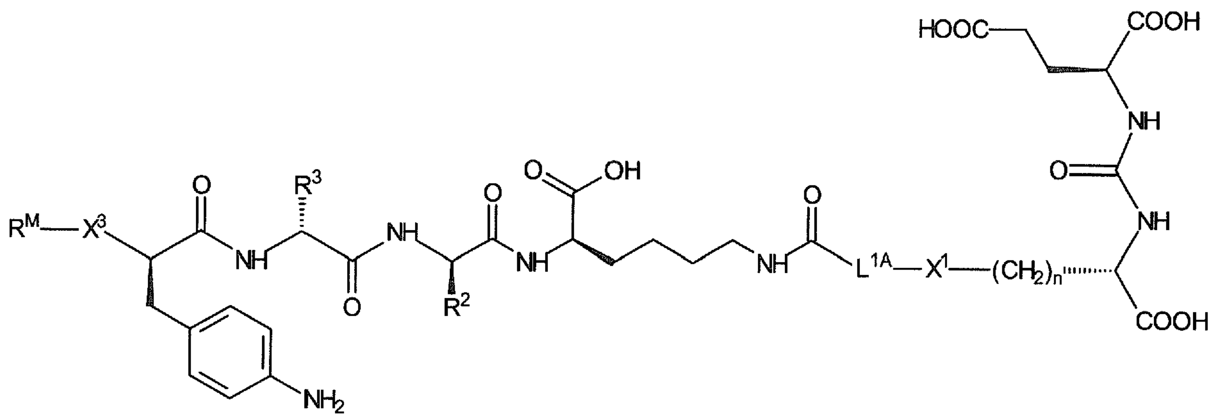

(Ih)

wherein n, $R^2$, $R^3$, $X^3$ and $R^M$ are are as defined above, including their preferred embodiments, and wherein the moiety $-L^{1A}-X^1-$ has a structure selected from:

$-R^{12A}-C(O)-NH-$ (L-7),

$-R^{14A}-C(O)-NH-R^{15A}-CH(COOH)-NH-C(O)-$ (L-8),

and

$-R^{17A}-C(O)-NH-CH(R^1)-C(O)-NH-$ (L-9);

wherein the bond at $R^{12A}$, $R^{14A}$ and $R^{17A}$, respectively, is attached to the carbon atom carrying the carbonyl group in formula (I), and wherein
$R^{12A}$ is a linear C2 to C10 alkanediyl, preferably a linear C4 to C8 alkanediyl,
$R^{14A}$, and $R^{15A}$ and $R^{17A}$ are independently selected from linear C2 to C6 alkanediyl,
and $R^1$ is a group $-CH_2$-phenyl which is optionally substituted on its aromatic ring with a substituent selected from -OH and -I.

Particularly preferred compounds of the invention are the following:

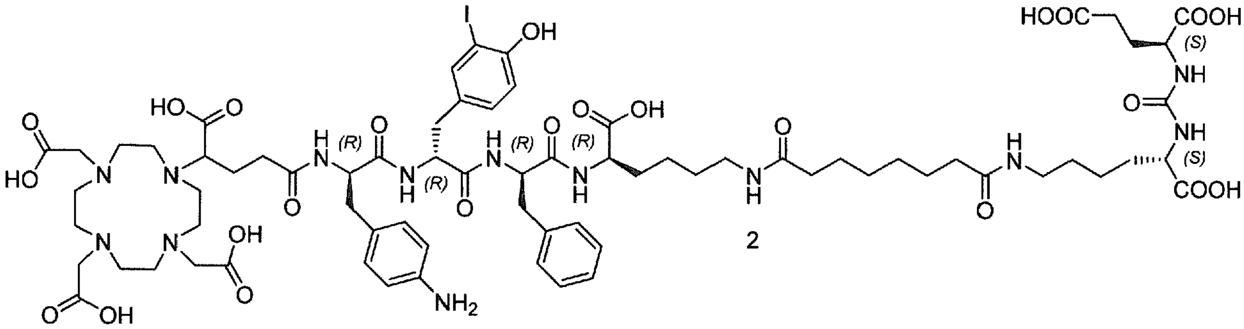

2

Chemical Formula: $C_{72}H_{102}IN_{13}O_{24}$
Molecular Weight: 1660,58

DOTAGA-(4-NH2)f-(I-y)-f-k-KuE **(PSMA-2)**

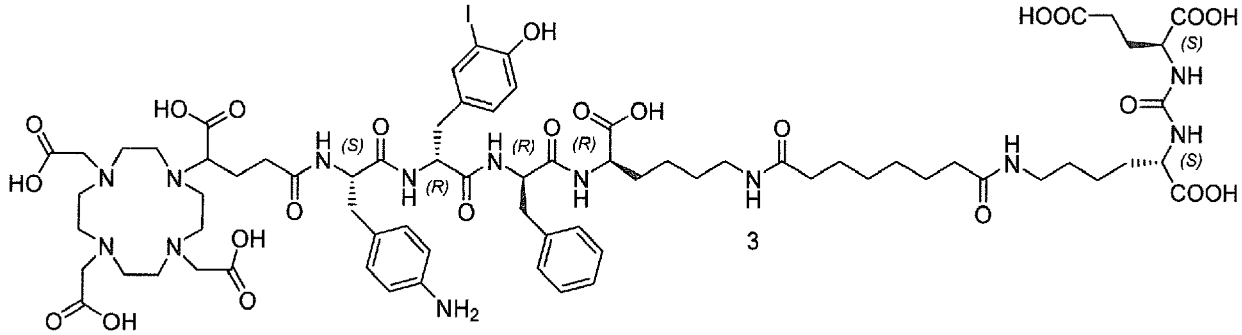

3

Chemical Formula: $C_{72}H_{102}IN_{13}O_{24}$
Molecular Weight: 1660,58

DOTAGA-(4-NH2)F-(I-y)-f-k-KuE **(PSMA-3)**

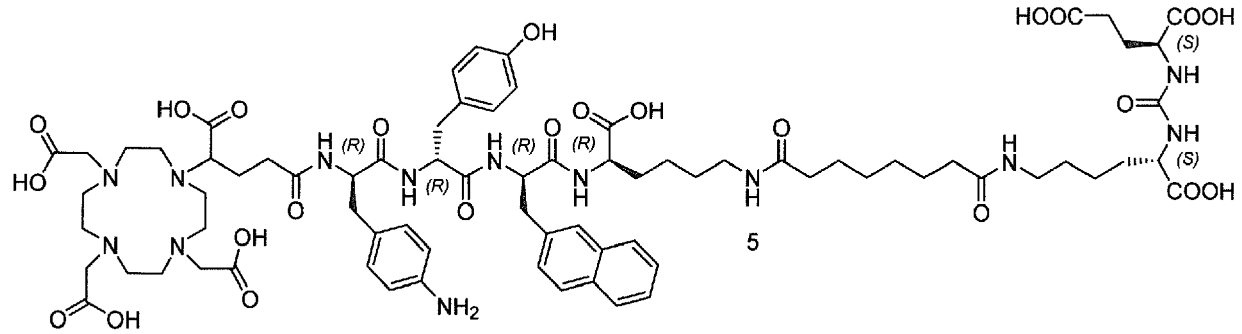

5

Chemical Formula: $C_{76}H_{105}N_{13}O_{24}$
Exact Mass: 1583,74

DOTAGA-(4-NH2)f-y-nal-k-KuE **(PSMA-5)**

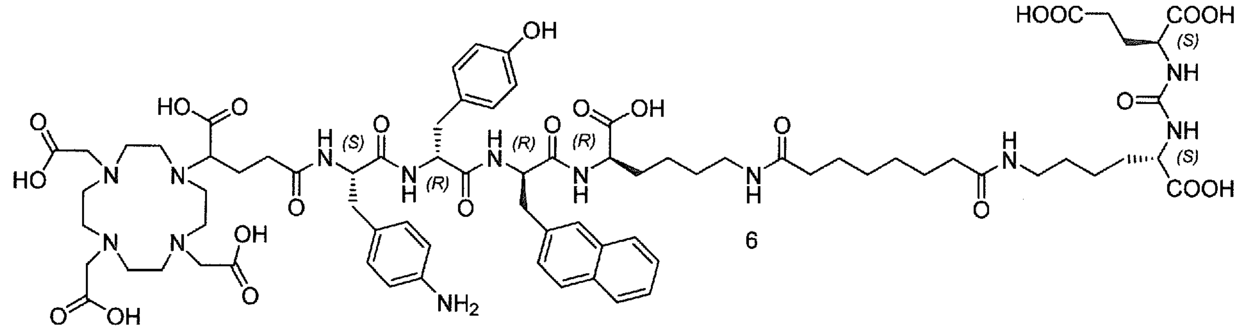

6

Chemical Formula: $C_{76}H_{105}N_{13}O_{24}$
Molecular Weight: 1584,74

DOTAGA-(4-NH2)F-y-nal-k-KuE **(PSMA-6)**

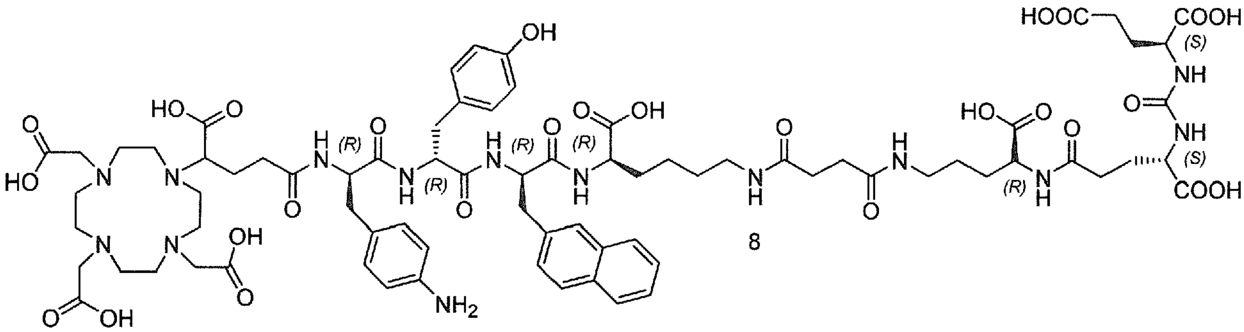

8

Chemical Formula: $C_{76}H_{102}N_{14}O_{27}$
Molecular Weight: 1643,72

DOTAGA-(4-NH2)f-y-nal-k(Suc-(HO-δ-orn-γ-EuE))-OH **(PSMA-8)**

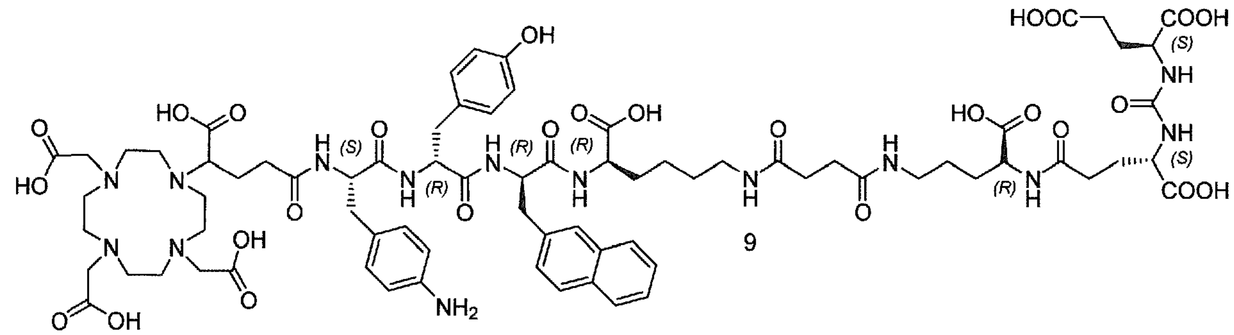

9

Chemical Formula: $C_{76}H_{102}N_{14}O_{27}$
Molecular Weight: 1643,72

DOTAGA-(4-NH2)F-y-nal-k(Suc-(HO-δ-orn-γ-EuE))-OH **(PSMA-9)**

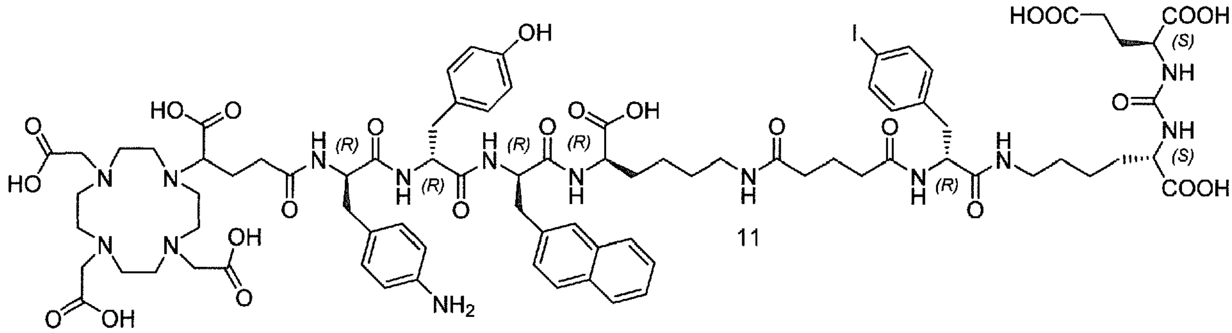

11

Chemical Formula: $C_{82}H_{107}IN_{14}O_{25}$
Molecular Weight: 1815,74

DOTAGA-(4-NH2)f-y-nal-k(Glut-(I-f)-KuE) **(PSMA-11)**

Preferred labeling schemes for these most preferred compounds are as defined herein above.

In a further aspect, the present invention provides a pharmaceutical composition comprising or consisting of one or more compounds or salts of the invention as disclosed herein above.

In a further aspect, the present invention provides a diagnostic composition comprising or consisting of one or more compounds or salts of the invention as disclosed herein above.

In a further aspect, the present invention provides a therapeutic composition comprising or consisting of one or more compounds or salts of the invention as disclosed herein above.

The pharmaceutical composition may further comprise pharmaceutically acceptable carriers, excipients and/or diluents. Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. It is particularly preferred that said administration is carried out by injection and/or delivery, e.g., to a site in the pancreas or into a brain artery or directly into brain tissue. The compositions may also be administered directly to the target site, e.g., by biolistic delivery to an external or internal target site, like the pancreas or brain. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Pharmaceutically active matter may be present in amounts between 0,1 ng and 10 mg/kg body weight per dose; however, doses below or above this exemplary range are envisioned, especially considering the aforementioned factors.

The above applies *mutatis mutandis* to diagnostic compositions.

To the extent the above disclosed pharmaceutical composition, diagnostic composition and therapeutic composition comprises one or more compounds or salts of the invention, it is preferred that no further pharmaceutically active compounds, diagnostically active compounds or therapeutically active compounds are present. In the alternative, further therapeutically active, diagnostically active or pharmaceutically active compounds may be present, for example, anticancer agents.

Combination of a therapeutic treatment with the compounds of the present invention might have a synergistic or cumulative treatment effect, similar to the treatment of neuroendocrine tumors with [$^{177}$Lu]DOTATATE radiotherapy in combination with chemotherapy or immunotherapies. A first phase 3 study comparing the combination of $^{177}$Lu PRRT and capecitabine (Xeloda; Genentech), an oral chemotherapy agent, with [$^{177}$Lu]DOTATATE alone has been started at Erasmus MC, Rotterdam in 2017 (van Essen M, Krenning EP, Kam BL, de Herder WW, van Aken MO, Kwekkeboom DJ. Report on short-term side effects of treatments with 177Lu-octreotate in combination with capecitabine in seven patients with gastroenteropancreatic neuroendocrine tumours. Eur J Nucl Med Mol Imaging. 2008;35:743-748).

Further studies on combination therapies, named peptide receptor chemoradionuclide therapy (PRCRT), have recently been published (Kong G, Callahan J, Hofman MS, et al. High clinical and morphologic response using 90Y-DOTA-octreotate sequenced with 177Lu-DOTA-octreotate induction peptide receptor chemoradionuclide therapy (PRCRT) for bulky neuroendocrine tumours. Eur J Nucl Med Mol Imaging. 2017;44:476-489). Similar "combined treatment approaches" will be carried out in the near future to improve the efficiency of PSMA-targeted radioligand therapies.

In a further aspect, the present invention provides one or more compounds or salts of the invention as disclosed herein above for use in medicine.

Preferred uses in medicine are in nuclear medicine such as nuclear diagnostic imaging, also named nuclear

molecular imaging, and/or targeted radiotherapy of diseases associated with an overexpression, preferably of PSMA on the diseased tissue.

In a further aspect, the present invention provides a compound or salt of the invention as defined herein above for use in a method of diagnosing and/or staging cancer, preferably prostate cancer.

Preferred indications are the detection or staging of cancer, such as, but not limited high grade gliomas, lung cancer and especially prostate cancer and metastasized prostate cancer, the detection of metastatic disease in patients with primary prostate cancer of intermediate-risk to high-risk, and the detection of metastatic sites, even at low serum PSA values in patients with biochemically recurrent prostate cancer. Another preferred indication is the imaging and visualization of neoangiogensis.

In terms of medical indications to be subjected to therapy, especially radiotherapy, cancer is a preferred indication. Prostate cancer is a particularly preferred indication.

In a further aspect, the present invention provides a compound or salt of the invention as defined herein above for use in a method of diagnosing and/or staging cancer, preferably prostate cancer.

As regards the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

In particular, the invention furthermore relates to the following items:

1. A compound of formula (I), or a pharmaceutically acceptable salt thereof,

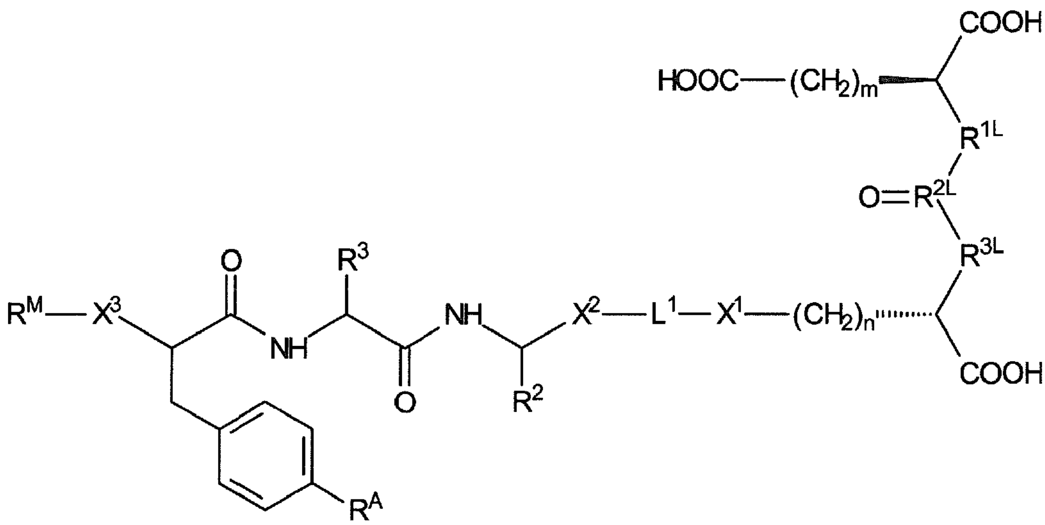

(I)

wherein:

| | |
|---|---|
| m | is an integer of 2 to 6, preferably 2 to 4, more preferably 2; |
| n | is an integer of 2 to 6, preferably 2 to 4, more preferably 4; |
| $R^{1L}$ | is $CH_2$, NH or O, preferably NH; |
| $R^{2L}$ | is C or P(OH), preferably C; |

$R^{3L}$ is $CH_2$, NH or O, preferably NH;

$X^1$ is selected from an amide bond, an ether bond, a thioether bond, an ester bond, a thioester bond, a urea bridge, an amine bond, and a group of the formula $-C(O)-NH-CH(R^1)-C(O)-NH-$,
wherein $R^1$ is a group $-CH_2$-phenyl which is optionally substituted on its aromatic ring with a substituent selected from -OH and -I; and is preferably an amide bond;

$L^1$ is a divalent linking group with a structure selected from an oligoamide, an oligoether, an oligothioether, an oligoester, an oligothioester, an oligourea, an oligo(ether-amide), an oligo(thioether-amide), an oligo(ester-amide), an oligo(thioester-amide), oligo(urea-amide), an oligo(ether-thioether), an oligo(ether-ester), an oligo(ether-thioester), an oligo ether-urea), an oligo(thioether-ester), an oligo(thioether-thioester), an oligo(thioether-urea), an oligo(ester-thioester), an oligo(ester-urea), and an oligo(thioester-urea), preferably with a structure selected from and oligoamide and an oligo(ester-amide);

$X^2$ is selected from an amide bond, an ether bond, a thioether bond, an ester bond, a thioester bond, a urea bridge, and an amine bond, and is preferably an amide bond;

$R^2$ is an aryl group or an aralkyl group, which aryl group or aralkyl group may be substituted on its aromatic ring with a substituent selected from halogen, preferably I, and -OH;

$R^3$ is an aryl group or an aralkyl group, which aryl group or aralkyl group may be substituted on its aromatic ring with a substituent selected from halogen, preferably I, and -OH;

$R^A$ is selected from $-NH_2$ and -OH, and is preferably $-NH_2$;

$X^3$ is selected from an amide bond, an ether bond, a thioether bond, an ester bond, a thioester bond, a urea bridge, an amine bond, and a group of the formula

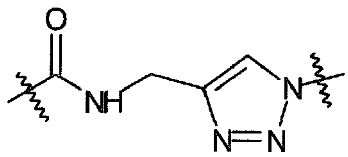
,

wherein the marked bond at the carbonyl group attaches $X^3$ to $R^M$ and the other marked bond attaches $X^3$ to the remainder of the compound of formula (I); and is preferably an amide bond;

$R^M$ is a marker group which comprises a chelating group optionally containing a chelated non-radioactive or radioactive cation.

2. The compound or salt of item 1, wherein m is 2, n is 2 or 4, $R^{1L}$ is NH, $R^{2L}$ is C, and $R^{3L}$ is NH.

3. The compound or salt of item 1 or 2, wherein n is 2 or 4.

4. The compound or salt of any one of items 1 to 3, wherein $L^1$ is a divalent linking group with a structure selected from an oligoamide which comprises a total of 1 to 5, more preferably a total of 1 to 3, and most preferably a total of 1 or 2 amide bonds within its backbone, and an oligo(ester-amide) which comprises a total of 2 to 5, more preferably a total of 2 to 3, and most preferably a total of 2 amide and ester bonds within its backbone.

5. The compound or salt of item 4, wherein $L^1$ represents a divalent linking group with an oligoamide structure which comprises one or two amide bonds within its backbone.

6. The compound or salt of any of items 1 to 5, wherein $X^1$ is selected from an amide bond and a group of the formula

$$-C(O)-NH-CH(R^1)-C(O)-NH-,$$

wherein $R^1$ is a group $-CH_2$-phenyl which is optionally substituted on its aromatic ring with a substituent selected from -OH and -I;
and $X^2$ is an amide bond.

7. The compound or salt of any of items 1 to 6, wherein the moiety $-X^2-L^1-X^1-$ has a structure selected from:

$$*-C(O)-NH-R^4-NH-C(O)-R^5-C(O)-NH- \quad (L-1),$$

$$*-C(O)-NH-R^6-NH-C(O)-R^7-C(O)-NH-R^8-NH-C(O)- \quad (L-2),$$

and

$$*\text{-C(O)-NH-R}^9\text{-NH-C(O)-R}^{10}\text{-C(O)-NH-CH(R}^1\text{)-C(O)-NH-} \quad \text{(L-3);}$$

wherein the bond marked with * is attached to the carbon atom carrying $R^2$ in formula (I), and wherein $R^4$ to $R^{10}$ are independently selected from C2 to C10 alkanediyl, preferably linear C2 to C10 alkanediyl, which alkanediyl groups may each be substituted by one or more substitutents independently selected from -OH, $-OCH_3$, -COOH, $-COOCH_3$, $-NH_2$, and -NHC(NH)$NH_2$, and $R^1$ is a group $-CH_2$-phenyl which is optionally substituted on its aromatic ring with a substituent selected from -OH and -I.

8. The compound or salt of item 7, wherein $R^4$ to $R^{10}$ are independently selected from optionally substituted linear C2 to C10 alkanediyl, and wherein
the total number of carbon atoms in $R^4$ and $R^5$ is 6 to 20, more preferably 6 to 16, without carbon atoms contained in optional substituents,
the total number of carbon atoms in $R^6$, $R^7$ and $R^8$ is 6 to 20, more preferably 6 to 16, without carbon atoms contained in optional substituents, and
the total number of carbon atoms in $R^9$ and $R^{10}$ is 4 to 16, more preferably 4 to 12, without carbon atoms contained in optional substituents.

9. The compound or salt of item 7, wherein the moiety $-X^2-L^1-X^1-$ has a structure selected from:

$$*\text{-C(O)-NH-CH(COOH)-R}^{11}\text{-NH-C(O)-R}^{12}\text{-C(O)-NH-} \quad \text{(L-4),}$$

$$*\text{-C(O)-NH-CH(COOH)-R}^{13}\text{-NH-C(O)-R}^{14}\text{-C(O)-NH-R}^{15}\text{-CH(COOH)-NH-C(O)-} \quad \text{(L-5),}$$

and

$$*\text{-C(O)-NH-CH(COOH)-R}^{16}\text{-NH-C(O)-R}^{17}\text{-C(O)-NH-CH(R}^1\text{)-C(O)-NH-} \quad \text{(L-6);}$$

wherein the bond marked with * is attached to the carbon atom carrying $R^2$ in formula (I), and wherein $R^{11}$, $R^{13}$ and $R^{16}$ are independently selected from linear C2 to C6 alkanediyl, preferably a linear C3 to C6 alkanediyl, $R^{12}$ is a linear C2 to C10 alkanediyl, preferably a linear C4 to C8 alkanediyl, $R^{14}$, $R^{15}$ and $R^{17}$ are independently selected from linear C2 to C6 alkanediyl,
and $R^1$ is a group $-CH_2$-phenyl which is optionally substituted on its aromatic ring with a substituent selected from -OH and -I.

10. The compound or salt of item 9, wherein
the total number of carbon atoms in $R^{11}$ and $R^{12}$ is 6 to 16, more preferably 6 to 14,
the total number of carbon atoms in $R^{13}$, $R^{14}$ and $R^{15}$ is 6 to 16, more preferably 6 to 14, and
the total number of carbon atoms in $R^{16}$ and $R^{17}$ is 4 to 12, more preferably 4 to 10.

11. The compound or salt of any of items 1 to 10, wherein $R^2$ is an aralkyl group of the formula $-CH_2$-phenyl or $-CH_2$--naphthyl, wherein the phenyl and the naphtyl group are optionally substituted with a substituent selected from halogen, preferably I, and -OH.

12. The compound or salt of item 11, wherein $R^2$ is an aralkyl group of the formula $-CH_2$-phenyl or $-CH_2$-(2-naphthyl).

13. The compound or salt of any of items 1 to 12, wherein $R^3$ is an aralkyl group of the formula $-CH_2$-phenyl or $-CH_2$-(2-naphthyl), wherein the phenyl and the naphtyl group are optionally substituted with a substituent selected from halogen, preferably I, and -OH.

14. The compound or salt of any of items 1 to 13, wherein $R^3$ is an aralkyl group of the formula $-CH_2$-phenyl, wherein the phenyl group is substituted with at least one substituent selected from -I and -OH, and is preferably substituted with -I and -OH.

15. The compound or salt of any of items 1 to 10,

wherein $R^2$ is a group of the formula

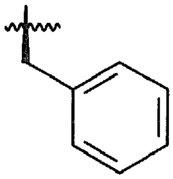

or

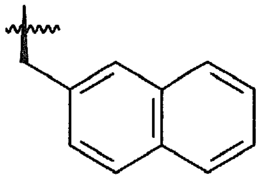

and $R^3$ is a group of the formula

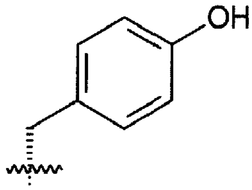

or

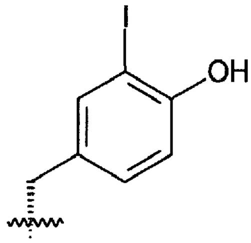

wherein marks the bond which attaches $R^2$ and $R^3$, respectively, to the remainder of the compound of formula (I).

16. The compound or salt of item 15,
wherein $R^2$ is a group of the formula

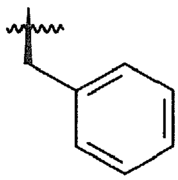

and $R^3$ is a group of the formula

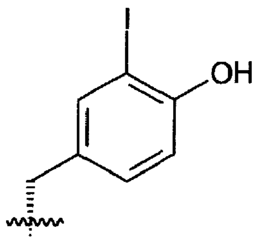

wherein marks the bond which attaches $R^2$ and $R^3$, respectively, to the remainder of the molecule.

17. The compound or salt of any of item 15,
wherein $R^2$ is a group of the formula

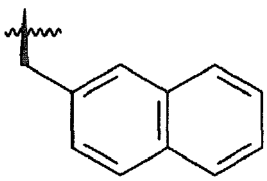

and $R^3$ is a group of the formula

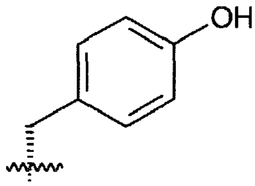

wherein marks the bond which attaches $R^2$ and $R^3$, respectively, to the remainder of the molecule.

18. The compound or salt of any of items 1 to 17, wherein $X^3$ is an amide bond or a group of the formula

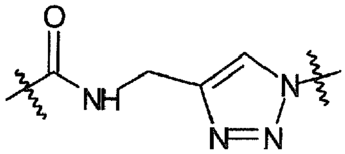

,

wherein the marked bond at the carbonyl group attaches $X^3$ to $R^M$ and the other marked bond attaches $X^3$ to the remainder of the compound.

19. The compound or salt of item 18, wherein $X^3$ is an amide bond -C(O)-NH- with the carbon atom being attached to $R^M$.

20. The compound or salt of any of items 1 to 19, wherein $R^M$ comprises a chelating group optionally containing a chelated non-radioactive or radioactive cation or is a chelating group optionally containing a chelated non-radioactive or radioactive cation.

21. The compound or salt of any of items 1 to 20, wherein the chelating group $R^M$ comprises at least one of

(i) a macrocyclic ring structure with 8 to 20 ring atoms of which 2 or more, preferably 3 or more, are selected from oxygen atoms, sulfur atoms and nitrogen atoms; and
(ii) an acyclic, open chain chelating structure with 8 to 20 main chain atoms of which 2 or more, preferably 3 or more are heteroatoms selected from oxygen atoms, sulfur atoms and nitrogen atoms.

22. The compound or salt of any of items 1 to 21, wherein the chelating group $R^M$ is a residue of a chelating agent selected from bis(carboxymethyl)-1,4,8,11-tetraazabicyclo[6.6.2]hexadecane (CBTE2a), cyclohexyl-1,2-diaminetetraacetic acid (CDTA), 4-(1,4,8,11-tetraazacyclotetradec-1-yl)-methylbenzoic acid (CPTA), N'-[5-[acetyl(hydroxy)amino]pentyl]-N-[5-[[4-[5-aminopentyl-(hydroxy)amino]-4-oxobutanoyl]amino]pentyl]-N-hydroxybutandiamide (DFO), 4,11-bis(carboxymethyl)-1,4,8,11-tetraazabicyclo[6.6.2]hexadecan (DO2A) 1,4,7,10-tetraazacyclododecan-N,N',N'',N'''-tetraacetic acid (DOTA), 2-[1,4,7,10-tetraazacyclododecane-4,7,10-triacetic acid]-pentanedioic acid (DOTAGA), N,N'-dipyridoxylethylendiamine-N,N'-diacetate-5,5'-bis(phosphat) (DPDP), diethylenetriaminepentaacetic acid (DTPA), ethylenediamine-N,N'-tetraacetic acid (EDTA), ethyleneglykol-O,O-bis(2-aminoethyl)-N,N,N',N'-tetraacetic acid (EGTA), N,N-bis(hydroxybenzyl)-ethylenediamine-N,N'-diacetic acid (HBED), hydroxyethyldiaminetriacetic acid (HEDTA), 1-(p-nitrobenzyl)-1,4,7,10-tetraazacyclodecan-4,7,10-triacetate (HP-DOA3), 6-hydrazinyl-N-methylpyridine-3-carboxamide (HYNIC), 1,4,7-triazacyclononan-1-succinic acid-4,7-diacetic acid (NODASA), 1-(1-carboxy-3-carboxypropyl)-4,7-(carbooxy)-1,4,7-triazacyclononane (NODAGA), 1,4,7-triazacyclononanetriacetic acid (NOTA), 4,11-bis(carboxymethyl)-1,4,8,11-tetraazabicyclo[6.6.2]hexadecane (TE2A), 1,4,8,11-tetraazacyclododecane-1,4,8,11-tetraacetic acid (TETA), terpyridin-bis(methyleneamintetraacetic acid (TMT), 1,4,7,10-tetraazacyclotridecan-N,N',N'',N'''-tetraacetic acid (TRITA), triethylenetetraaminehexaacetic acid (TTHA), *N*,*N*'-bis[(6-carboxy-2-pyridil)methyl]-4,13-diaza-18-crown-6 ($H_2$macropa), and 4-amino-4-{2-[(3-hydroxy-1,6-dimethyl-4-oxo-1,4-dihydro-pyridin-2-ylmethyl)-carbamoyl]-ethyl} heptanedioic acid bis-[(3-hydroxy-1,6-dimethyl-4-oxo-1,4-dihydro-pyridin- 2-ylmethyl)-amide] (THP);
which residue is provided by covalently binding a carboxyl group contained in the chelating agent to the remainder of the compound via an ester or an amide bond.

23. The compound or salt of item 22, wherein the chelating agent is selected from DOTA and DOTAGA.

24. The compound or salt of item 22 or 23, wherein $X^3$ is the amide bond attaching the chelating group to the remainder of the compound.

25. The compound or salt of item 24, wherein $R^M$-$X^3$- is a group of the formula

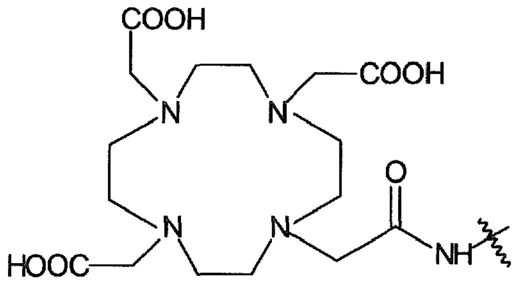

(M-1),

or

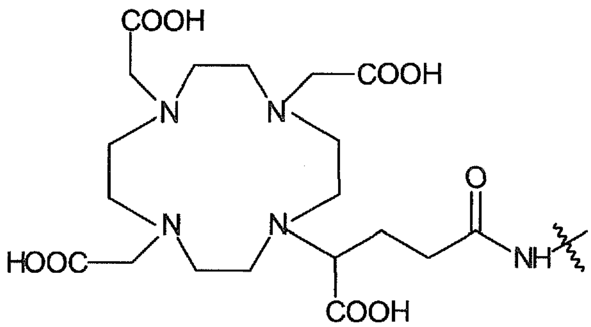

(M-2)

wherein the bond marked with 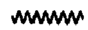 is attached to the remainder of the compound of formula (I), and wherein the chelating group may contain a chelated non-radioactive or radioactive cation.

26. The compound or salt of any of items 1 to 25, wherein the chelating group $R^M$ comprises a chelated cation, preferably a chelated radioactive cation selected from a cation of $^{44}Sc$, $^{47}Sc$, $^{51}Cr$, $^{52m}Mn$, $^{58}Co$, $^{52}Fe$, $^{56}Ni$, $^{57}Ni$, $^{62}Cu$, $^{64}Cu$, $^{67}Cu$, $^{66}Ga$, $^{68}Ga$, $^{67}Ga$, $^{89}Zr$, $^{90}Y$, $^{89}Y$, $^{94m}Tc$, $^{99m}Tc$, $^{97}Ru$, $^{105}Rh$, $^{109}Pd$, $^{111}Ag$, $^{110m}In$, $^{111}In$, $^{113m}In$, $^{114m}In$, $^{117m}Sn$, $^{121}Sn$, $^{127}Te$, $^{142}Pr$, $^{143}Pr$, $^{149}PM$, $^{151}Pm$, $^{149}Tb$, $^{153}Sm$, $^{157}Gd$, $^{161}Tb$, $^{166}Ho$, $^{165}Dy$, $^{169}Er$, $^{169}Yb$, $^{175}Yb$, $^{172}Tm$, $^{177}Lu$, $^{186}Re$, $^{188}Re$, $^{191}Pt$, $^{197}Hg$, $^{198}Au$, $^{199}Au$, $^{212}Pb$, $^{203}Pb$, $^{211}At$, $^{212}Bi$, $^{213}Bi$, $^{223}Ra$, $^{225}Ac$, and $^{227}Th$, or a cationic molecule comprising $^{18}F$, such as $^{18}F$-$[AlF]^{2+}$.

27. The compound or salt of item 26, wherein the chelating group $R^M$ comprises a chelated cation selected from a cation of $^{44}Sc$, $^{47}Sc$, $^{64}Cu$, $^{67}Cu$, $^{68}Ga$, $^{90}Y$, $^{111}In$, $^{161}Tb$, $^{166}Ho$, $^{177}Lu$, $^{188}Re$, $^{212}Pb$, $^{212}Bi$, $^{213}Bi$, $^{225}Ac$, and $^{227}Th$ or a cationic molecule comprising $^{18}F$.

28. The compound of any of items 1 to 27, which has the formula (Ia) or (Ib), or a pharmaceutically acceptable salt thereof:

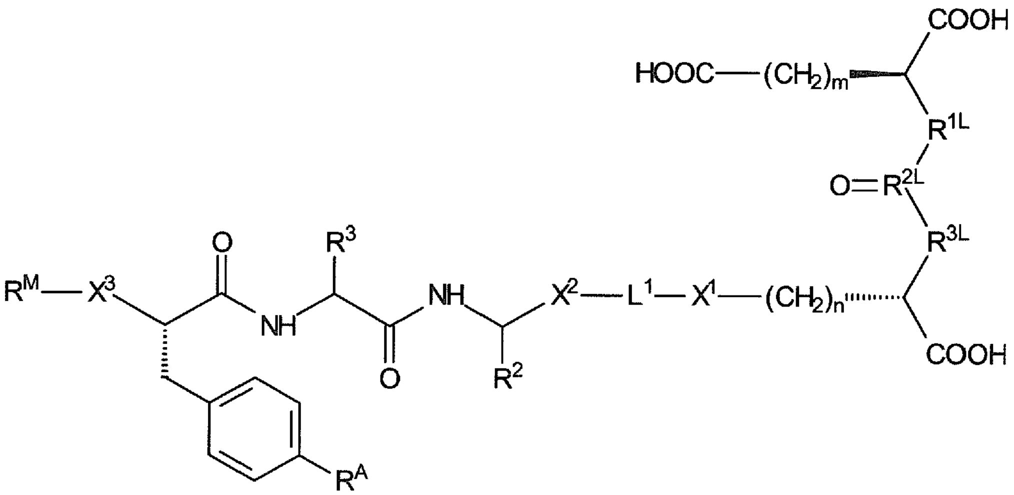

(Ia)

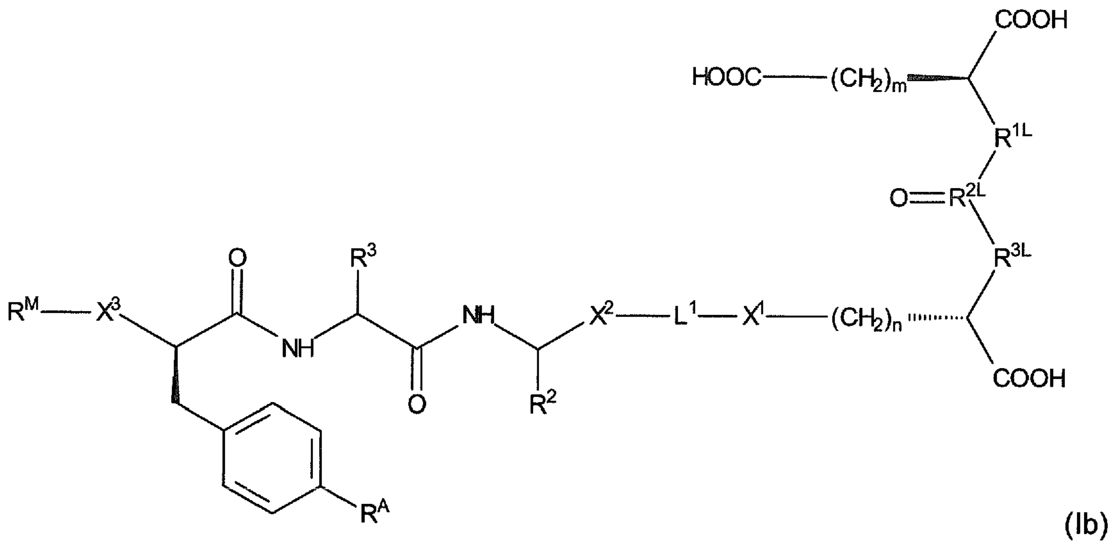

(Ib)

wherein $R^{1L}$, $R^{2L}$, $R^{3L}$, m, n, $X^1$, $L^1$, $X^2$, $R^2$, $R^3$, $R^A$, $X^3$ and $R^M$ are defined as in the preceding items.

29. The compound of any of items 1 to 28, which has the formula (Ic) or (Id), or a pharmaceutically acceptable salt thereof:

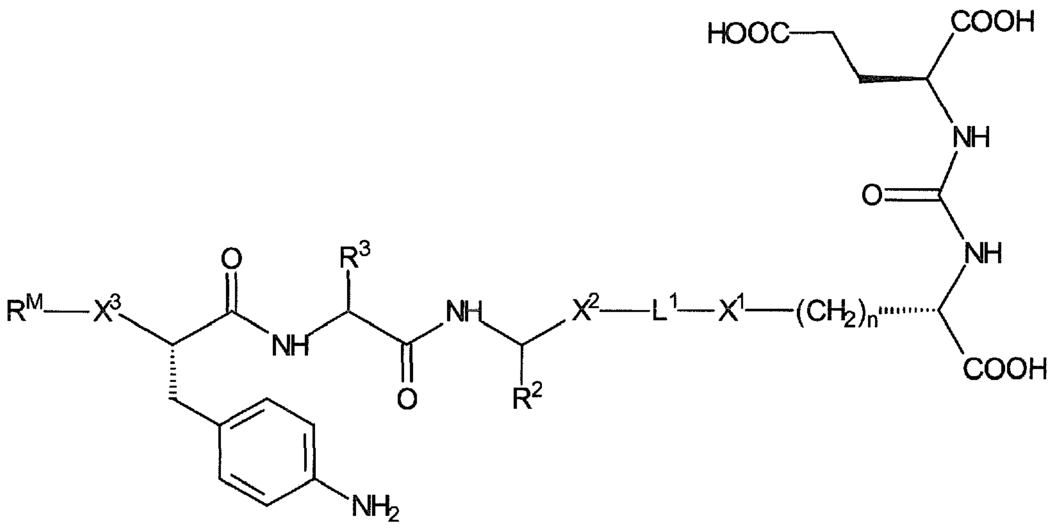

(Ic)

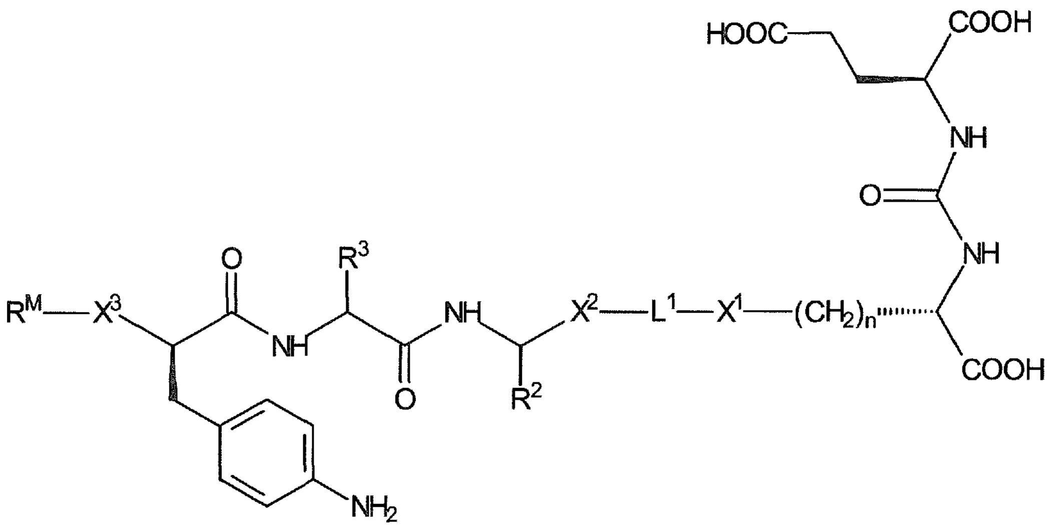

(Id)

wherein n, $X^1$, $L^1$, $X^2$, $R^2$, $R^3$, $X^3$ and $R^M$ are defined as in the preceding items.

30. The compound of item 29, which has the formula (Ie) or (If), or a pharmaceutically acceptable salt thereof:

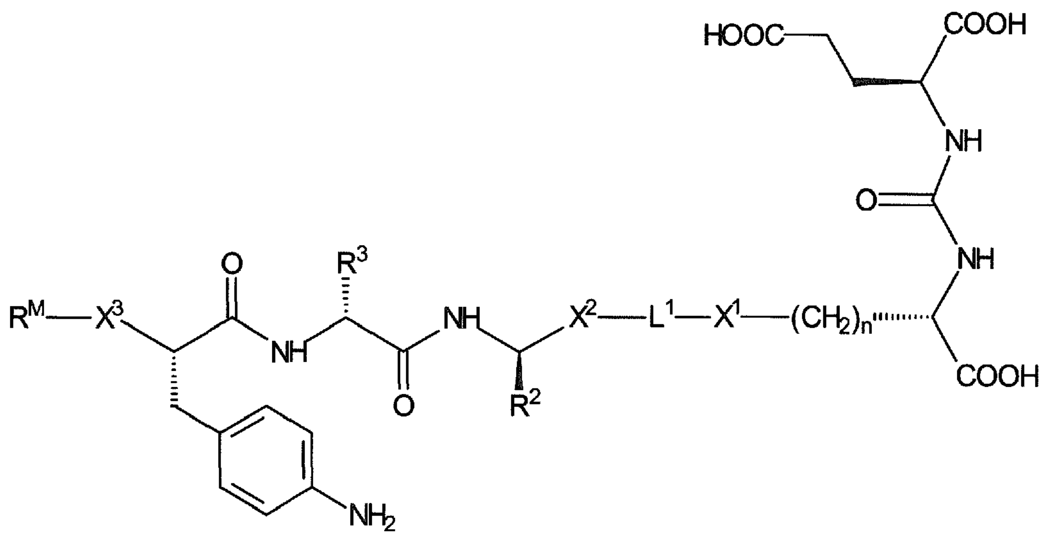

(Ie)

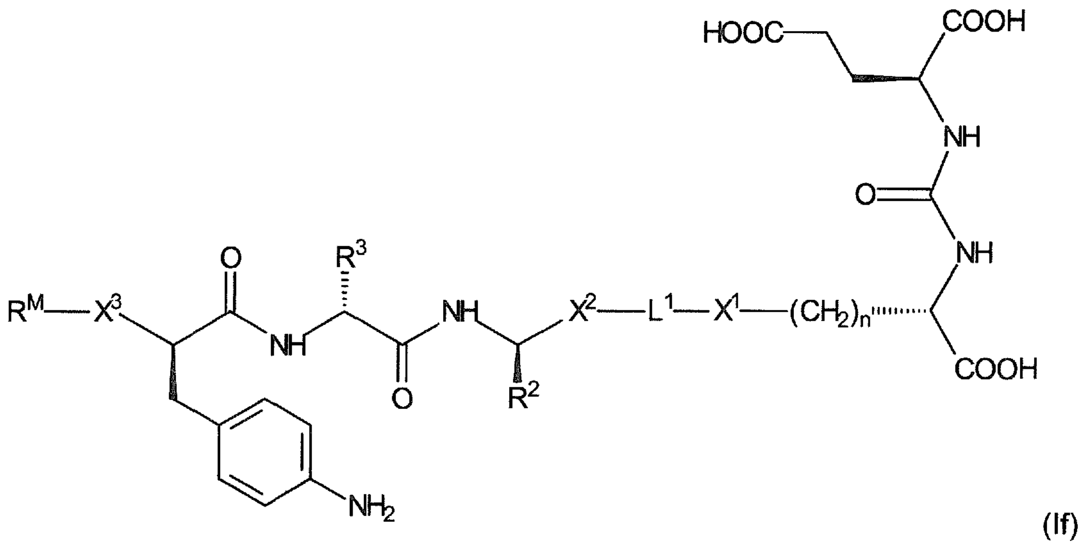

(If)

wherein n, $X^1$, $L^1$, $X^2$, $R^2$, $R^3$, $X^3$ and $R^M$ are defined as in the preceding items.

31. The compound of item 30, which has the formula (Ig) or (Ih), or a pharmaceutically acceptable salt thereof:

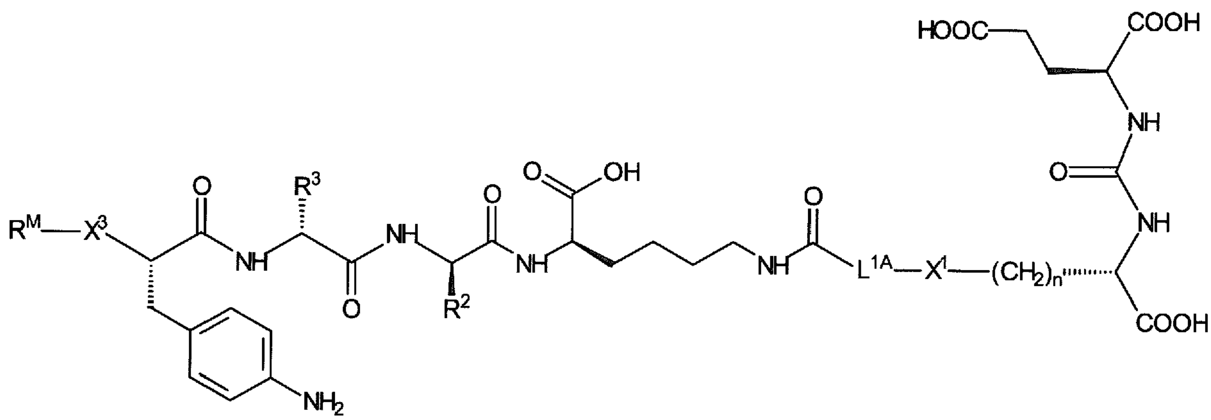

(Ig)

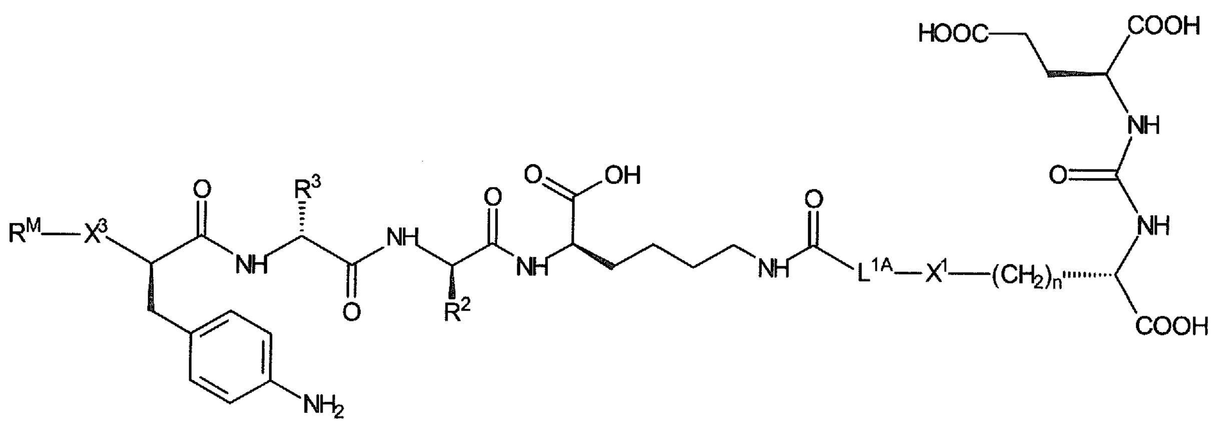

(Ih)

wherein n, $R^2$, $R^3$, $X^3$ and $R^M$ are defined as in the preceding items, and wherein the moiety $-L^{1A}-X^1-$ has a structure selected from:

$-R^{12A}-C(O)-NH-$ (L-7),

$-R^{14A}-C(O)-NH-R^{15A}-CH(COOH)-NH-C(O)-$ (L-8),

and

$-R^{17A}-C(O)-NH-CH(R^1)-C(O)-NH-$ (L-9);

wherein the bond at $R^{12A}$, $R^{14A}$ and $R^{17A}$, respectively, is attached to the carbon atom carrying the carbonyl group in formula (I), and wherein
$R^{12A}$ is a linear C2 to C10 alkanediyl, preferably a linear C4 to C8 alkanediyl,
$R^{14A}$, and $R^{15A}$ and $R^{17A}$ are independently selected from linear C2 to C6 alkanediyl, and $R^1$ is a group $-CH_2$-phenyl which is optionally substituted on its aromatic ring with a substituent selected from -OH and -I.

32. The compound or salt of any one of the preceding items, wherein said compound or salt has one of the following formulae:

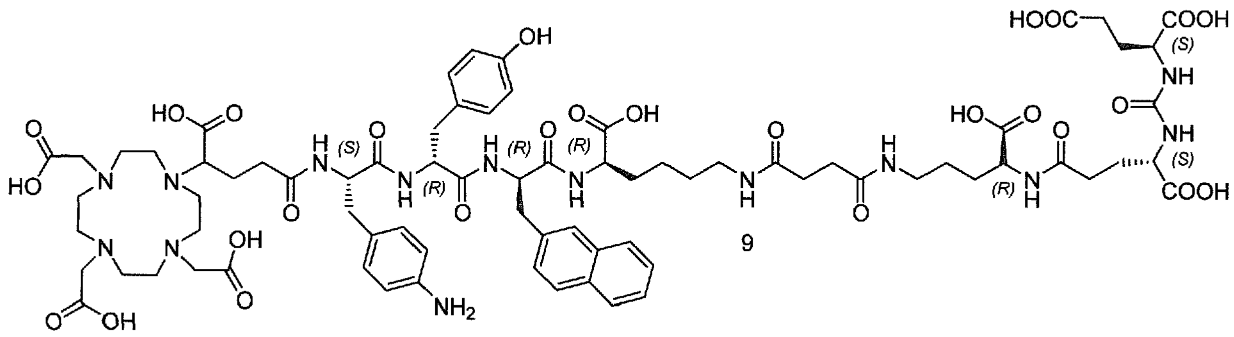

**(Ii)**

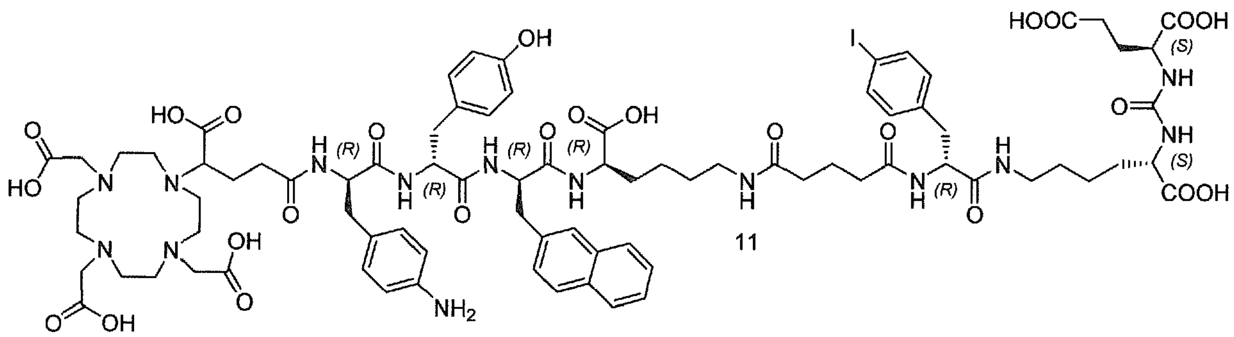

**(Ij).**

33. A pharmaceutical or diagnostic composition comprising or consisting of one or more compounds or salts in accordance with any one of items 1 to 32.

34. A compound or salt in accordance with any one of items 1 to 32 for use in a method of diagnosing and/or treating

(a) cancer including prostate cancer; or
(b) neoangiogenesis/angiogenesis.

The figures show:

**Figure 1:** Exemplary correlation of determination of the nine reference substances in OriginPro 2016G.

**Figure 2:** Radio-HPLC of analyses from homogenized organs and body fluids from mice after 60 min p.i., ~ 11 MBq of [$^{177}$Lu]PSMA-2.

**Figure 3:** Maximum intensity projections (MIP) of μPET scans in LNCaP xenograft bearing mice. Dynamic MIP (summed up frames 1 to 1.5 h p.i.) of [$^{68}$Ga]PSMA-1, [$^{68}$Ga]PSMA-2 and [$^{68}$Ga]PSMA-3 (0.15 to 0.25 nmol peptide, respectively).

**Figure 4:** Maximum intensity projections (MIP) of μPET scans in LNCaP xenograft bearing mice. Dynamic MIP (summed up frames 1 to 1.5 h p.i.) of [$^{68}$Ga]PSMA-4 and [$^{68}$Ga]PSMA-6 (0.15 to 0.25 nmol peptide, respectively).

**Figure 5:** Maximum intensity projections (MIP) of μPET scans in LNCaP xenograft bearing mice. Dynamic MIP (summed up frames 1 to 1.5 h p.i.) of [$^{68}$Ga]PSMA-7, [$^{68}$Ga]PSMA-8 and [$^{68}$Ga]PSMA-9 (0.15 to 0.25 nmol peptide, respectively).

**Figure 6:** Maximum intensity projections (MIP) of μPET scans in LNCaP xenograft bearing mice. Dynamic MIP (summed up frames 1 to 1.5 h p.i.) of [$^{68}$Ga]PSMA-10 and [$^{68}$Ga]PSMA-11 (0.15 to 0.25 nmol peptide, respectively).

**Figure 7:** Dynamic μPET Imaging of [$^{68}$Ga]PSMA-1, [$^{68}$Ga]PSMA-2 and [$^{68}$Ga]PSMA-3. Left (A): Activity accumulation in tumor. Right (B): Activity accumulation in kidney; 0.15 - 0.25 nmol PSMA Inhibitor in LNCaP Xenograft bearing CB-17 SCID mice.

**Figure 8:** Dynamic μPET Imaging of [$^{68}$Ga]PSMA-4 and [$^{68}$Ga]PSMA-6. Left (A): Activity accumulation in tumor. Right (B): Activity accumulation in kidney; 0.15 - 0.25 nmol PSMA Inhibitor in LNCaP Xenograft bearing CB-17 SCID mice.

**Figure 9:** Dynamic μPET Imaging of [$^{68}$Ga]PSMA-7, [$^{68}$Ga]PSMA-8 and [$^{68}$Ga]PSMA-9. Left (A): Activity accumulation in tumor. Right (B): Activity accumulation in kidney; 0.15 - 0.25 nmol PSMA Inhibitor in LNCaP Xenograft bearing CB-17 SCID mice.

**Figure 10:** Dynamic μPET Imaging of [$^{68}$Ga]PSMA-10 and [$^{68}$Ga]PSMA-11. Left (A): Activity accumulation in tumor. Right (B): Activity accumulation in kidney; 0.15 - 0.25 nmol PSMA Inhibitor in LNCaP Xenograft bearing CB-17 SCID mice.

**Figure 11:** Biodistribution data (in %ID/g). Biodistribution in LNCaP tumor xenograft bearing CB-17 SCID mice for [$^{177}$Lu]PSMA-1, [$^{177}$Lu]PSMA-2 and [$^{177}$Lu]PSMA-3 at 1 h p.i. (n = 4; respectively).

**Figure 12:** Biodistribution data (in %ID/g). Biodistribution in LNCaP tumor xenograft bearing CB-17 SCID mice for [$^{177}$Lu]PSMA-1, [$^{177}$Lu]PSMA-2 and [$^{177}$Lu]PSMA-9 at 1 h p.i. (n = 4; respectively).

**Figure 13:** Biodistribution data (in %ID/g). Biodistribution in LNCaP tumor xenograft bearing CB-17 SCID mice for [$^{177}$Lu]PSMA-10 and [$^{177}$Lu]PSMA-11 at 1 h p.i. (left) and 24 h p.i. (right) (n = 4; respectively).

**Figure 14:** Biodistribution data (in %ID/g). Biodistribution in LNCaP tumor xenograft bearing CB-17 SCID mice for $^{177}$Lu-1 (PSMA I&T), [$^{177}$Lu]PSMA-10 and [$^{177}$Lu]PSMA-11 at 1 h p.i. (left) and 24 h p.i. (right) (n = 4; respectively).

The examples illustrate the invention.

**Example 1: Material and Methods**

General

The Fmoc-(9-fluorenylmethoxycarbonyl-) and all other protected amino acid analogs were purchased from Bachem (Bubendorf, Switzerland) or Iris Biotech (Marktredwitz, Germany). PSMA I&T (**1**), PSMA (k,nal,y) (PSMA-**4**) and PSMA ((4-i)f,k,nal,y) (PSMA-**10**) were provided by Dr. Wirtz. The tritylchloride polystyrene (TCP) resin was obtained from PepChem (Tubingen, Germany). Chematech (Dijon, France) delivered the chelator DOTAGA-anhydride. All necessary solvents and other organic reagents were purchased from either Alfa Aesar (Karlsruhe, Germany), Sigma-Aldrich (Munich, Germany) or VWR (Darmstadt, Germany). Solid phase synthesis of the peptides was carried out by manual operation using an Intelli-Mixer syringe shaker (Neolab, Heidelberg, Germany). Analytical reversed-phase high performance liquid chromatography (RP-HPLC) was performed on a Nucleosil 100 C18 (5 μm, 125 × 4.0 mm) column (CS GmbH, Langerwehe, Germany) using a Shimadzu gradient HPLC System (Shimadzu Deutschland GmbH, Neufahrn, Germany). Analysis of the peptides was performed by applying different gradients of 0.1% (v/v) trifluoroacetic acid (TFA) in $H_2O$ (solvent A) and 0.1% TFA (v/v) in acetonitrile (MeCN) (solvent B) with a constant flow of 1 mL/min (specific gradients are cited in the text). The Shimadzu SPD 20 A prominence UV/VIS detector (Shimadzu Deutschland GmbH) was used at 220 nm and 254 nm. Human serum albumin (HSA) binding was determined using a Chiralpak HSA 50 x 3 mm 5μm Analytical Column connected to a Chiralpak HSA 10 x 3 mm 5μm Guard Cartridge (Daicel Chemical Industries) purchased from Chiral Technologies Europe (France). Nonlinear regression for the HSA binding was performed using OriginPro 2016G. Both retention times $t_R$ as well as the capacity factors K' are cited in the text. Preparative RP-HPLC of the peptides was achieved on a Shimadzu HPLC system using a Multospher 100 RP 18-5 (250 × 20 mm) column (CS GmbH, Langerwehe, Germany) with a constant flow of 5 mL/min. Analytical and preparative Radio RP-HPLC of the radioiodinated reference ligand was performed using a Nucleosil 100 C18 (5 μm, 125 × 4.0 mm) column. Radioactivity was detected through connection of the outlet of the UV-photometer to a NaI(TI) well-type scintillation counter from EG&G Ortec (Munich, Germany). The $^{68}$Ga-and $^{177}$Lu-labeled compounds were analyzed as published previously

(Šimeček, J., et al., A Monoreactive Bifunctional Triazacyclononane Phosphinate Chelator with High Selectivity for Gallium-68. ChemMedChem, 2012. 7(8): p. 1375-1378; Weineisen, M., et al., Development and first in human evaluation of PSMA I&T-A ligand for diagnostic imaging and endoradiotherapy of prostate cancer. Journal of Nuclear Medicine, 2014. 55(supplement 1): p. 1083-1083). ESI-mass spectra were acquired on an expression$^L$ CMS mass spectrometer (Advion Ltd., Harlow, UK) and on a Varian 500-MS IT mass spectrometer (Agilent Technologies, Santa Clara, USA). For the HSA binding experiment a Chiralpak HSA 50 x 3 mm 5μm Analytical Column, (Daicel Chemical Industries, LTD) connected to a Chiralpak HSA 10 x 3 mm 5μm Guard Cartridge (Daicel Chemical Industries ; Chiral Technologies Europe) was used.

General synthesis protocols

*Fmoc-based solid-phase peptide synthesis with various amino acids (AA) with following standard Fmoc strategy*

**TCP-resin loading:** TCP-resin (1.95 mmol/g) is loaded with Fmoc-AA-OH (1.5 eq.) in anhydrous DCM with DIPEA (4.5 eq.) at room temperature (RT) for 2 h. The remaining tritylchloride is capped by addition of 2 mL/g methanol for 15 min. After that, the resin is filtered and thoroughly washed with DCM (2 x), with DMF (2 x) and methanol, respectively and stored under vacuum overnight. The loading is determined using the weight differences:

$$\frac{(m_{total} - m_{net\ weight}) \times 1000}{(M_{As} - M_{HCl}) \times m_{weight\ of\ resin}} = mmol/g$$

$m_{total}$: Δ mass of loaded resin (Fmoc-AA-OH and HCl); $M_{As}$: molar mass of amino acid $m_{net}$ weight: mass of used resin; $M_{HCl}$: molar mass of hydrochloric acid

**Peptide synthesis via TBTU/HOBt coupling:** A solution of Fmoc-AA(tboc)-OH or Fmoc-AA(Dde)-OH (2.0 eq.), TBTU (2.0 eq.), HOBt (2.0 eq.), DIPEA (5.2 eq.) in DMF (8 ml/g resin) was added to the resin-bound free amine peptide and shaken for 2 h at RT and washed with DMF (6 x 2 min). The coupling with secondary or aromatic amines was performed employing a different protocol. 3.0 eq. of Fmoc-AA-OH was dissolved in DMF (8 mL/g resin) together with HATU (3.0 eq.), HOAt (3.0 eq.) and DIPEA (6.0 eq.) and stirred for 15 min. The preactivated solution was added to the resin bound secondary peptide and shaken for 2 h at RT. After completion of the reaction, the resin was washed 6 times with DMF. In general, all peptidic scaffolds were synthesized as previously described (Weineisen, M., et al., Development and first in human evaluation of PSMA I&T-A ligand for diagnostic imaging and endoradiotherapy of prostate cancer. Journal of Nuclear Medicine, 2014. 55(supplement 1): p. 1083-1083; Weineisen, M., et al., Synthesis and preclinical evaluation of DOTAGA-conjugated PSMA ligands for functional imaging and endoradiotherapy of prostate cancer. EJNMMI research, 2014. 4(1): p. 1).

**On-resin Fmoc-deprotection:** The resin-bound Fmoc peptide was treated with 20% piperidine in DMF (v/v) for 5 min and a second time for 15 min. Afterwards, the resin was washed thoroughly with DMF (8 x 2 min).

**On-resin Dde-deprotection:** The Dde-protected peptide (1.0 eq.) was dissolved in a solution of 2% hydrazine monohydrate in DMF (v/v). After 15 min, the deprotected peptide was washed with DMF (6 x), if bound to resin, or precipitated in diethyl ether to give the crude product.

**On-resin Alloc-deprotection:** The allyloxy-protecting group was removed from the resin-bound peptide using a solution of 6 mL DCM containing triisopropylsilane (TIPS) (50.0 eq.) and (triphenyl)palladium(0) ($Pd(PPh_3)_4$) ( 0.3 eq.). The resin was treated with this solution for 1.5 h at RT. Finally, the resin was washed with DCM (3 x 2 min) to remove the $Pd(PPh_3)_4$. **tBu/tBoc deprotection:** Removal of tBu/tBoc-protecting groups was carried out by dissolving the crude product in TFA and stirring for 40 min at RT. After precipitation in diethyl ether, the crude product was dissolved in water and purified using preparative HPLC.

**Peptide cleavage from the resin:**

**A) With preservation of side chain protecting groups:** The fully protected, resin-bound peptide was dissolved in a mixture of DCM/TFE/AcOH (v/v/v; 6/3/1) and shaken for 30 min. The solution was filtered off and the resin was dissolved in another cleavage solution for another 30 min. The fractions were combined and the solvent was concentrated under reduced pressure. The filtrate was redissolved in toluene and concentrated under reduced pressure to remove the acetic acid. Precipitation in water resulted in the crude, side chain protected peptide.

**B) Cleavage with concurrent deprotection of all acid labile protecting groups:** The fully protected, resin bound

peptide was dissolved in a mixture of TFA/TIPS/water (v/v/v; 95/2.5/2.5) and shaken for 30 min. The solution was filtered off and the resin was treated in the same way for another 30 min. Afterwards, the fractions were combined and the solvent was concentrated under a constant flow of nitrogen. The crude peptide was precipitated in diethyl ether and left to dry overnight.

Synthesis of the inhibitors PSMA 1-6 and PSMA 10 and 11

*Binding motif Lys-urea-Glu ((OtBu)KuE(OtBu)$_2$) (1)*

The synthesis of the carboxyl-protected Lys-urea-Glu binding motif (EuK) was synthesized as previously described by solution phase synthesis (Weineisen, M., et al., Synthesis and preclinical evaluation of DOTAGA-conjugated PSMA ligands for functional imaging and endoradiotherapy of prostate cancer. EJNMMI research, 2014. 4(1): p. 1). The crude product was filtered through celite, the solvent was evaporated in vacuo, and the desired product was obtained as a waxy solid (4.33 g, 91.5% yield). HPLC (10 to 90% B in 15 min): $t_R$ = 12.6 min; K' = 6.4. Calculated monoisotopic mass ($C_{24}H_{45}N_3O_7$): 487.6 found: m/z = 488.3 $[M+H]^+$, 510.3 $[M+Na]^+$.

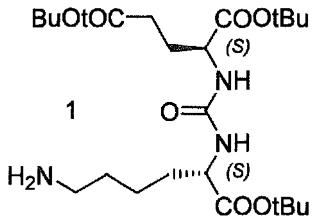

1

Chemical Formula: $C_{24}H_{45}N_3O_7$
Molecular Weight: 487,64

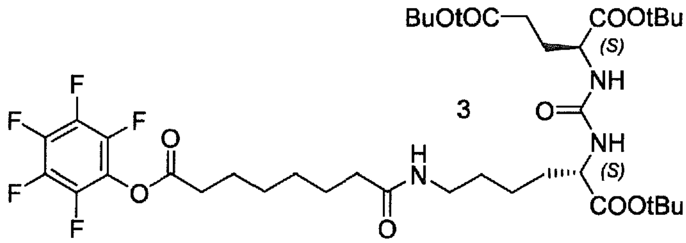

3

Chemical Formula: $C_{38}H_{56}F_5N_3O_{10}$
Molecular Weight: 809,87

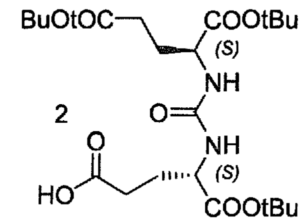

2

Chemical Formula: $C_{23}H_{40}N_2O_9$
Molecular Weight: 488,58

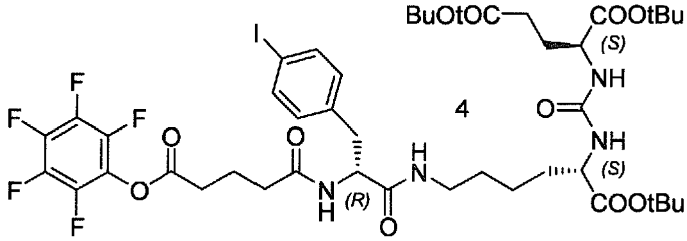

4

Chemical Formula: $C_{44}H_{58}F_5IN_4O_{11}$
Molecular Weight: 1040,86

**Scheme 1** Fragments used for the synthesis of PSMA 1-6 and PSMA 10-11

*Binding motif Glu-urea-Glu* ((OtBu)EuE(OtBu)$_2$) (2)

The synthesis of the carboxyl-protected Glu-urea-Glu binding motif (EuE) was similarly synthesized as previously described (Weineisen, M., et al., Synthesis and preclinical evaluation of DOTAGA-conjugated PSMA ligands for functional imaging and endoradiotherapy of prostate cancer. EJNMMI research, 2014. 4(1): p. 1). The desired product was obtained as waxy solid (4.10 g, 84 % yield). HPLC (10% to 90% B in 15 min): $t_R$ = 11.3 min; K' = 7.69. Calculated monoisotopic mass ($C_{23}H_{49}N_2O_9$): 488.3; found: m/z = 489.4 $[M+H]^+$, 516.4 $[M+Na]^+$.

*Di-pentafluorophenyl suberate (Sub(OPfp)$_2$)*

To a solution of 2.0 g (11.5 mmol, 1.0 eq.) suberic acid in 30 mL tetrahydrofurane, 2.8 mL (34.5 mmol, 3.0 eq.) pyridine, 7.1 mL (46.0 mmol, 4.0 eq.) DIC in 15 mL THF and 8.47 g (46.0 mmol, 4.0 eq.) pentafluorophenol in 15 mL tetrahydrofurane were successively added. Progress of the active ester formation was monitored using TLC (ethyl acetate/petroleum ether (55 - 65°C) (1/9)). After app. 2 h at RT, the reaction mixture was filtered, and the solvent was evaporated in vacuo. The crude product was purified via silica gel flash-chromatography using an eluent mixture of ethyl acetate/petroleum ether (1/9). The product was obtained as a yellow crystalline solid in 68% yield. Calculated monoisotopic mass for Sub(OPfp)$_2$ ($C_{20}H_{12}O_4F_{10}$) = 506.1 (Product is not detectable using ESI-MS).

*OPfp-Sub-(OtBu)KuE(OtBu)$_2$ (3)*

To a solution of 400 mg (0.8 mmol, 1.0 eq.) (OtBu)KuE(OtBu)$_2$ in 100 mL THF 274 μl (1.6 mmol, 2.0 eq.) DIPEA were added. This solution was added dropwise (within 30 min) to a solution of 1.6 g (3.2 mmol, 4.0 eq.) Sub(OPfp)$_2$. After stirring for additional 2 h at RT, the reaction mixture was concentrated in vacuo, and the crude product was purified via silica gel flash-chromatography using a stepwise gradient of ethyl acetate in petroleum ether (55 - 65°C) of 10%, 50%, 90% and pure ethyl acetate (200 mL each). OPfp-Sub-(OtBu)KuE(OtBu)$_2$ was obtained as a yellowish oil in 58% yield. Calculated monoisotopic mass ($C_{38}H_{56}F_5N_3O_{10}$) = 809.4 found: m/z = 810.6 [M+H]$^+$, 832.4 [M+Na]$^+$.

*Di-pentafluorophenyl glutarate (Glut(OPfp)$_2$)*

To 2.0 g (15.1 mmol, 1.0 eq.) glutaric acid in 15 mL THF was added 3.7 mL (45.4 mmol, 3.0 eq.) pyridine, 9.5 mL (60.5 mmol, 4.0 eq.) DIC in 10 mL THF and 11.1 g (60.5 mmol, 4.0 eq.) pentafluorophenol in 10 mL THF. After 2 h the solvent was removed in vacuo, the crude dissolved in petrol ether was filtered and purified using silica gel flash chromatography (petrol ether/ethyl acetate = 95/5) yielding 6.1 g (87%) of a white crystalline solid. HPLC (10 to 100% B in 15 min): $t_R$ = 17.5 min K' = 7.75. Calculated monoisotopic mass ($C_{19}H_{36}N_4O_6$) = 416.3 found: m/z = 417.1 [M+H]$^+$.

*(4-i)f-(OtBu) KuE(OtBu)$_2$*

A solution of 0.5 g (0.97 mmol, 1.2 eq.) Fmoc-D-4-iodo-Phe, 0.2 g (1.22 mmol, 1.5 eq.) HOAt, 0.2 mL (0.16 g, 1.22 mmol, 1.5 eq.) DIC and 0.6 mL (0.47 g, 3.65 mmol, 4.5 eq.) DIPEA in 15 mL THF was stirred at RT for 1 h. After addition of 395 mg (0.81 mmol, 1.0 eq.) (OtBu)KuE(OtBu)$_2$ (**1**) in 5 mL THF the reaction mixture was stirred overnight. Water (20 mL) was added and extracted with 25 mL ethyl acetate (3×), followed by 20 mL H2O (3 ×) and 25 mL brine. The organic phase was dried over $MgSO_4$ and the solvent was evaporated in vacuo yielding 2.0 g (> 100%) Fmoc-(4-i)f-(OtBu)KuE(OtBu)$_2$ as a white solid, which was dissolved in 25.0 mL DMF and 5.0 mL piperidine was added and stirred for 2 h. The crude product was purified using HPLC (58% B isocratic). HPLC (10 to 90% B in 20 min): $t_R$ = 15.6 min. K' = 8.2. Calculated monoisotopic mass ($C_{33}H_{53}IN_4O_8$): 760.3 found: m/z = 761.4 [M+H]$^+$, 783.4 [M+Na]$^+$, 799.4 [M+K]$^+$, 593.3 [M-3 tBu +H]$^+$, 649.3 [M-2 tBu +H]$^+$, 705.3 [M-tBu +H]$^+$.

*OPfp-Glut-(4-i)f-(OtBu)KuE(OtBu)$_2$ (4)*

At 0 °C 270 mg (0.35 mmol, 1.0 eq.) (3-i)f-(OtBu)KuE(OtBu)$_2$ and 122 μL (0.71 mmol, 2.0 eq.) DIPEA in 20 mL THF were slowly added to 660 mg (1.42 mmol, 4.0 eq.) Glut(OPfp)$_2$ dissolved in 10 mL THF. After 2 h at RT, the solvent was removed in vacuo and the crude product was purified using silica gel flash chromatography (petrol ether/ethyl acetate: 10/1→1/10) yielding 143 mg (39%). HPLC (10 to 90% B in 15 min): $t_R$ = 18.0 min K' = 9.6. Calculated monoisotopic mass ($C_{44}H_{58}F_5IN_4O_{11}$): 1,040.3 found: m/z = 1,041.2 [M+H]$^+$, 1,063.6 [M+Na]$^+$.

*Preparation of the peptides 5-8*

The general synthesis route is depicted in **Scheme 2**

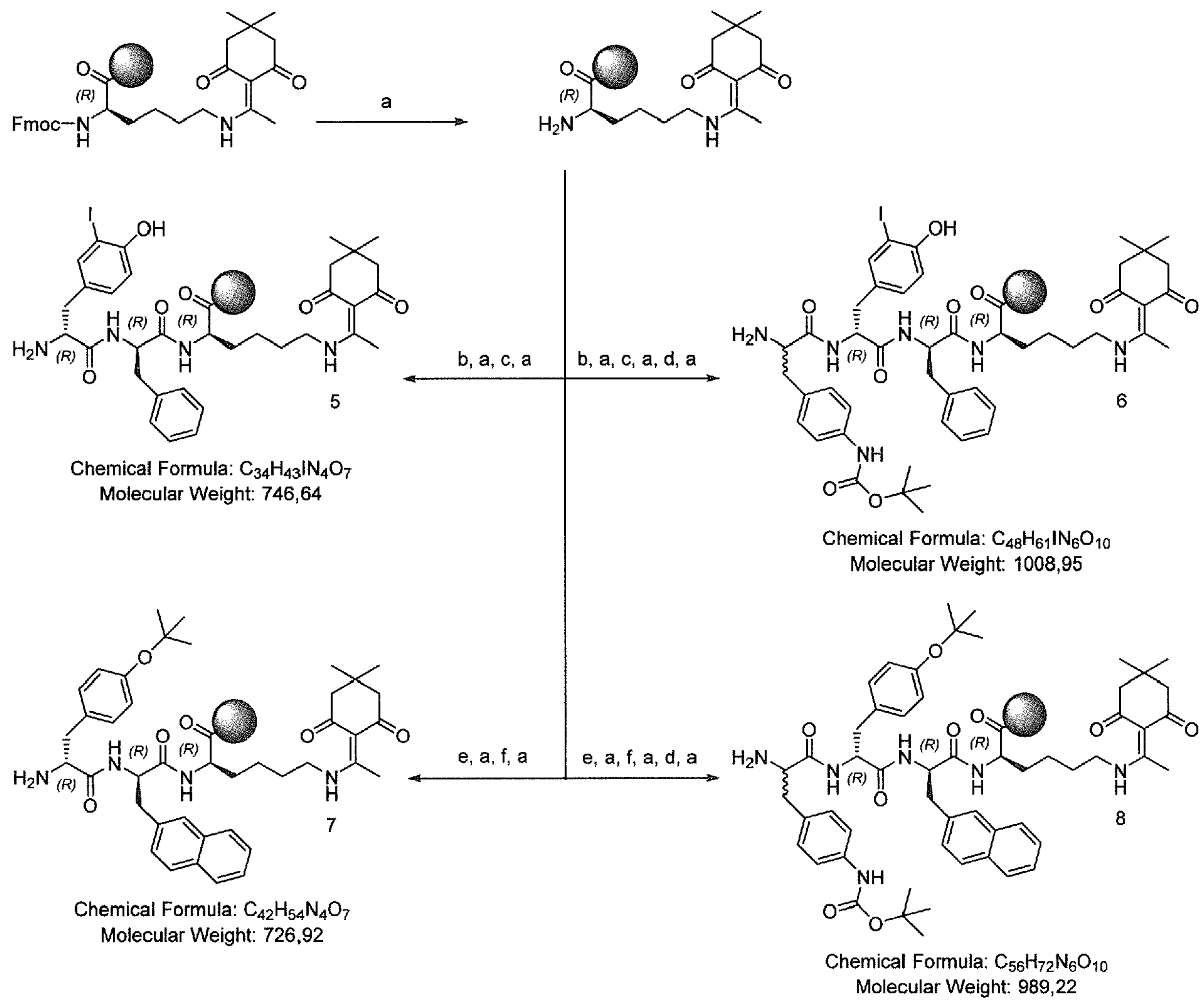


**Scheme 2 Synthesis of the peptides 5-8 (a)** 20% piperidine in DMF, [DMF]; **(b)** Fmoc-D-Phe-OH, HOBt, TBTU, DIPEA, [DMF]; **(c)** Fmoc-D-Tyr(3-I)-OH, HOBt, TBTU, DIPEA, [DMF]; **(d)** Fmoc-D/L-Phe(4-NHBoc)-OH, HOBt, TBTU, DIPEA, [DMF]; **(e)** Fmoc-D-2Nal-OH, HOBt, TBTU, DIPEA, [DMF]; **(f)** Fmoc-D-Tyr(tBu)-OH, HOBt, TBTU, DIPEA, [DMF].

**Iodo-D-Tyr-D-Phe-D-Lys-(Dde)-OH; (I-y)fk(Dde)** (**5**) was synthesized through solid phase strategy as previously described (Weineisen, M., et al., Development and first in human evaluation of PSMA I&T-A ligand for diagnostic imaging and endoradiotherapy of prostate cancer. Journal of Nuclear Medicine, 2014. 55(supplement 1): p. 1083-1083; Weineisen, M., et al., Synthesis and preclinical evaluation of DOTAGA-conjugated PSMA ligands for functional imaging and endoradiotherapy of prostate cancer. EJNMMI research, 2014. 4(1): p. 1).
**D/L-Phe(4-NHBoc)-iodo-D-Tyr-D-Phe-D-Lys-(Dde)-OH** ; **(4-NHBoc)f(I-y)fk(Dde)** (**6**) was synthesized through solid phase strategy as peptide **5**
**D-Tyr(tBu)-D-Nal-D-Lys-(Dde)-OH ; y(tBu)fk(Dde)** (**7**) was synthesized through solid phase strategy as peptide **5**
**D/L-Phe(4-NHBoc)-D-Tyr(tBu)-D-Nal-D-Lys-(Dde)-OH ; (4-NHBoc)fy(tBu)fk(Dde)** (**8**) was synthesized through solid phase strategy as peptide **5**

*Condensation of the peptides with the chelator DOTAGA (15-18)*

The condensation of the peptides **5-8** and the respective chelator DOTAGA-anhydride are described in several publications and summarized as follows: The N-terminal deprotected peptides (1.0 eq.) were dissolved together with DOTAGA-anhydride (1.5 eq.) and DIPEA (10.0 eq.) in dry DMF. The reaction was allowed to stir overnight and precipitated

in diethyl ether. The dry crude product was Dde-deprotected at RT and afterwards purified through HPLC and yielded in the peptides **15 -18** (Weineisen, M., et al., Synthesis and preclinical evaluation of DOTAGA-conjugated PSMA ligands for functional imaging and endoradiotherapy of prostate cancer. EJNMMI research, 2014. 4(1): p. 1; Weineisen, M., et al., 68Ga- and 177Lu-Labeled PSMA I&T: Optimization of a PSMA-Targeted Theranostic Concept and First Proof-of-Concept Human Studies. Journal of Nuclear Medicine, 2015. 56(8): p. 1169-1176).

**DOTAGA-iodo-D-Tyr-D-Phe-D-Lys-OH ; (DOTAGA-(I-y)-f-k) (15)**

HPLC (10 to 90% B in 15 min): $t_R$ = 6.9 min K' = 2.5. Calculated monoisotopic mass ($C_{43}H_{61}IN_8O_{14}$): 1,040.3 found: m/z = 1,041.5 $[M+H]^+$.

**DOTAGA-D/L-Phe(4-NHBoc)-iodo-D-Tyr-D-Phe-D-Lys-OH ; (DOTAGA-(4-NHBoc)f-(I-y)-f-k) (16)**

HPLC (10 to 90% B in 15 min): $t_R$ = 8.1 min K' = 3.1. Calculated monoisotopic mass ($C_{57}H_{79}IN_{10}O_{17}$): 1,302.5 found: m/z = 1,303.3 $[M+H]^+$, 652.3 $[M+2H]^{2+}$.

**DOTAGA-D-Tyr(tBu)-D-Nal-D-Lys-OH ; (DOTAGA-y(tBu)-nal-k) (17)**

HPLC (10 to 90% B in 15 min): $t_R$ = 7.9 min K' = 3.0. Calculated monoisotopic mass ($C_{51}H_{72}N_8O_{14}$): 1,020.5 found: m/z = 1,021.4 $[M+H]^+$.

**DOTAGA-D/L-Phe(4-NHBoc)-D-Tyr(tBu)-D-Nal-D-Lys-OH ; (DOTAGA-(4-NHBoc)-f-y(tBu)-nal-k) (18)**

HPLC (10 to 90% B in 15 min): $t_R$ = 9.6 min K' = 3.8. Calculated monoisotopic mass ($C_{65}H_{90}N_{10}O_{17}$): 1282.7 found: m/z = 1283.2 $[M+H]^+$, 642.6 $[M+2H]^{2+}$.

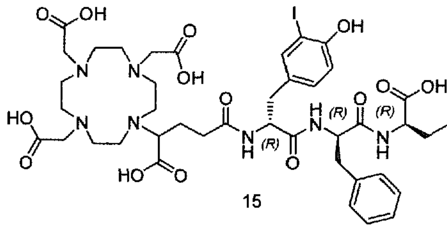

15

Chemical Formula: $C_{43}H_{61}IN_8O_{14}$
Molecular Weight: 1040,91

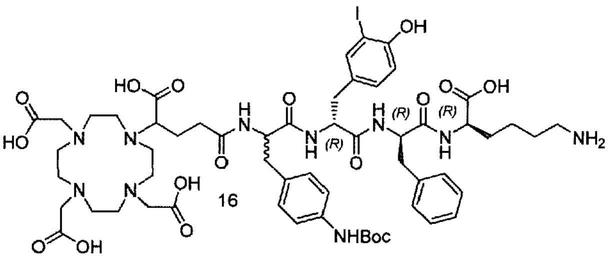

16

Chemical Formula: $C_{57}H_{79}IN_{10}O_{17}$
Molecular Weight: 1303,22

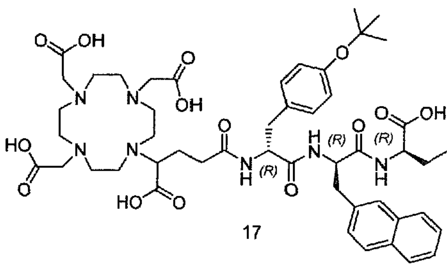

17

Chemical Formula: $C_{51}H_{72}N_8O_{14}$
Molecular Weight: 1021,18

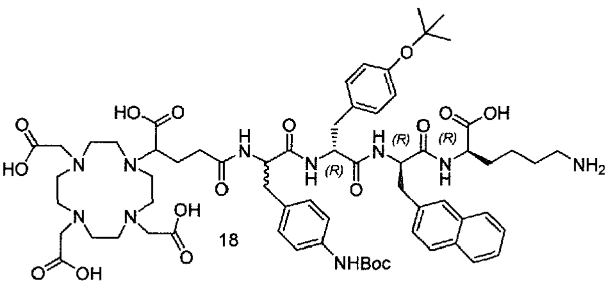

18

Chemical Formula: $C_{65}H_{90}N_{10}O_{17}$
Molecular Weight: 1283,49

**Scheme 3** DOTAGA conjugated peptides 15-18

*Preparation of the inhibitors PSMA 1-6*

Either Peptide **15** or **17** (1.0 eq.) or the D or L isomer of peptides **16** and **18** (1.0 eq.) were added to a solution of **3** (2.0 eq.) in DMF. After 5 min stirring DIPEA (5.0 eq.) was added and further stirred overnight. Deprotection was carried out using TFA for 45 min at RT. The crude products were purified through RP-HPLC.

**DOTAGA-(I-y)-f-k-KuE (PSMA-1):** HPLC (25 to 55% B in 15 min): $t_R$ = 11.5 min, K' = 5.4. Calculated monoisotopic mass ($C_{63}H_{92}IN_{11}O_{23}$): 1,497.5 found: m/z = 1,498.3 $[M+H]^+$, 1,520.1 $[M+Na]^+$.

**DOTAGA-(4-NH$_2$)f-(I-y)-f-k-KuE (PSMA-2):** HPLC (20 to 50% B in 20 min): $t_R$ = 13.8 min, K' = 3.6. Calculated monoisotopic mass ($C_{72}H_{102}IN_{13}O_{24}$): 1,659.6 found: m/z = 1660.5 $[M+H]^+$, 831.1 $[M+2H]^{2+}$

**DOTAGA-(4-NH$_2$)F-(I-y)-f-k-KuE (PSMA-3):** HPLC (20 to 50% B in 20 min): $t_R$ = 12.6 min, K' = 3.2. Calculated monoisotopic mass ($C_{72}H_{102}IN_{13}O_{24}$): 1,659.6 found: m/z = 1660.1 $[M+H]^+$, 831.3 $[M+2H]^{2+}$.

**DOTAGA-y-nal-k-KuE (PSMA-4):** HPLC (25 to 55% B in 15 min.): $t_R$ = 12.5 min K' = 6.14. Calculated monoisotopic mass ($C_{67}H_{95}N_{11}O_{23}$) = 1,421.7 found: m/z = 1,422.8 $[M+H]^+$, 712.1 $[M+2H]^{2+}$.

**DOTAGA-(4-NH$_2$)f-y-nal-k-KuE (PSMA-5):** HPLC (20 to 35% B in 15 min.): $t_R$ = 14.6 min K' = 3.9. Calculated monoisotopic mass ($C_{76}H_{105}N_{13}O_{24}$) = 1,583.7 found: m/z = 1584.4 $[M+H]^+$, 793.1 $[M+2H]^{2+}$.

**DOTAGA-(4-$NH_2$)F-y-nal-k-KuE (PSMA-6):** HPLC (20 to 50% B in 15 min.): $t_R$ = 10.9 min K' = 2.6. Calculated monoisotopic mass ($C_{76}H_{105}N_{13}O_{24}$) = 1,583.7 found: m/z = 1584.4 $[M+H]^+$, 793.2 $[M+2H]^{2+}$.

*Preparation of the inhibitors PSMA 10 and 11*

Into a solution of OPfp-Glut-(l-f)-(OtBu)KuE$(OtBu)_2$ (1.0 eq.) in DMF was added either **17** or the D-isomer of **18** (1.0 eq.) and stirred for 5 min at RT. Afterwards, DIPEA (5 eq.) was added and the mixture further stirred overnight. Deprotection of the tBu/tBoc-esters was achieved in TFA within 45 min and the crude products were precipitated in diethyl ether and purified by HPLC

**DOTAGA-y-nal-k(Glut-(l-f)-KuE) (PSMA-10):** HPLC (35 to 60% B in 15 min): $t_R$ = 10.5 min K' = 5.2. Calculated monoisotopic mass ($C_{73}H_{97}IN_{12}O_{24}$): 1,652.6 found: m/z = 1,654.9 $[M+H]^+$, 1,676.0 $[M+Na]^+$, 827.7 $[M+2H]^{2+}$.

**DOTAGA-(4-$NH_2$)f-y-nal-k(Glut-(l-f)-KuE) (PSMA-11):** HPLC (30 to 40% B in 20 min): $t_R$ = 11.5 min K' = 2.8. Calculated monoisotopic mass ($C_{82}H_{107}IN_{14}O_{25}$): 1,814.7found: m/z = 1,815.6 $[M+H]^+$, 908.4 $[M+2H]^{2+}$

Synthesis of the inhibitors PSMA 7-9

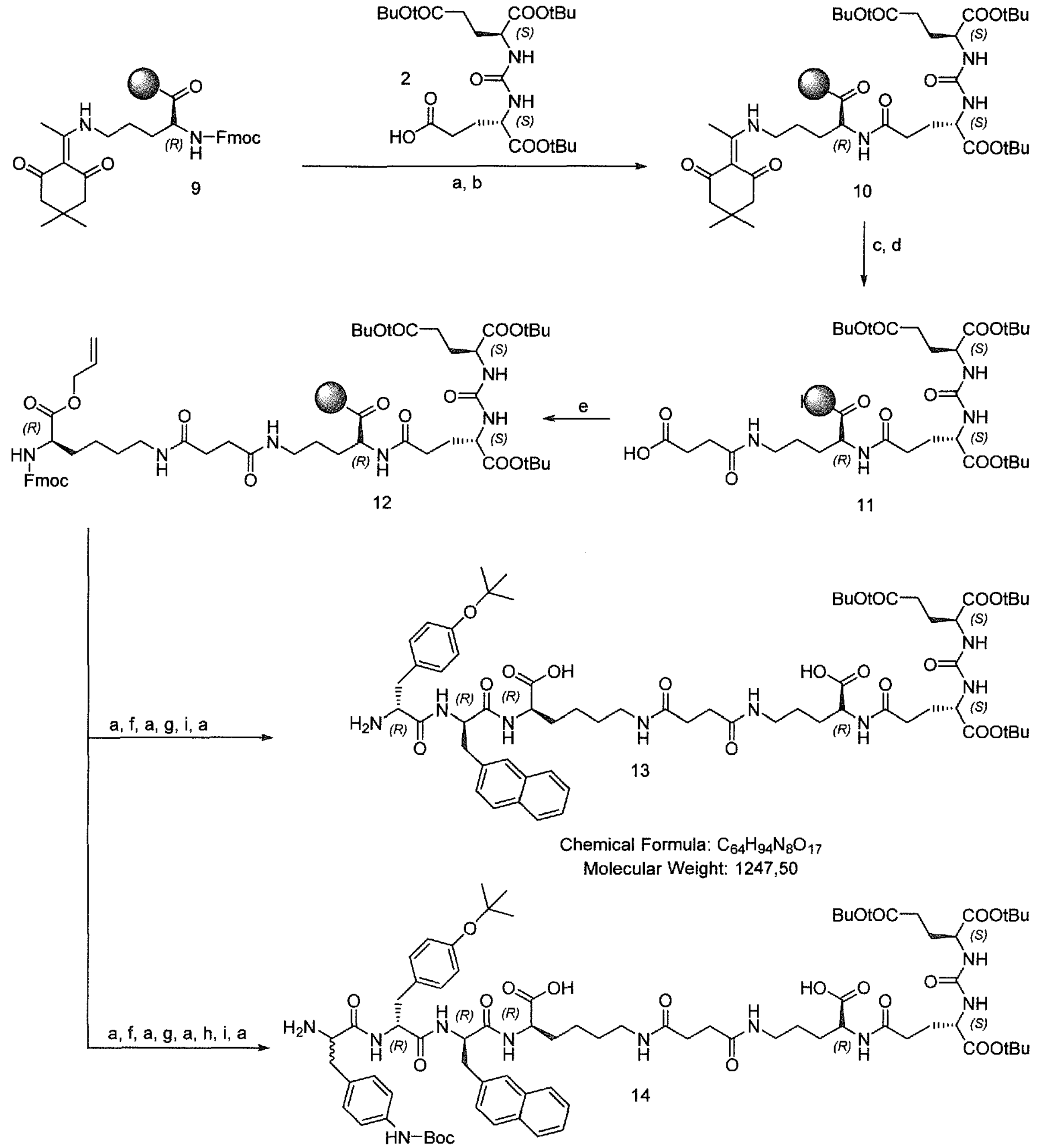


2, 9, 10, 11, 12, 13, 14

Chemical Formula: $C_{64}H_{94}N_8O_{17}$
Molecular Weight: 1247,50

Chemical Formula: $C_{78}H_{112}N_{10}O_{20}$
Molecular Weight: 1509,80

**Scheme 4** **(a)** 20% piperidine in DMF, [DMF]; **(b)** **2**, HOBt, TBTU, DIPEA, [DMF]; **(c)** 2% hydrazine, [DMF]; **(d)** glutaric anhydride, [DMF]; **(e)** Fmoc-D-Lys-OAll, HOBt, TBTU, DIPEA, [DMF]; **(f)** Fmoc-D-2Nal-OH, HOBt, TBTU, DIPEA, [DMF]; **(g)** Fmoc-D-Tyr(tBu)-OH, HOBt, TBTU, DIPEA, [DMF]; **(h)** Fmoc-D/L-Phe(4-NHBoc)-OH, HOBt, TBTU, DIPEA, [DMF]; **(i)** TIPS, $Pd(PPh_3)_4$, [DCM].

*Preparation of the peptides 13 and 14*

The general procedure is depicted in **Scheme 4.** The resin bound amino acid Fmoc-D-Orn(Dde)-OH was Fmoc deprotected using 20% piperidine in DMF (v/v) for 5 min and a second time for 5 min to assure that the Dde protecting

group is not affected. After a washing step with DMF (6 x 2min) the resin bound amino acid was further conjugated to **2** (2.0 eq.) with a mixture of TBTU (2.0 eq.), HOBt (2.0 eq.) and DIPEA (5.2 eq.) in DMF for 4 h at RT and resulted in **10.** Subsequently, the resin-bound peptide 10 was Dde deprotected using a solution of 2% hydrazine monohydrate in DMF (v/v). After 15 min, the deprotected peptide was washed with DMF (6 x). Conjugation to with glutaric anhydride (4.0 eq.) for 4 h in DMF yielded in **11.** After a further washing step with DMF (6 x) the peptides **13** and **14** were consecutively synthesized using a standard Fmoc/tBu/Alloc synthesis protocol as previously published (Weineisen, M., et al., Synthesis and preclinical evaluation of DOTAGA-conjugated PSMA ligands for functional imaging and endoradiotherapy of prostate cancer. EJNMMI research, 2014. 4(1): p. 1; Eder, M., et al., 68Ga-complex lipophilicity and the targeting property of a urea-based PSMA inhibitor for PET imaging. Bioconjugate chemistry, 2012. 23(4): p. 688-697). The fully protected, resin-bound peptides **13** and **14** were dissolved in a mixture of DCM/TFE/AcOH (v/v/v; 6/3/1) and shaken for 30 min. The solution was filtered off and the resin was dissolved in another cleavage solution for another 30 min. The fractions were combined and the solvent was concentrated under reduced pressure. The filtrate was redissolved in toluene and concentrated under reduced pressure to remove the acetic acid. Precipitation in water resulted in the crude, side chain protected peptides.

**D-Tyr(tBu)-D-Nal-D-Lys-(Suc-(δ-D-Orn-(tBuO-γ-EuE(OtBu)-OtBu)) (13):** HPLC (10 to 90% B in 15 min): $t_R$ = 11.7 min K' = 4.9. Calculated monoisotopic mass ($C_{64}H_{94}N_8O_{17}$): 1,246.7 found: m/z = 1,247,8 $[M+H]^+$.

**D/L-Phe(NHBoc)-D-Tyr(tBu)-D-Nal-D-Lys-(Suc-(δ-D-Orn-(tBuO-γ-EuE(OtBu)-OtBu)) (14):** HPLC (10 to 90% B in 15 min): $t_R$ = 13.7 min K' = 5.9. Calculated monoisotopic mass ($C_{78}H_{112}N_{10}O_{20}$): 1,508.8 found: m/z = 1,509.9 $[M+H]^+$.

*Preparation of the inhibitors PSMA 7-9*

Either peptide **13** or the D or L isomer of peptide **14** (1.0 eq.) or the D were added to a solution containing DOTAGA-anhydride (1.5 eq.) and DIPEA (10.0 eq.) in dry DMF. The reaction was allowed to stir overnight and precipitated in diethyl ether. The dry crude products were treated with TFA for 45 min at RT for tBu/tBoc deprotection and afterwards purified through HPLC to obtain the inhibitors PSMA 7-9 (Weineisen, M., et al., Synthesis and preclinical evaluation of DOTAGA-conjugated PSMA ligands for functional imaging and endoradiotherapy of prostate cancer. EJNMMI research, 2014. 4(1): p. 1; Weineisen, M., et al., 68Ga- and 177Lu-Labeled PSMA I&T: Optimization of a PSMA-Targeted Theranostic Concept and First Proof-of-Concept Human Studies. Journal of Nuclear Medicine, 2015. 56(8): p. 1169-1176).

**DOTAGA-y-nal-k(Suc-(HO-δ-orn-γ-EuE))-OH (PSMA-7):** HPLC (10 to 60 % B in 15 min): $t_R$ = 8.2 min K' = 7.8. Calculated monoisotopic mass ($C_{67}H_{92}N_{12}O_{26}$): 1,480.6 found: m/z = 1,481.7 $[M+H]^+$, 741.2 $[M+2H]^+$.

**DOTAGA-(4-NH$_2$)f-y-nal-k(Suc-(HO-δ-orn-γ-EuE))-OH (PSMA-8):** HPLC (25 % isocratic B in 20 min): $t_R$ = 10.5 min K' = 2.5. Calculated monoisotopic mass ($C_{76}H_{102}N_{14}O_{27}$): 1,642.7 found: m/z = 1,642.8 $[M+H]^+$, 822.8 $[M+2H]^{2+}$.

**DOTAGA-(4-NH$_2$)F-y-nal-k(Suc-(HO-δ-orn-γ-EuE))-OH (PSMA-9):** HPLC (25 % isocratic B in 20 min): $t_R$ = 11.3 min K' = 2.8. Calculated monoisotopic mass ($C_{76}H_{102}N_{14}O_{27}$): 1,642.7 found: m/z = 1,642.6 $[M+H]^+$, 822.3 $[M+2H]^{2+}$.

*$^{nat}$Lu-compounds*

The corresponding $^{nat}Lu^{III}$-complexes were prepared from a 2 mM aqueous solution of the PSMA inhibitor with a 2.5 molar excess of $LuCl_3$ (20 mM solution), heated to 95 °C for 30 min. After cooling, the $^{nat}Lu^{III}$-chelate formation was confirmed using HPLC and MS. The resulting 1 mM aqueous solutions of the respective $^{nat}$Lu-complexes were then diluted and used in the in vitro $IC_{50}$ studies without further processing.

**[$^{nat}$Lu]PSMA I&T ([$^{nat}$Lu]PSMA-1):** HPLC (25 to 55% in 15 min): DCM containing triisopropylsilane (TIPS) (50.0 eq) and (triphenyl)palladium(0) ($Pd(PPh_3)_4$) ( = 11.9 min, K' = 5.6. Calculated monoisotopic mass for $^{nat}$Lu-PSMA I&T ($C_{63}H_{89}IN_{11}O_{23}Lu$): 1,669.46 found: m/z = 1,669.9 $[M+H]^+$, 1,691.9 $[M+Na]^+$.

**[$^{nat}$Lu]DOTAGA-(4-NH$_2$)f-(I-y)-f-k-KuE ([$^{nat}$Lu]PSMA-2):** HPLC (10 to 60% B in 15 min): $t_R$ = 8.86 min, K' = 3.3. Calculated monoisotopic mass ($C_{72}H_{99}IN_{13}O_{24}Lu$): 1,831.5 found: m/z = 1833.2 $[M+H]^+$, 917.3 $[M+2H]^{2+}$

**[$^{nat}$Lu]DOTAGA-(4-NH$_2$)F-(I-y)-f-k-KuE ([$^{nat}$Lu]PSMA-3):** HPLC (10 to 50% B in 15 min): $t_R$ = 10.24 min, K' = 4.1. Calculated monoisotopic mass ($C_{72}H_{99}IN_{13}O_{24}Lu$): 1,831.5 found: m/z = 1832.7 $[M+H]^+$, 917.4 $[M+2H]^{2+}$

**[$^{nat}$Lu]DOTAGA-y-nal-k-KuE ([$^{nat}$Lu]PSMA-4):** HPLC (25 to 55% B in 15 min.): $t_R$ = 11.6 min K' = 8.7. Calculated monoisotopic mass ($C_{67}H_{92}N_{11}O_{23}$ Lu) = 1,593.6 found: m/z = 1,595.0 $[M+H]^+$, 798.2 $[M+2H]^{2+}$.

**[$^{nat}$Lu]DOTAGA-(4-NH$_2$)f-y-nal-k-KuE ([$^{nat}$Lu]PSMA-5):** HPLC (10 to 60% B in 15 min.): $t_R$ = 9.0 min K' = 3.5. Calculated monoisotopic mass ($C_{76}H_{102}N_{13}O_{24}$ Lu) = 1,755.7 found: m/z = 1,756.4 $[M+H]^+$, 879.0 $[M+2H]^{2+}$.

**[$^{nat}$Lu]DOTAGA-(4-NH$_2$)F-y-nal-k-KuE ([$^{nat}$Lu]PSMA-6):** HPLC (10 to 60% B in 15 min.): $t_R$ = 9.1 min K' = 3.6. Calculated monoisotopic mass ($C_{76}H_{102}N_{13}O_{24}$ Lu) = 1,755.7 found: m/z = 1,756.6 $[M+H]^+$, 878.8 $[M+2H]^{2+}$.

**[$^{nat}$Lu]DOTAGA-y-nal-k(Suc-(HO-δ-orn-γ-EuE))-OH ([$^{nat}$Lu]PSMA-7):** HPLC (10 to 60 % B in 15 min): $t_R$ = 7.8 min K' = 2.9. Calculated monoisotopic mass ($C_{67}H_{89}N_{12}O_{26}$ Lu): 1,652.5 found: m/z = 1,653.9 $[M+H]^+$.

**[$^{nat}$Lu]DOTAGA-(4-NH$_2$)f-y-nal-k(Suc-(HO-δ-orn-γ-EuE))-OH ([$^{nat}$Lu]PSMA-8):** HPLC (10 to 60 % B in 15 min): $t_R$ = 7.3 min K' = 2.7. Calculated monoisotopic mass ($C_{67}H_{99}N_{14}O_{27}Lu$): 1,814.6 found: m/z = 1,815.9 $[M+H]^+$, 908.3 $[M+2H]^{2+}$.

**[$^{nat}$Lu]DOTAGA-(4-NH$_2$)F-y-nal-k(Suc-(HO-δ-orn-γ-EuE))-OH ([$^{nat}$Lu]PSMA-9):** HPLC (10 to 60 % B in 15 min): $t_R$ = 7.2 min K' = 2.6. Calculated monoisotopic mass ($C_{67}H_{99}N_{14}O_{27}Lu$): 1,814.6 found: m/z = 1,815.6 $[M+H]^+$, 908.3 $[M+2H]^{2+}$.
**[$^{nat}$Lu]DOTAGA-y-nal-k(Glut-(I-f)-KuE) ([$^{nat}$Lu]PSMA-10):** HPLC (25 to 55% B in 15 min): $t_R$ = 10.6 min K' = 4.9. Calculated monoisotopic mass ($C_{73}H_{94}IN_{12}O_{24}Lu$): 1,825.5 found: m/z = 1,826.5 $[M+H]^+$, 913.8 $[M+2H]^{2+}$.
**[$^{nat}$Lu]DOTAGA-(4-NH$_2$)f-y-nal-k(Glut-(I-f)-KuE) ([$^{nat}$Lu]PSMA-11):** HPLC (10 to 60% B in 20 min): $t_R$ = 9.7 min K' = 3.9. Calculated monoisotopic mass ($C_{82}H_{104}IN_{14}O_{25}Lu$): 1,986.6 found: m/z = 1,987.5 $[M+H]^+$, 994.3 $[M+2H]^{2+}$.

Radiolabeling

*$^{68}$Ga-labeling*

The $^{68}$Ge/$^{68}$Ga generator was eluted with 1.0 M aqueous HCl, from which a fraction of 1.25 mL, containing approximately 80% of the activity (600 - 800 MBq), was transferred into a reaction vial (ALLTECH, 5 mL). The vial was beforehand loaded with 5 nmol of the respective compound and 950 μL of an aqueous HEPES solution (2.7 M). The reaction vial was heated for 5 min at 95°C with subseq.uent fixation of the radiolabeled compound on a preconditioned SPE cartridge (C8 light, SepPak). After purging the cartridge with 10 mL water in advance, the elution of the radiolabeled PSMA inhibitors from the cartridge was achieved with a 1:1 (v/v) mixture of EtOH and water, 1 mL phosphate buffered saline (PBS) and again 1 mL water. At the end of radiolabeling, the EtOH was evaporated in vacuo and the tracer used without any further purification. Radiochemical purity was controlled using Radio-TLC (1.0 M sodium citrate buffer and 0.06 M 1:1 $NH_4OAC$ /MeOH buffer).

*$^{177}$Lu-labeling*

The $^{177}$Lu-labeled compounds, for in vitro studies, were prepared as previously described (Sosabowski, J.K. and S.J. Mather, Conjugation of DOTA-like chelating agents to peptides and radiolabeling with trivalent metallic isotopes. Nat. Protocols, 2006. 1(2): p. 972-976) with minor modifications and used without further purification. In short, to a reaction volume of 10 μL 1.0 M $NH_4OAc$ buffer pH = 5.9 was added 0.75 to 1.0 nmol tracer (7.5 to 10 μL), 10 to 40 MBq $^{177}LuCl_3$ (Specific Activity ($A_S$) > 3000 GBq/mg, 740 MBq/mL, 0.04 M HCl, ITG, Garching, Germany) and finally filled up to 100 μL with trace-pure Water (Merck, Darmstadt, Germany). The reaction mixture was heated for 30 min at 95°C and the radiochemical purity was determined using Radio-TLC.

*$^{125}$I-labeling*

Briefly, 0.1 mg of the stannylated precursor ($SnBu_3$-BA)(OtBu)KuE$(OtBu)_2$ was dissolved in a solution containing 20 μL peracetic acid, 5.0 μL (21.0 MBq) [$^{125}$I]NaI (74 TBq/mmol, 3.1 GBq/mL, 40 mM NaOH, Hartmann Analytic, Braunschweig, Germany), 20 μL MeCN and 10 μL acetic acid. The reaction solution was incubated for 10 min at RT, loaded on a cartridge and rinsed with 10 mL water (C18 Sep Pak Plus cartridge, preconditioned with 10 mL MeOH and 10 mL water). After elution with 2.0 mL of a 1:1 mix (v/v) of EtOH/MeCN, the radioactive solution was evaporated to dryness under a gentle nitrogen stream and treated with 200 μL TFA for 30 min with subsequent evaporation of TFA. The crude product of ([$^{125}$I]I-BA)KuE was purified by RP-HPLC (20% to 40% B in 20 min): $t_R$ = 13.0 min; K' = 6.2.

In vitro experiments

*Determination of $IC_{50}$*

After removal of the culture medium, the cells were treated once with 500 μL of HBSS (Hank's balanced salt solution, Biochrom, Berlin, Germany, with addition of 1% bovine serum albumin (BSA)) and left 15 min on ice for equilibration in 200 μL HBSS (1% BSA). Next, 25 μL per well of solutions, containing either HBSS (1% BSA, control) or the respective ligand in increasing concentration ($10^{-10}$ - $10^{-4}$ M in HBSS (1% BSA)), were added with subsequent addition of 25 μL of ([$^{125}$I]I-BA)KuE (2.0 nM) in HBSS (1% BSA). All experiments were performed at least three times for each concentration. After 60 min incubation on ice, the experiment was terminated by removal of the medium and consecutive rinsing with 200 μL of HBSS. The media of both steps were combined in one fraction and represent the amount of free radioligand. Afterwards, the cells were lysed with 250 μL of 1.0 M NaOH and united with the 200 μL HBSS of the following wash step. Quantification of bound and free radioligand was accomplished in a γ-counter.

*Internalization*

Subsequent to the removal of the culture medium, the cells were washed once with 500 μL DMEM-F12 (5%

BSA) and left to equilibrate for at least 15 min at 37°C in 200 μL DMEM-F12 (5% BSA). Each well was treated with either 25 μL of either DMEM-F12 (5% BSA) or a 100 μM PMPA solution for blockade. Next, 25 μL of the respective $^{68}Ga$-and $^{177}Lu$-labeled PSMA inhibitors (2.0 nM / 5.0 nM) was added and the cells incubated at 37°C for 5, 15, 30 and 60 min, respectively. The experiment was terminated by placing the 24-well plate on ice for 3 min and consecutive removal of the medium. Each well was rinsed with 250 μL HBSS and the fractions from these first two steps combined, representing the amount of free radioligand. Removal of surface bound activity was accomplished by incubation of the cells with 250 μL of ice-cold PMPA (10 μM in PBS) solution for 5 min and rinsed again with another 250 μL of ice-cold PBS. The internalized activity was determined by incubation of the cells in 250 μL 1.0 M NaOH and the combination with the fraction of a subsequent wash step with 250 μL 1.0 M NaOH. Each experiment (control and blockade) was performed in triplicate for each time point. Free, surface bound and internalized activity was quantified in a γ-counter.

*HSA binding*

The mobile phase was freshly prepared for each experiment and only used for one day. The column was kept at room temperature and each run was stopped after detection of the signal to reduce the acquisition time. All substances were dissolved in a 0.5mg/ml concentration in 50% 2-Propanol and 50% 50mM pH 6.9 ammonium acetate buffer. The chosen reference substances display a range of HSA binding from 13% to 99% since a broad variety of albumin binding regarding the peptides was assumed. All nine reference substances were injected consecutively to establish a non-linear regression with OriginPro 2016G. The literature HSA binding [%] was obtained from Valko et. al. or Yamazaki et al. (Yamazaki, K. and M. Kanaoka, Computational prediction of the plasma protein-binding percent of diverse pharmaceutical compounds. Journal of pharmaceutical sciences, 2004. 93(6): p. 1480-1494). Figure 1 shows the result of the calibration.

**Table 1:** Reference substances used for the calibration of the HSA-column.

| Reference | $t_R$ | Log $t_R$ | Lit. HSA% | Log K HSA |
|---|---|---|---|---|
| p-benzylalcohol | 2.40 | 0.38 | 13.15 | -0.82 |
| Aniline | 2.72 | 0.43 | 14.06 | -0.79 |
| Phenol | 3.28 | 0.52 | 20.69 | -0.59 |
| Benzoic Acid | 4.08 | 0.61 | 34.27 | -0.29 |
| Carbamazepine | 4.15 | 0.62 | 75.00 | 0.46 |
| p-nitrophenol | 5.62 | 0.75 | 77.65 | 0.52 |
| Estradiol | 8.15 | 0.91 | 94.81 | 1.19 |
| Probenecid | 8.84 | 0.95 | 95.00 | 1.20 |
| Glibenclamide | 29.18 | 1.47 | 99.00 | 1.69 |

The retention time is shown exemplary for a conducted experiment; $t_R$ retention time; Lit. HSA literature value of human serum albumin binding in [%]; Log K HAS logarithmic K of human serum albumin binding

*Lipophilicity*

To a volume of 500 μL n-octanol, 0.5 to 1.0 MBq of the radiolabeled PSMA inhibitor in 500 μL PBS (pH = 7.4) was added and rigorously vortexted for 3 min (n = 6). For quantitative phase separation, the mixture was centrifuged at 6,000 g for 5 min (Biofuge 15, Heraus Sepatech, Osterode, Germany). The activity from samples of 100 μL of each phase were measured in a γ-counter to obtain the $logP_{(o/w)}$ value.

In vivo experiments

All animal experiments were carried out in accordance with the general animal welfare regulations in Germany (Deutsches Tierschutzgesetz, approval #55.2-1-54-2532-71-13). For the tumor model, LNCaP cells (approx. $10^7$ cells) were suspended 1:1 in serum-free DMEM-F12 medium and Matrigel (BD Biosciences, Germany) and inoculated onto the right shoulder of male, 6 to 8 weeks old CB-17 SCID mice (Charles River Laboratories, Sulzfeld, Germany). Animals were used after the tumor size reached 4 to 8 mm in diameter for experiments.

*Metabolic stability*

25 MBq of [$^{177}Lu$]PSMA (k,f,(3-i)y,(4-NH2)f) ([$^{177}Lu$]PSMA-**2**) was injected into the tail vein of a healthy CB17-SCID mouse, which was sacrificed after 60 min. Samples of the urine and blood were immediately taken. Kidneys were frozen with liquid nitrogen, homogenized and extracted with 500 μL PMPA Solution (400 μM in PBS). After centrifugation at 15,000 g for 5 min, the suspension was ultra-filtrated and analyzed by radio-HPLC. The blood sample was centrifuged at 6,000 g for 5 min to obtain the plasma. To remove the proteins from the plasma, ice-cold MeCN was added to the sample and incubated for 10 min at 4°C. After concomitant centrifugation and ultrafiltration, the sample was analyzed by radio-HPLC. The urine sample was used without any further purification.

*μPET imaging*

Imaging experiments were conducted using a Siemens Inveon small animal PET and data analyzed by the associated Inveon Research Workplace software. Mice were anaesthetised with isoflurane and the $^{68}$Ga-radiolabeled PSMA inhibitors (0.2 nmol, 7.0 to 12 MBq) were injected via tail vein. Dynamic imaging was carried out after on-bed injection for 90 min. The static blockade image was obtained, after 1 h p.i. with 15 min acquisition time. Blockade was done by coinjection of 8mg/kg of PMPA. All images were reconstructed using an OSEM3D algorithm without scanner and attenuation correction.

*Biodistribution*

Approximately 4.0 to 11.0 MBq (0.2 nmol) of the $^{177}$Lu-labeled PSMA Inhibitors were injected into the tail vein of LNCaP tumor-bearing male CB-17 SCID mice and sacrificed after 1 h and 24 h (n = 4, respectively). Selected organs were removed, weighted and measured in a γ-counter.

**Example 2: Results**

<u>Structures of PSMA Inhibitors</u>

PSMA-2, PSMA-3, PSMA-5, PSMA-6, PSMA-8, PSMA-9, and PSMA-11 are especially preferred compounds of the invention.

PSMA-4, PSMA-7 and PSMA-10 are used as reference compounds for the quantitative assessment of the performance of compounds of the invention.

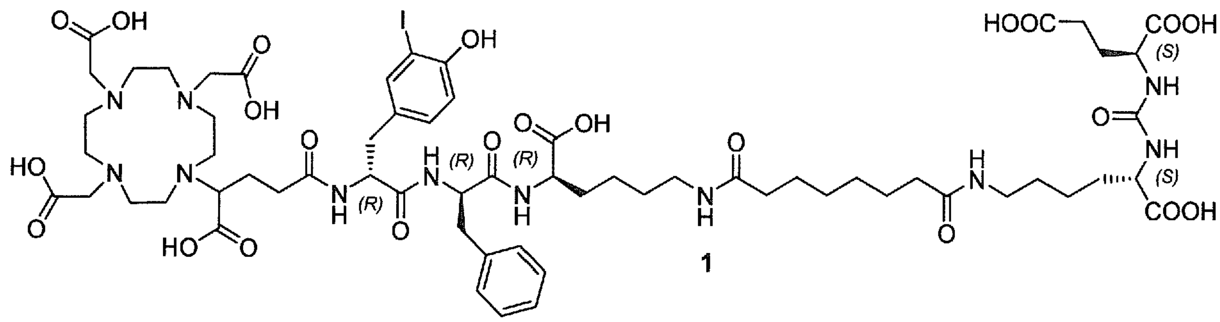

**1**

Chemical Formula: $C_{63}H_{92}IN_{11}O_{23}$

Molecular Weight: 1498,39

PSMA I&T ; parent compound **(PSMA-1)**

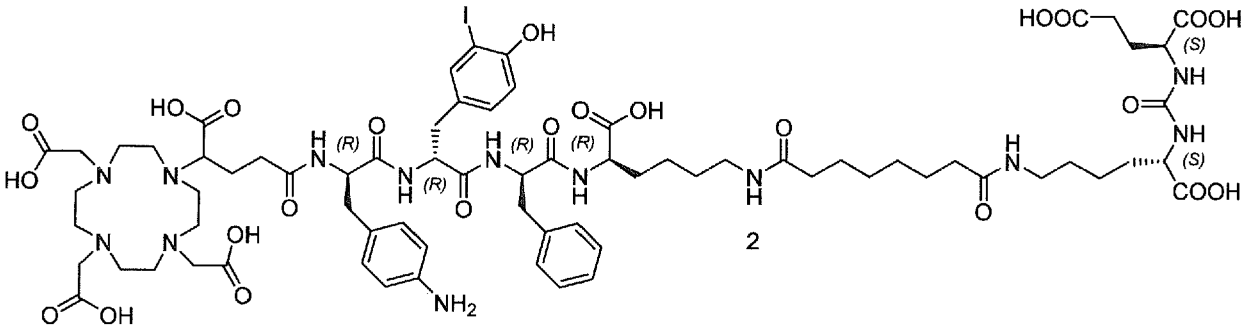

2

Chemical Formula: $C_{72}H_{102}IN_{13}O_{24}$
Molecular Weight: 1660,58

DOTAGA-(4-NH2)f-(I-y)-f-k-KuE **(PSMA-2)**

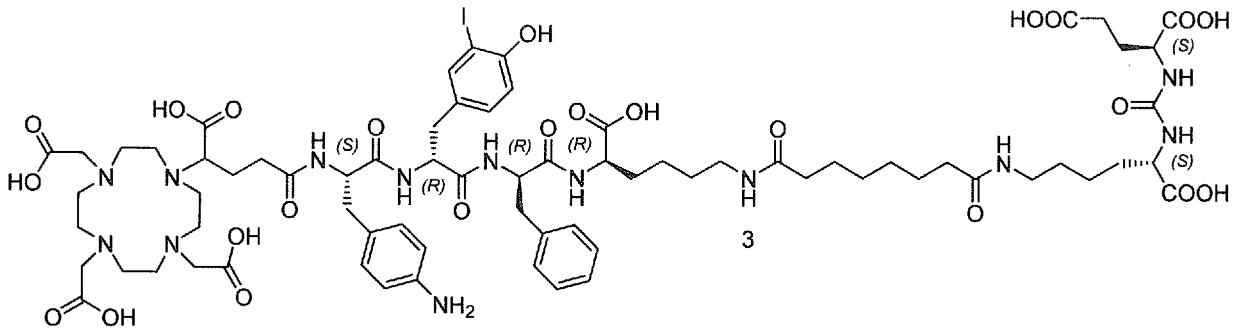

3

Chemical Formula: $C_{72}H_{102}IN_{13}O_{24}$
Molecular Weight: 1660,58

DOTAGA-(4-NH2)F-(I-y)-f-k-KuE **(PSMA-3)**

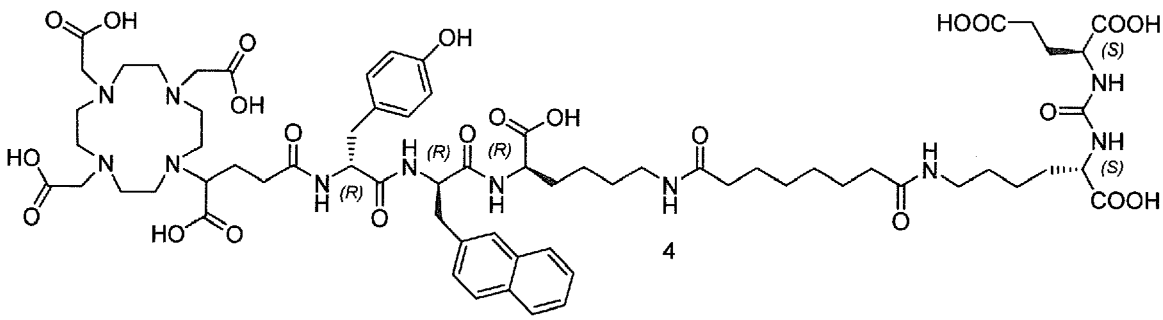

4

Chemical Formula: $C_{67}H_{95}N_{11}O_{23}$
Molecular Weight: 1422,55

DOTAGA-γ-nal-k-KuE **(PSMA-4)**

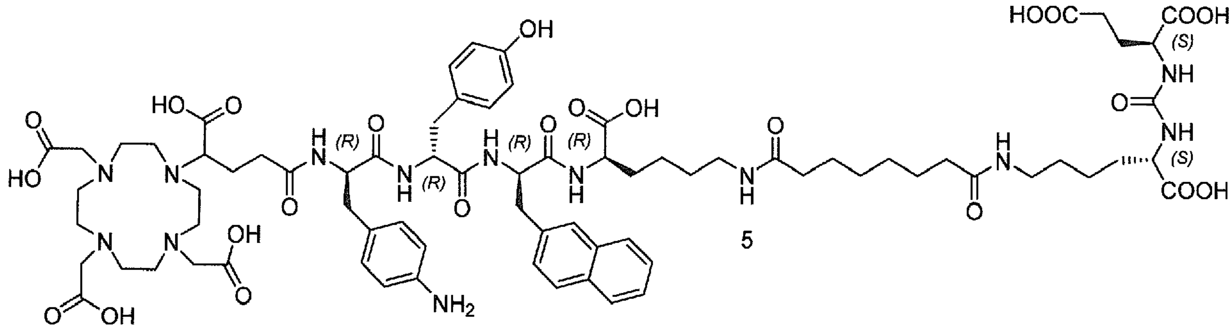

5

Chemical Formula: $C_{76}H_{105}N_{13}O_{24}$
Exact Mass: 1583,74

DOTAGA-(4-NH2)f-y-nal-k-KuE **(PSMA-5)**

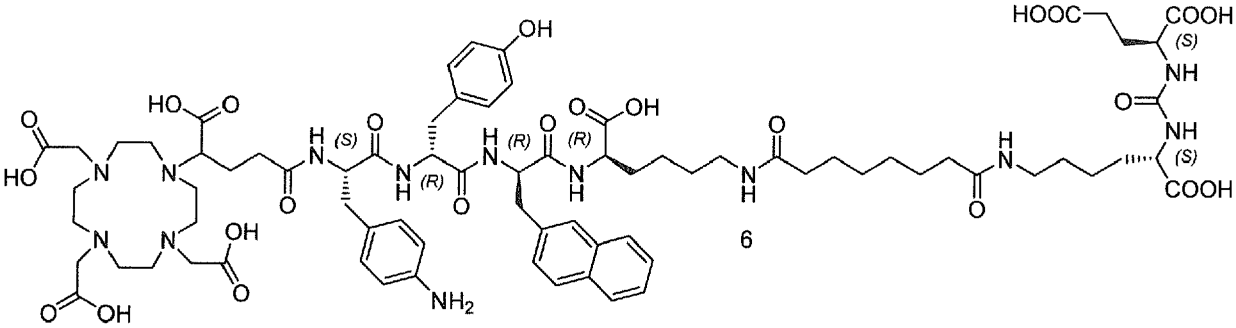

6

Chemical Formula: $C_{76}H_{105}N_{13}O_{24}$
Molecular Weight: 1584,74

DOTAGA-(4-NH2)F-y-nal-k-KuE **(PSMA-6)**

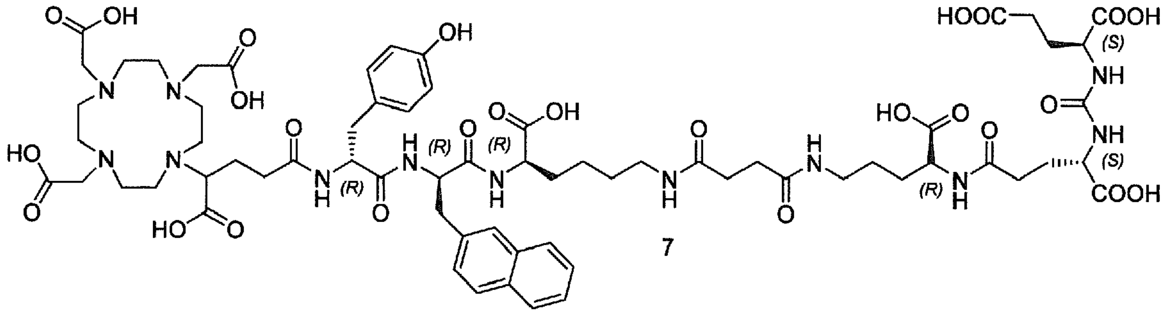

7

Chemical Formula: $C_{67}H_{92}N_{12}O_{26}$
Molecular Weight: 1481,53

DOTAGA-y-nal-k(Suc-(HO-6-orn-γ-EuE))-OH **(PSMA-7)**

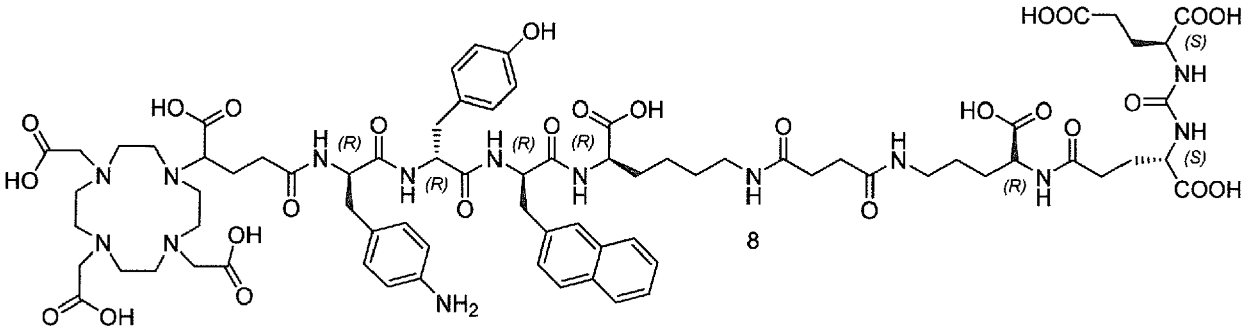

8

Chemical Formula: $C_{76}H_{102}N_{14}O_{27}$
Molecular Weight: 1643,72

DOTAGA-(4-NH2)f-y-na)-k(Suc-(HO-δ-orn-γ-EuE))-OH **(PSMA-8)**

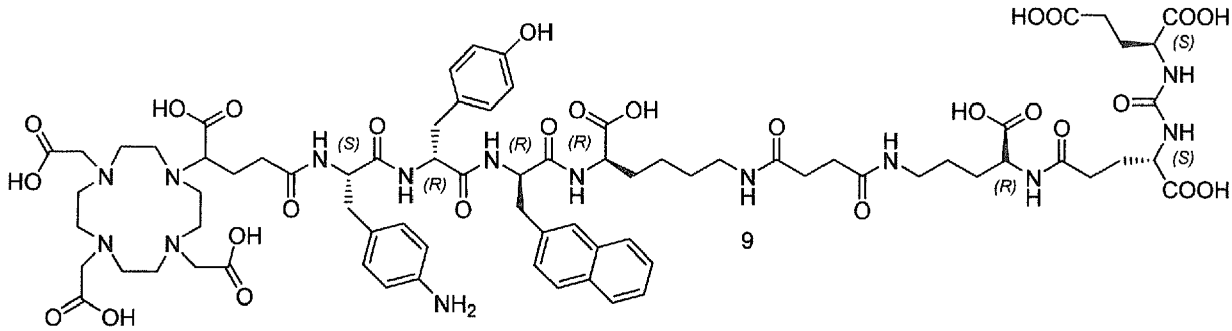

9

Chemical Formula: $C_{76}H_{102}N_{14}O_{27}$
Molecular Weight: 1643,72

DOTAGA-(4-NH2)F-y-nal-k(Suc-(HO-δ-orn-γ-EuE))-OH **(PSMA-9)**

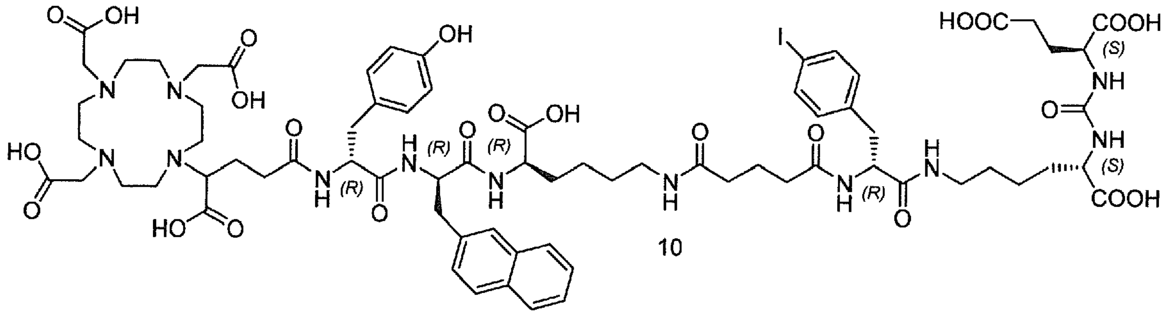

10

Chemical Formula: $C_{73}H_{97}IN_{12}O_{24}$
Molecular Weight: 1653,54

DOTAGA-y-nal-k(Glut-(I-f)-KuE) **(PSMA-10)**

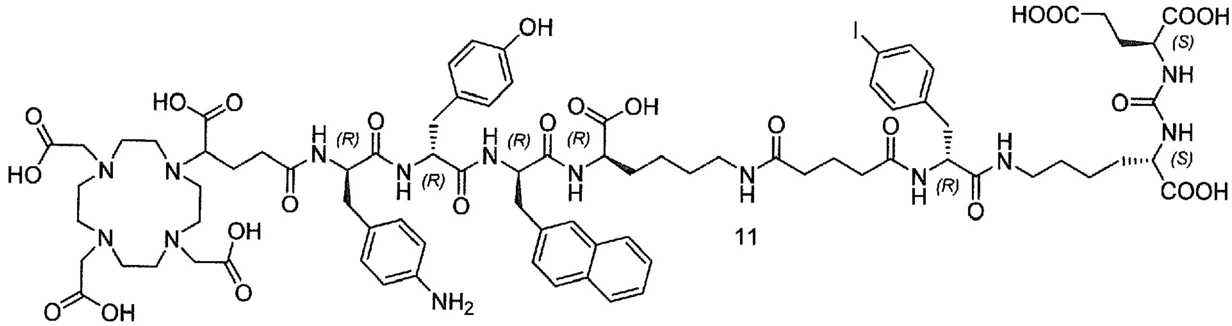

11

Chemical Formula: $C_{82}H_{107}IN_{14}O_{25}$
Molecular Weight: 1815,74

DOTAGA-(4-NH2)f-y-nal-k(Glut-(I-f)-KuE) **(PSMA-11)**

In vitro data of the investigated PSMA Inhibitors and metabolite analysis of [$^{177}$Lu]PSMA-2

See Figure 2 for radio-HPLC of analyses from homogenized organs and body fluids from mice after 60 min p.i.

*Binding affinities*

**Table 2**: PSMA binding affinities ($IC_{50}$) and internalization [%] of the investigated compounds

| **PSMA Inhibitor** | | **$IC_{50}$ [nM]** | **Internalization [%]** |
|---|---|---|---|
| [$^{nat/177}$Lu]PSMA I&T | [$^{nat/177}$Lu]PSMA-**1** | 7.9 ± 2.4 | 75.5 ± 1.6 |
| [$^{nat/177}$Lu]PSMA (k,f,(3-i)y,**(4-NH$_2$)f)** | [$^{nat/177}$Lu]PSMA-**2** | 2.3 ± 0.6 | 122.15 ± 1.3 |
| [$^{nat/177}$Lu]PSMA (k,f,(3-i)y,**(4-NH$_2$)F)** | [$^{nat177}$Lu]PSMA-**3** | 2.8 ± 0.3 | 166.92 ± 2.4 |
| [$^{nat/177}$Lu]PSMA (k,nal,y) | [$^{nat177}$Lu]PSMA-**4** | 2.1 ± 0.8 | 71.7 ± 0.7 |
| [$^{nat/177}$Lu]PSMA (k,nal,**(4-NH$_2$)f)** | [$^{nat/177}$Lu]PSMA-**5** | 5.8 ± 1.0 | 111.3 ± 11.1 |
| [$^{nat/177}$Lu]PSMA (k,nal,**(4-NH$_2$)F)** | [$^{nat/177}$Lu]PSMA-**6** | 1.1 ± 0.1 | 108.8 ± 2.5 |
| [$^{nat/177}$Lu]PSMA (EuE,k,nal,y) | [$^{nat/177}$Lu]PSMA-**7** | 3.1 ± 1.0 | 216.2 ± 9.2 |
| [$^{nat/177}$Lu]PSMA (EuE,k,nal,**(4-NH$_2$)f)** | [$^{nat/177}$Lu]PSMA-**8** | 2.5 ± 0.6 | 207.3 ± 8.5 |
| [$^{nat/177}$Lu]PSMA (EuE,k,nal,**(4-NH$_2$)F)** | [$^{nat/177}$Lu]PSMA-**9** | 2.5 ± 0.6 | 245,0 ± 4.2 |
| [$^{nat/177}$Lu]PSMA (EuK,(4-i)f,k,nal,y) | [$^{nat/177}$Lu]PSMA-**10** | 6.1 ± 1.6 | 118.6 ± 0.5 |
| [$^{nat/177}$Lu]PSMA (EuK,(4-i)f,k,nal,**(4-NH$_2$)f)** | [$^{nat177}$Lu]PSMA-**11** | 6.8 ± 3.2 | 155.1 ± 7.9 |

*Lipophilicities*

**Table 3**: Lipophilicity shown as logP and binding [%] to human serum albumin (HSA) of the PSMA inhibitors

| **PSMA Inhibitor** | | **logP** | **HSA [%]** |
|---|---|---|---|
| [$^{177/nat}$Lu]PSMA I&T | [$^{nat/177}$Lu]PSMA-**1** | -4.12 ± 0.1 | 78.6 |
| [$^{nat/177}$Lu]PSMA (k,f,(3-i)y,**(4-NH2)f)** | [$^{nat/177}$Lu]PSMA-**2** | -4.11 ± 0.06 | 82.5 |
| [$^{nat/177}$Lu]PSMA (k,f,(3-i)y,**(4-NH$_2$)F)** | [$^{nat/177}$Lu]PSMA-**3** | n.d. | 91.9 |
| [$^{nat/177}$Lu]PSMA (k,nal,y) | [$^{nat/177}$Lu]PSMA-**4** | -4.11 ± 0.06 | 82.6 |
| [$^{nat/177}$Lu]PSMA (k,nal,**(4-NH$_2$)f)** | [$^{nat/177}$Lu]PSMA-**5** | -4.11 ± 0.06 | n.d. |
| [$^{nat/177}$Lu]PSMA (k,nal,**(4-NH$_2$)F)** | [$^{nat/177}$Lu]PSMA-**6** | n.d. | 83.17 |
| [$^{nat/177}$Lu]PSMA (EuE,k,nal,y) | [$^{nat/177}$Lu]PSMA-**7** | n.d. | n.d. |
| [$^{nat/177}$Lu]PSMA (EuE,k,nal,**(4-NH$_2$)f)** | [$^{nat/177}$Lu]PSMA-**8** | -4.02 ± 0,25 | n.d. |

(continued)

| PSMA Inhibitor | | logP | HSA [%] |
|---|---|---|---|
| $[^{nat/177}Lu]$PSMA (EuE,k,nal,**(4-NH$_2$)F)** | $[^{nat/177}Lu]$PSMA-**9** | -4.01 ± 0.11 | n.d. |
| $[^{nat/177}Lu]$PSMA (EuK,(4-i)f,k,nal,y) | $[^{nat/177}Lu]$PSMA-**10** | -3.05 ± 0.02 | 96.61 |
| $[^{nat/177}Lu]$PSMA (EuK,(4-i)f,k,nal,**(4-NH$_2$) f)** | $[^{nat/177}Lu]$PSMA-**11** | -3.47 ± 0.24 | 97.63 |

μPET-Imaging of $^{68}$Ga-labeled PSMA Inhibitors 1-11 in LNCaP Xenograft bearing CB-17 SCID mice

Figures 3 to 10 show the results of μPET-Imaging.

Biodistribution

Figures 11 to 14 show biodistribution data.

## Claims

1. A compound of formula (I), or a pharmaceutically acceptable salt thereof,

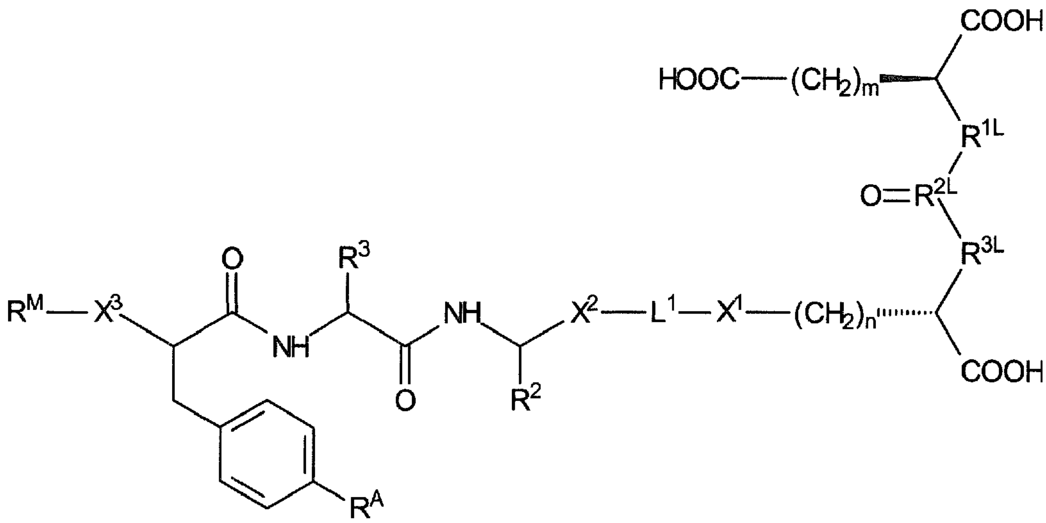

(I)

wherein:

m is an integer of 2 to 6;
n is an integer of 2 to 6;
$R^{1L}$ is $CH_2$, NH or O;
$R^{2L}$ is C or P(OH);
$R^{3L}$ is $CH_2$, NH or O;
$X^1$ is selected from an amide bond, an ether bond, a thioether bond, an ester bond, a thioester bond, a urea bridge, an amine bond, and a group of the formula -C(O)-NH-CH($R^1$)-C(O)-NH-,
wherein $R^1$ is a group -$CH_2$-phenyl which is optionally substituted on its aromatic ring with a substituent selected from -OH and -I;
$L^1$ is a divalent linking group with a structure selected from an oligoamide, an oligoether, an oligothioether, an oligoester, an oligothioester, an oligourea, an oligo(ether-amide), an oligo(thioether-amide), an oligo(ester-amide), an oligo(thioester-amide), oligo(urea-amide), an oligo(ether-thioether), an oligo(ether-ester), an oligo(ether-thioester), an oligo ether-urea), an oligo(thioether-ester), an oligo(thioether-thioester), an oligo(thioether-urea), an oligo(ester-thioester), an oligo(ester-urea), and an oligo(thioester-urea);
$X^2$ is selected from an amide bond, an ether bond, a thioether bond, an ester bond, a thioester bond, a urea

bridge, and an amine bond;

$R^2$ is an aryl group or an aralkyl group, which aryl group or aralkyl group may be substituted on its aromatic ring with a substituent selected from halogen and -OH;

$R^3$ is an aryl group or an aralkyl group, which aryl group or aralkyl group may be substituted on its aromatic ring with a substituent selected from halogenand -OH;

$R^A$ is $-NH_2$;

$X^3$ is selected from an amide bond, an ether bond, a thioether bond, an ester bond, a thioester bond, a urea bridge, an amine bond, and a group of the formula

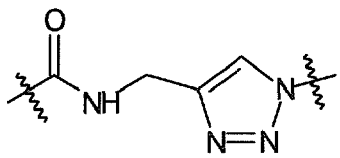

,

wherein the marked bond at the carbonyl group attaches $X^3$ to $R^M$ and the other marked bond attaches $X^3$ to the remainder of the compound of formula (I);

$R^M$ is a marker group which comprises a chelating group optionally containing a chelated non-radioactive or radioactive cation.

**2.** The compound or salt of claim 1, wherein m is 2, n is 2 or 4, $R^{1L}$ is NH, $R^{2L}$ is C, and $R^{3L}$ is NH.

**3.** The compound or salt of claim 1 or 2, wherein $L^1$ is a divalent linking group with a structure selected from an oligoamide and an oligo(ester-amide), which comprises a total of 1 to 5bonds selected from amide and ester bonds within its backbone.

**4.** The compound or salt of any of claims 1 to 3, wherein the moiety $-X^2-L^1-X^1-$ has a structure selected from:

$$*-C(O)-NH-R^4-NH-C(O)-R^5-C(O)-NH- \quad (L-1),$$

$$*-C(O)-NH-R^6-NH-C(O)-R^7-C(O)-NH-R^8-NH-C(O)- \quad (L-2),$$

and

$$*-C(O)-NH-R^9-NH-C(O)-R^{10}-C(O)-NH-CH(R^1)-C(O)-NH- \quad (L-3);$$

wherein the bond marked with * is attached to the carbon atom carrying $R^2$ in formula (I), and wherein $R^4$ to $R^{10}$ are independently selected from C2 to C10 alkanediyl, which alkanediyl groups may each be substituted by one or more substitutents independently selected from -OH, $-OCH_3$, -COOH, $-COOCH_3$, $-NH_2$, and $-NHC(NH)NH_2$, and $R^1$ is a group $-CH_2$-phenyl which is optionally substituted on its aromatic ring with a substituent selected from -OH and -I.

**5.** The compound or salt of claim 4, wherein the moiety $-X^2-L^1-X^1-$ has a structure selected from:

$$*-C(O)-NH-CH(COOH)-R^{11}-NH-C(O)-R^{12}-C(O)-NH- \quad (L-4),$$

$$*-C(O)-NH-CH(COOH)-R^{13}-NH-C(O)-R^{14}-C(O)-NH-R^{15}-CH(COOH)-NH-C(O)- \quad (L-5),$$

and

$$*-C(O)-NH-CH(CO0H)-R^{16}-NH-C(O)-R^{17}-C(O)-NH-CH(R^1)-C(O)-NH- \quad (L-6);$$

wherein the bond marked with * is attached to the carbon atom carrying $R^2$ in formula (I), and wherein $R^{11}$, $R^{13}$ and $R^{16}$ are independently selected from linear C2 to C6 alkanediyl, $R^{12}$ is a linear C2 to C10 alkanediyl-alkanediyl,

$R^{14}$, $R^{15}$ and $R^{17}$ are independently selected from linear C2 to C6 alkanediyl,

and $R^1$ is a group $-CH_2$-phenyl which is optionally substituted on its aromatic ring with a substituent selected from

-OH and -I.

**6.** The compound or salt of any of claims 1 to 5, wherein $R^2$ is a group of the formula

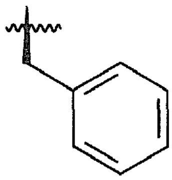

or

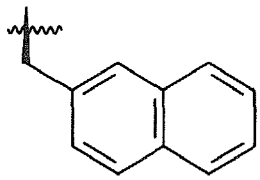

and $R^3$ is a group of the formula

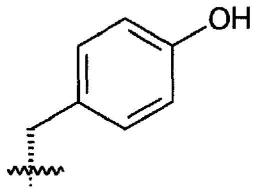

or

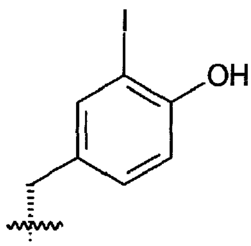

wherein 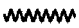 marks the bond which attaches $R^2$ and $R^3$, respectively, to the remainder of the compound of formula (I).

**7.** The compound or salt of any of claims 1 to 6, wherein $X^3$ is an amide bond or a group of the formula

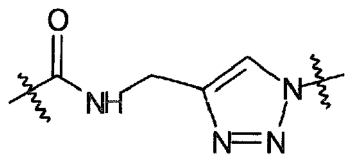

,

wherein the marked bond at the carbonyl group attaches $X^3$ to $R^M$ and the other marked bond attaches $X^3$ to the remainder of the molecule.

**8.** The compound or salt of claim 7, wherein $X^3$ is an amide bond -C(O)-NH- with the carbon atom being attached to $R^M$.

**9.** The compound or salt of any of claims 1 to 8, wherein the chelating group comprises at least one of

(i) a macrocyclic ring structure with 8 to 20 ring atoms of which 2 or more are selected from oxygen atoms, sulfur atoms and nitrogen atoms; and
(ii) an acyclic, open chain chelating structure with 8 to 20 main chain atoms of which 2 or more are heteroatoms selected from oxygen atoms, sulfur atoms and nitrogen atoms.

**10.** The compound or salt of claims 9, wherein $R^M$-$X^3$- is a group of the formula

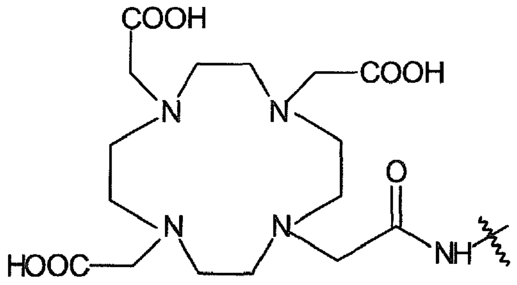

(M-1),

or

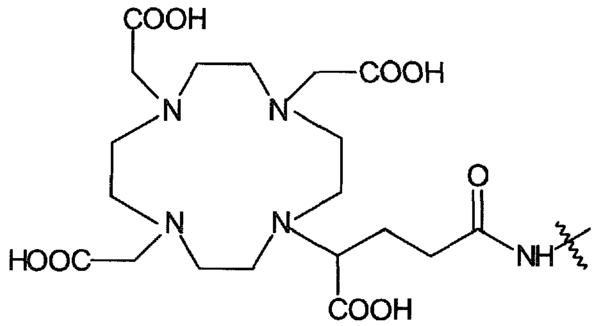

(M2)

wherein the bond marked with  is attached to the remainder of the compound of formula (I), and wherein the chelating group may contain a chelated non-radioactive or radioactive cation.

**11.** The compound of any of claims 1 to 10, which has the formula (Ia) or (Ib), or a pharmaceutically acceptable salt thereof:

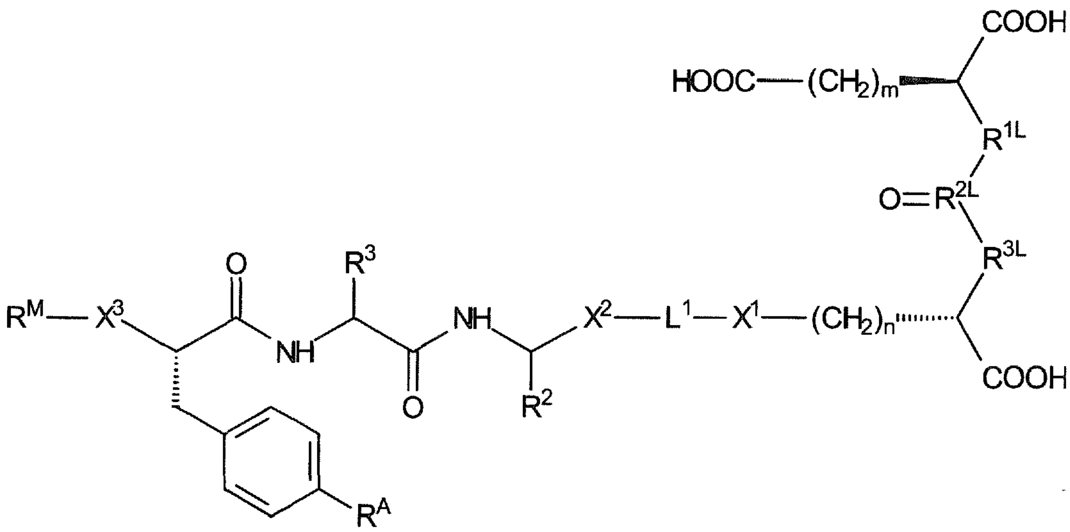

(Ia)

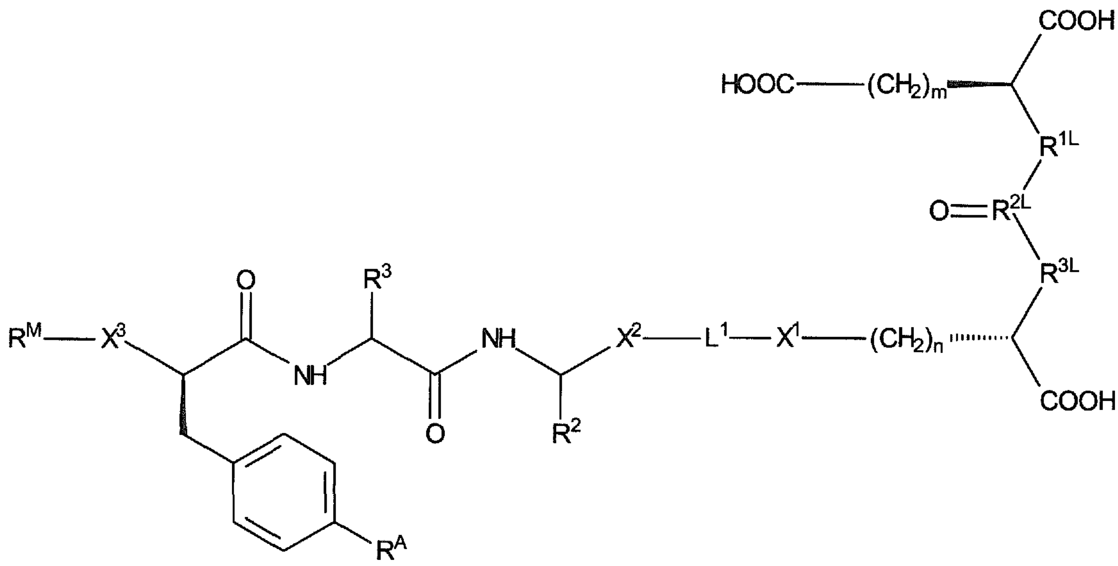

(Ib)

wherein $R^{1L}$, $R^{2L}$, $R^{3L}$, m, n, $X^1$, $L^1$, $X^2$, $R^2$, $R^3$, $R^A$, $X^3$ and $R^M$ are defined as in the preceding claims.

**12.** The compound or salt of claim 11, which has the formula (Ig) or (Ih), or a pharmaceutically acceptable salt thereof:

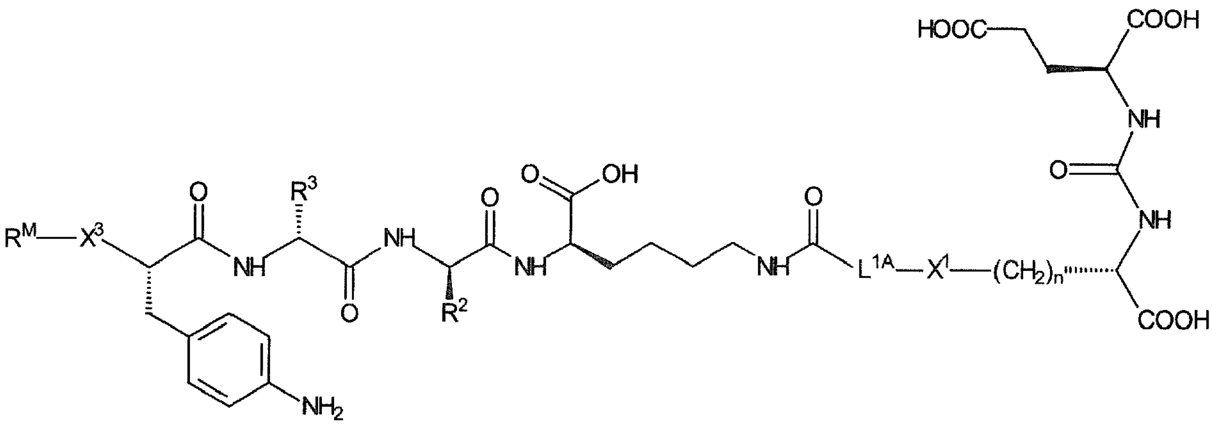

(Ig)

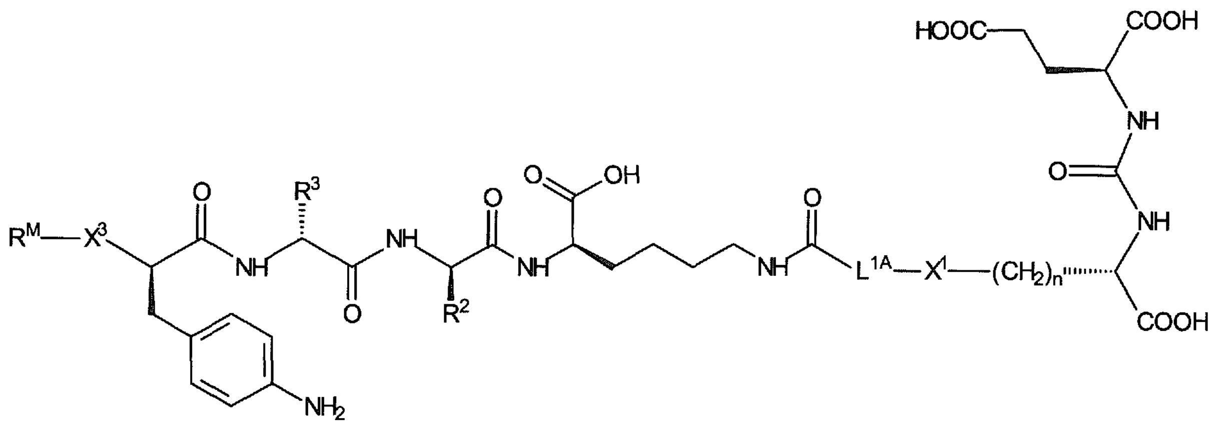

(Ih)

wherein n, $R^2$, $R^3$, $X^3$ and $R^M$ are defined as in the preceding items, and wherein the moiety $-L^{1A}-X^1-$ has a structure selected from:

$-R^{12A}-C(O)-NH-$ (L-7),

$-R^{14A}-C(O)-NH-R^{15A}-CH(COOH)-NH-C(O)-$ (L-8),

and

$-R^{17A}-C(O)-NH-CH(R^1)-C(O)-NH-$ (L-9);

wherein the bond at $R^{12A}$, $R^{14A}$ and $R^{17A}$, respectively, is attached to the carbon atom carrying the carbonyl group in formula (I), and wherein

$R^{12A}$ is a linear C2 to C10 alkanediyl, preferably a linear C4 to C8 alkanediyl,

$R^{14A}$, and $R^{15A}$ and $R^{17A}$ are independently selected from linear C2 to C6 alkanediyl, and $R^1$ is a group $-CH_2$-phenyl which is optionally substituted on its aromatic ring with a substituent selected from -OH and -I.

**13.** The compound or salt of any one of the preceding claims, wherein said compound or salt has one of the following formulae:

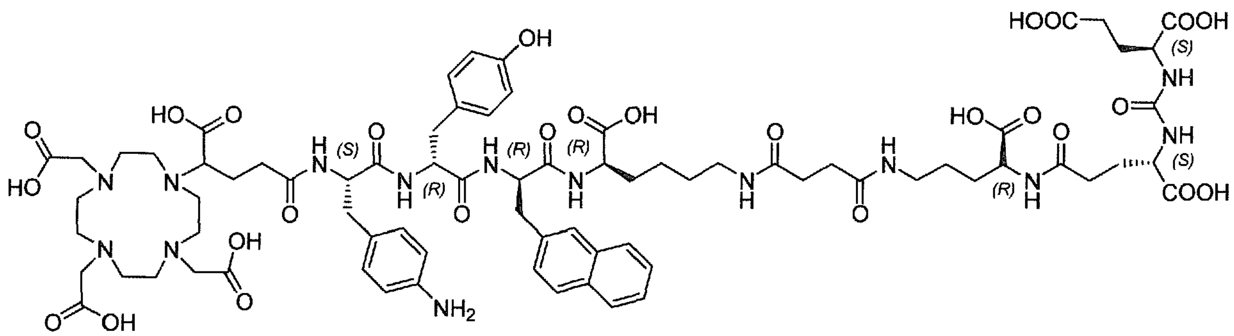

(Ii)

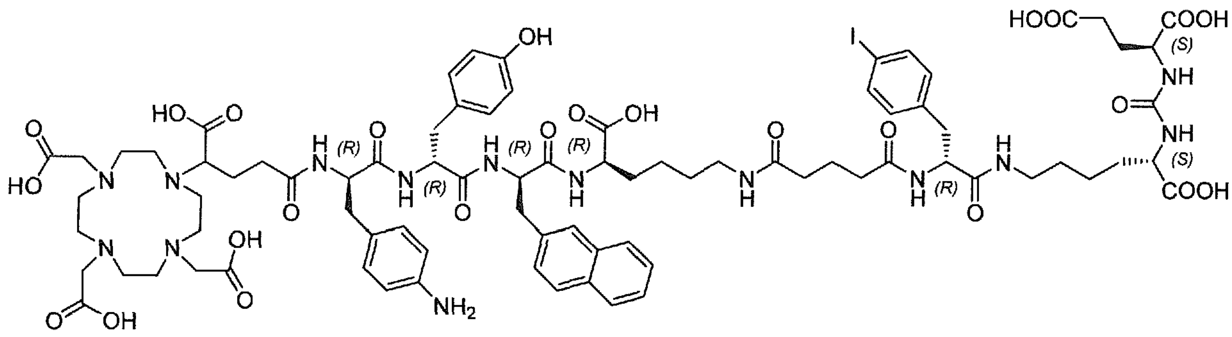

(Ij).

14. A pharmaceutical or diagnostic composition comprising or consisting of one or more compounds or salts in accordance with any one of the preceding claims.

15. A compound or salt in accordance with any one of claims 1 to 13 for use in a method of diagnosing and/or treating

(a) cancer including prostate cancer; or
(b) neoangiogenesis/angiogenesis.

**Figure 1**

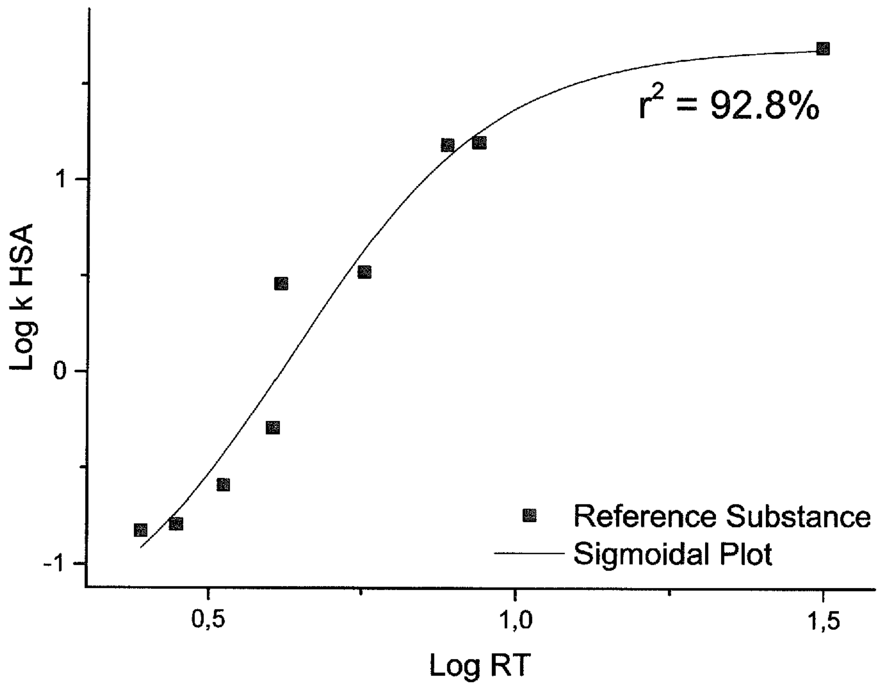


**Figure 2**

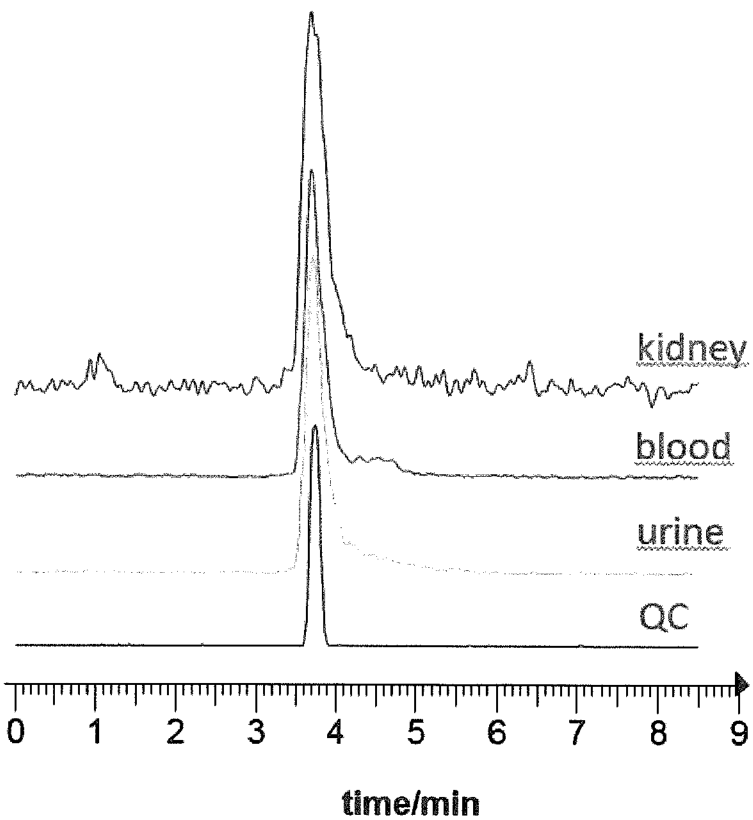

Figure 3

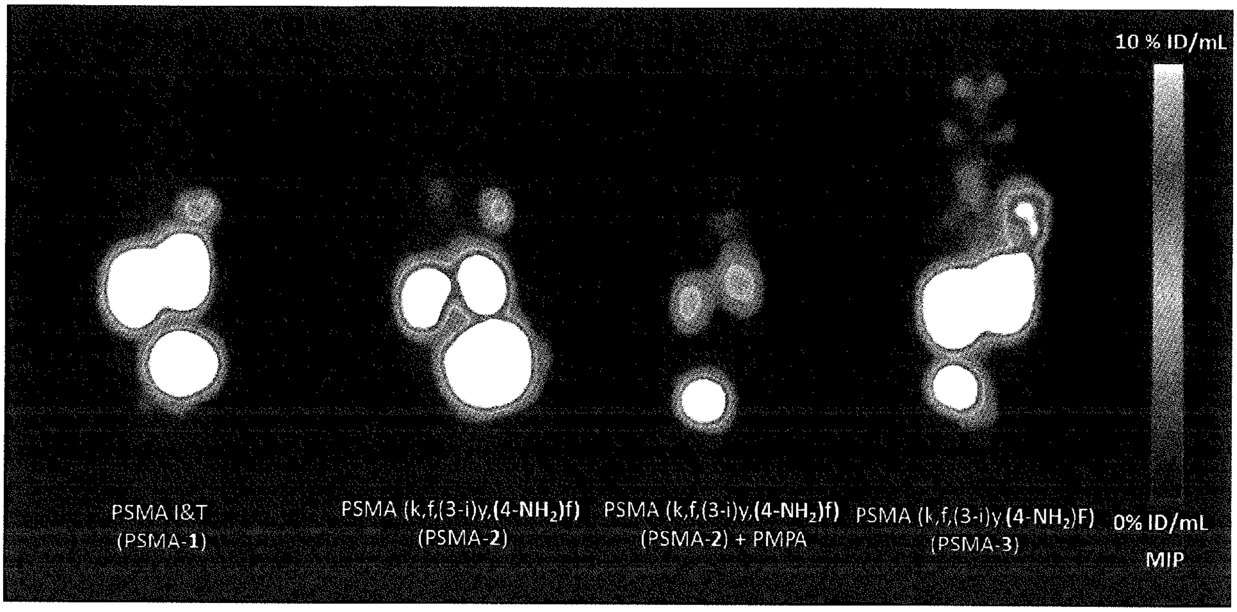


Figure 4

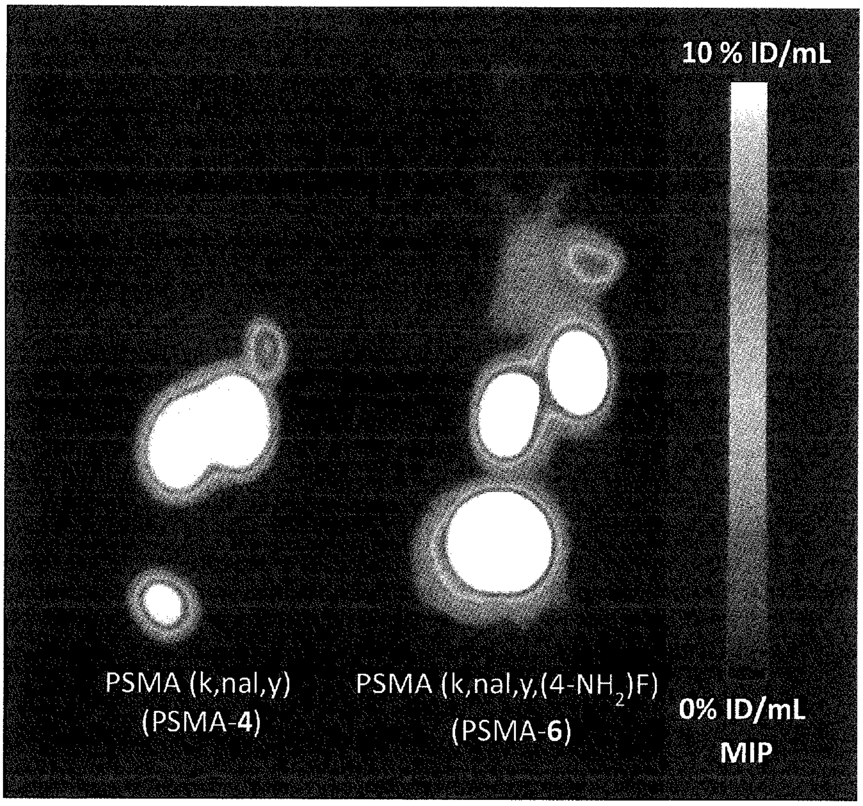

**Figure 5**

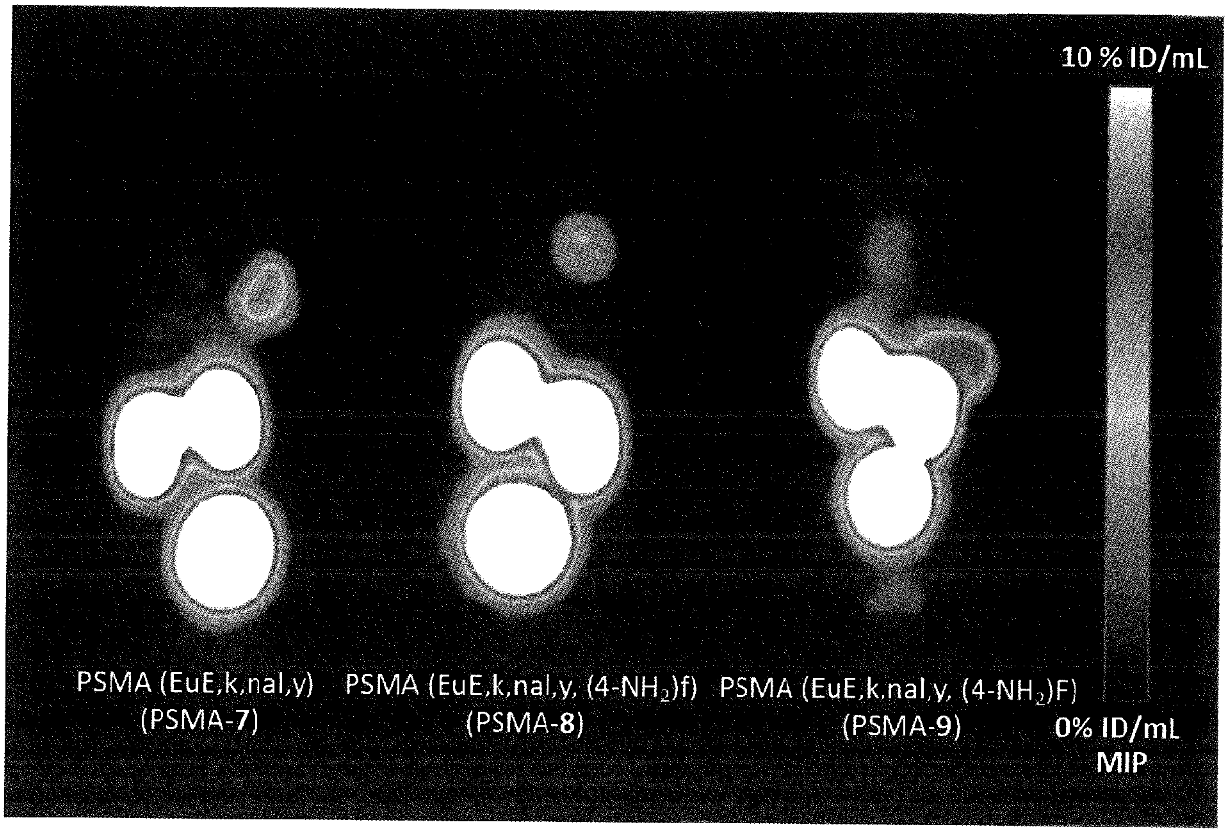
10 % ID/mL
PSMA (EuE,k,nal,y)
(PSMA-7)
PSMA (EuE,k,nal,y, (4-NH2)f)
(PSMA-8)
PSMA (EuE,k,nal,y, (4-NH2)F)
(PSMA-9)
0% ID/mL
MIP

Figure 6

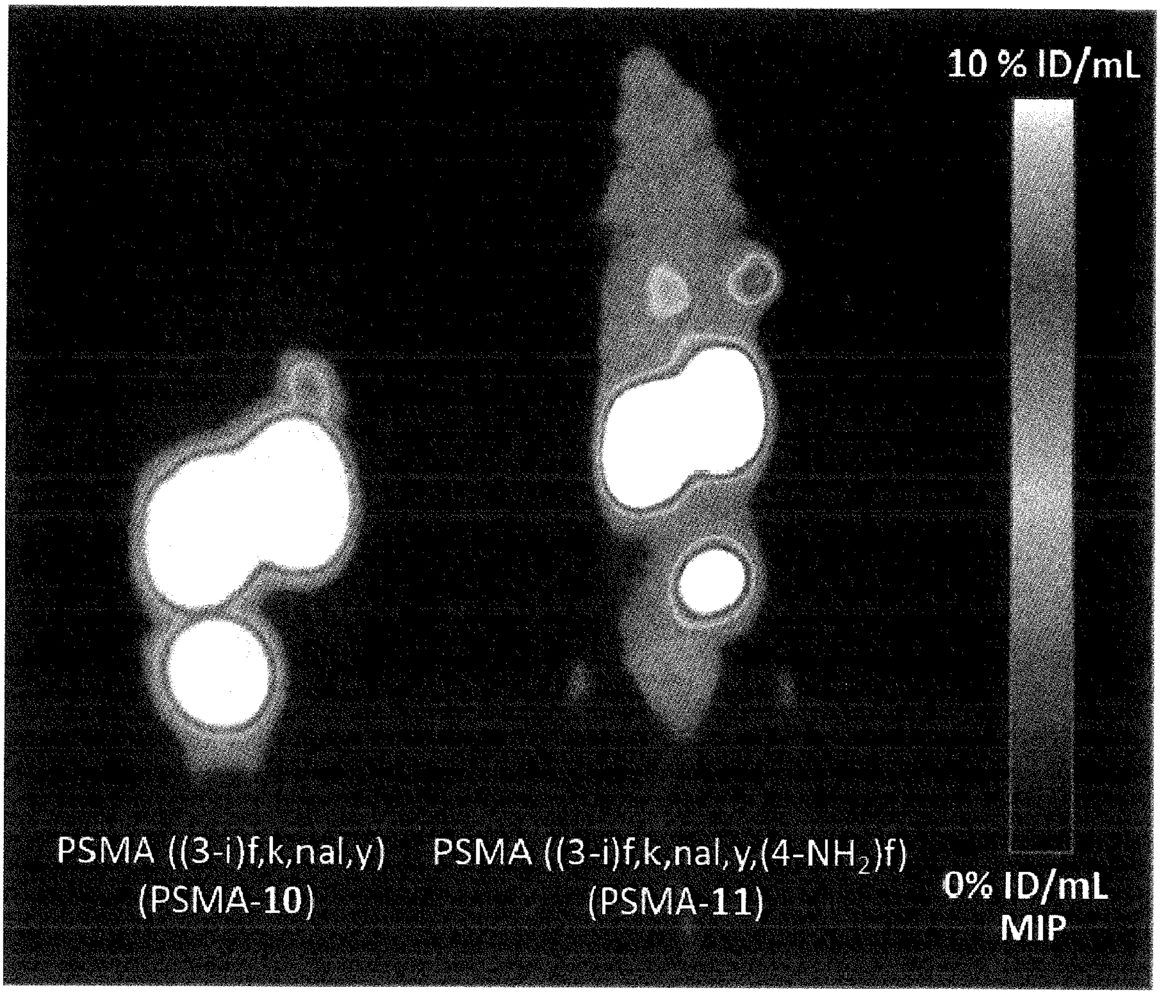
10 % ID/mL
PSMA ((3-i)f,k,nal,y)
(PSMA-10)
PSMA ((3-i)f,k,nal,y,(4-NH$_2$)f)
(PSMA-11)
0% ID/mL
MIP

Figure 7

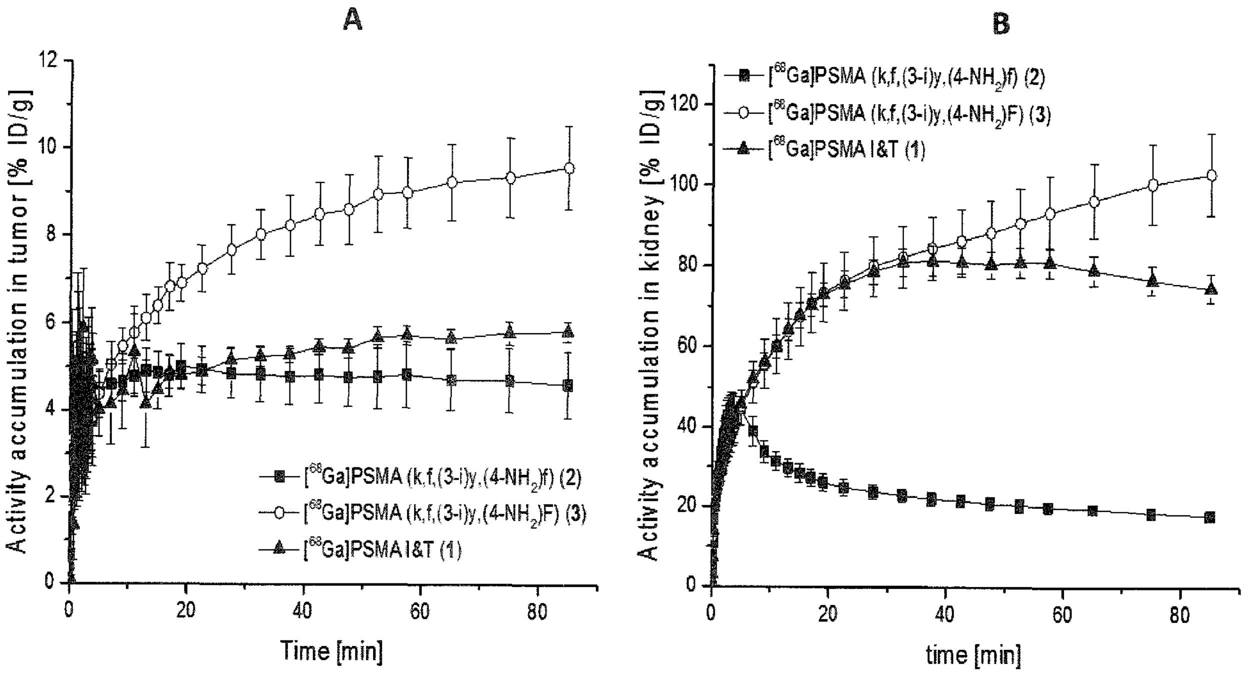
A
Activity accumulation in tumor [% ID/g]
Time [min]
[68Ga]PSMA (k,f,(3-i)y,(4-NH2)f) (2)
[68Ga]PSMA (k,f,(3-i)y,(4-NH2)F) (3)
[68Ga]PSMA I&T (1)
B
Activity accumulation in kidney [% ID/g]
time [min]
[68Ga]PSMA (k,f,(3-i)y,(4-NH2)f) (2)
[68Ga]PSMA (k,f,(3-i)y,(4-NH2)F) (3)
[68Ga]PSMA I&T (1)

Figure 8

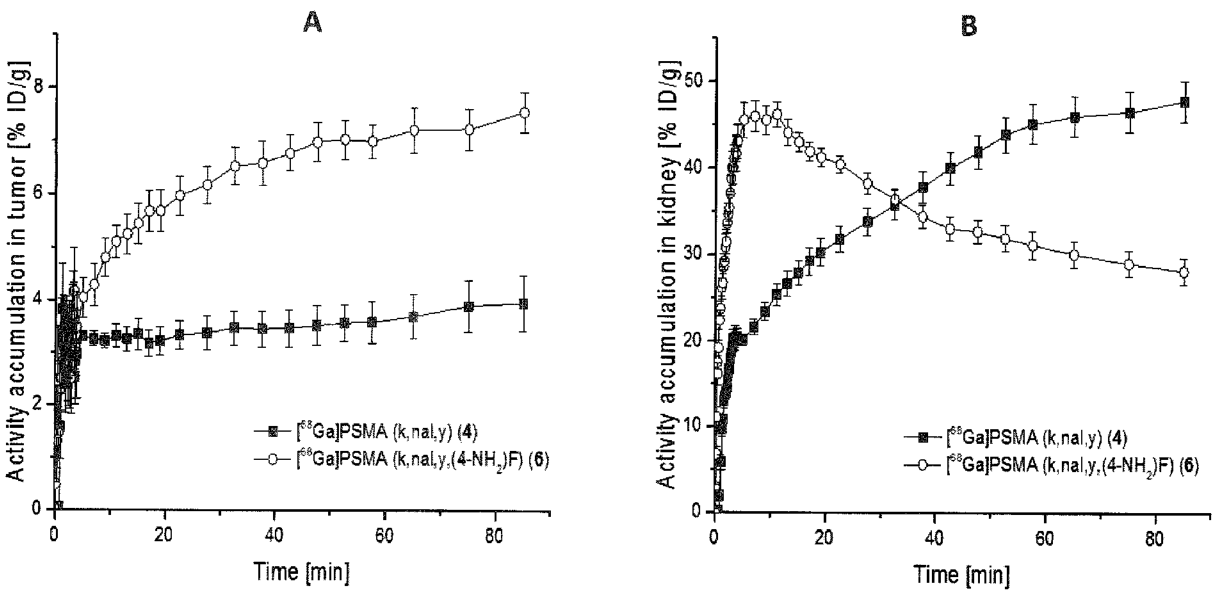
A
Activity accumulation in tumor [% ID/g]
Time [min]
[68Ga]PSMA (k,nal,y) (4)
[68Ga]PSMA (k,nal,y,(4-NH2)F) (6)
B
Activity accumulation in kidney [% ID/g]
Time [min]
[68Ga]PSMA (k,nal,y) (4)
[68Ga]PSMA (k,nal,y,(4-NH2)F) (6)

Figure 9

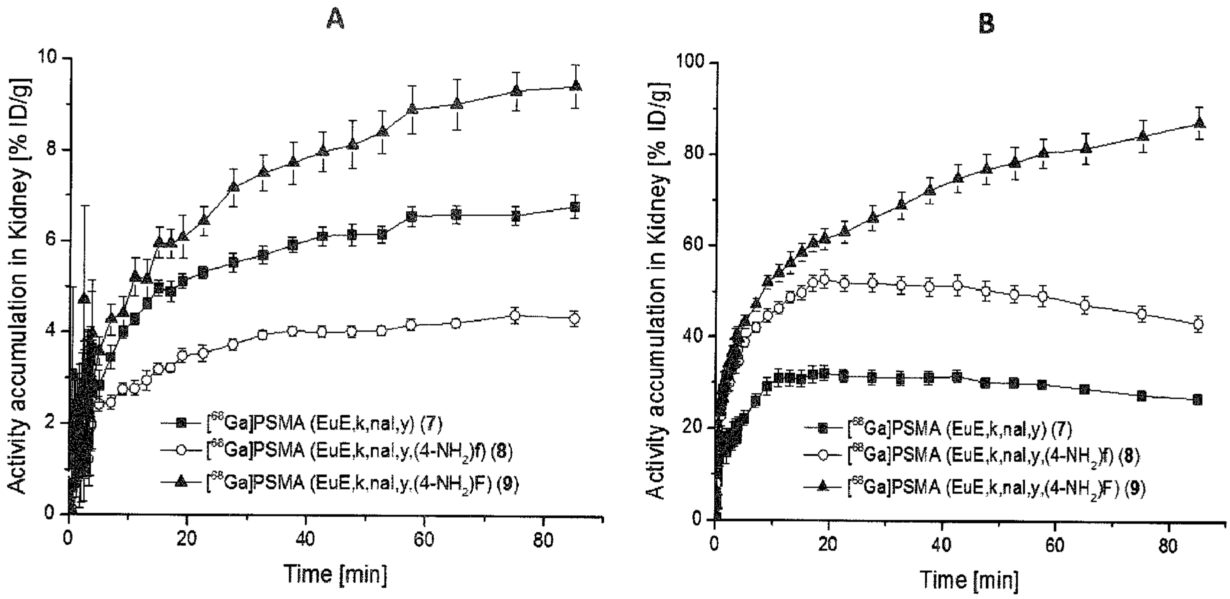
A
B
Activity accumulation in Kidney [% ID/g]
Time [min]
[68Ga]PSMA (EuE,k,nal,y) (7)
[68Ga]PSMA (EuE,k,nal,y,(4-NH2)f) (8)
[68Ga]PSMA (EuE,k,nal,y,(4-NH2)F) (9)

Figure 10

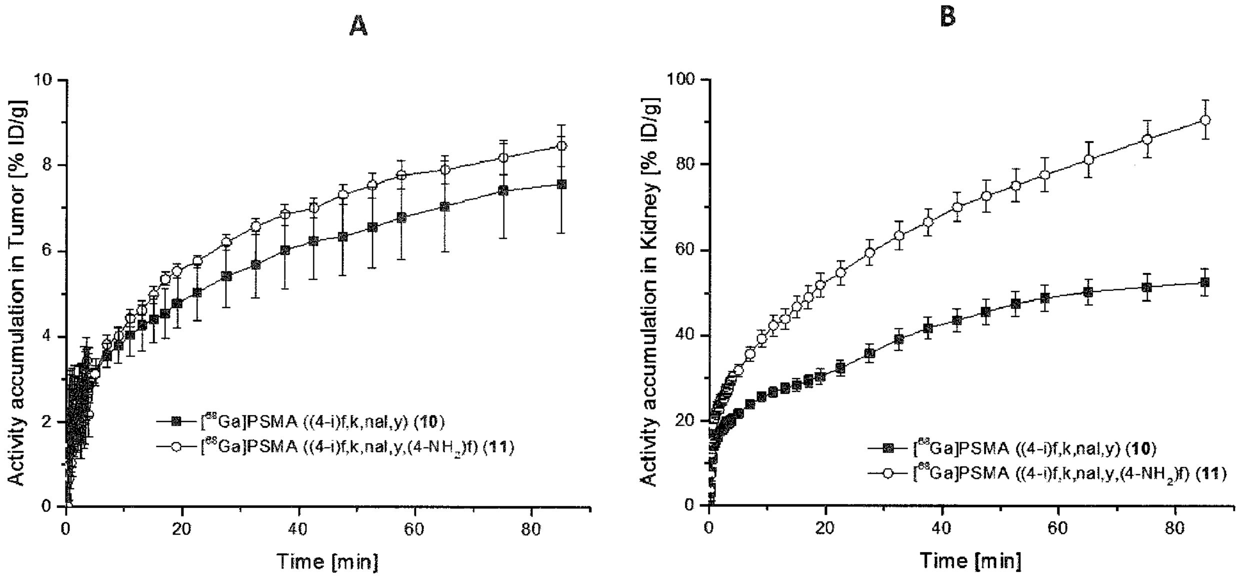
A
B
Activity accumulation in Tumor [% ID/g]
Activity accumulation in Kidney [% ID/g]
Time [min]
[$^{68}$Ga]PSMA ((4-i)f,k,nal,y) (10)
[$^{68}$Ga]PSMA ((4-i)f,k,nal,y,(4-NH$_2$)f) (11)

Figure 11

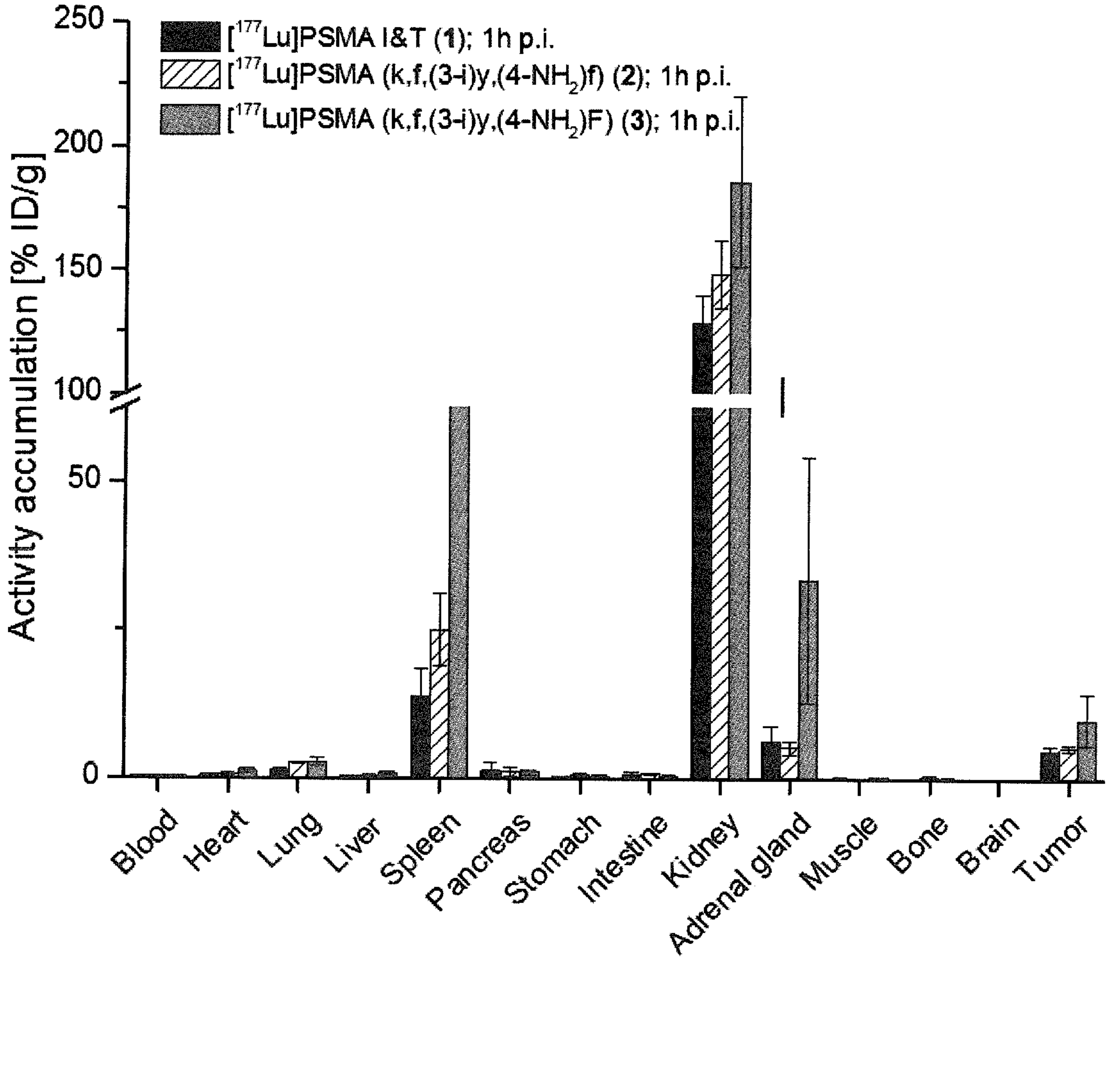
[177Lu]PSMA I&T (1); 1h p.i.
[177Lu]PSMA (k,f,(3-i)y,(4-NH2)f) (2); 1h p.i.
[177Lu]PSMA (k,f,(3-i)y,(4-NH2)F) (3); 1h p.i.
Activity accumulation [% ID/g]
250
200
150
100
50
0
Blood
Heart
Lung
Liver
Spleen
Pancreas
Stomach
Intestine
Kidney
Adrenal gland
Muscle
Bone
Brain
Tumor

**Figure 11 continued**

| | [$^{177}$Lu]PSMA I&T (1) | | [$^{177}$Lu]PSMA (k,f,(3-i)y,(4-$NH_2$)f) (2) | | [$^{177}$Lu]PSMA (k,f,(3-i)y,(4-$NH_2$)F) (3) | |
|---|---|---|---|---|---|---|
| Organ | [% ID/g] | SD | [% ID/g] | SD | [% ID/g] | SD |
| Blood | 0,37 | 0,10 | 0,36 | 0,04 | 0,35 | 0,14 |
| Heart | 0,58 | 0,15 | 0,74 | 0,26 | 1,31 | 0,30 |
| Lung | 1,32 | 0,45 | 2,64 | 0,18 | 2,84 | 0,71 |
| Liver | 0,37 | 0,10 | 0,48 | 0,04 | 0,88 | 0,35 |
| Spleen | 13,80 | 4,59 | 25,03 | 6,08 | 86,65 | 34,74 |
| Pancreas | 1,30 | 1,39 | 1,20 | 0,69 | 1,06 | 0,37 |
| Stomach | 0,29 | 0,06 | 0,63 | 0,19 | 0,49 | 0,06 |
| Intestine | 0,59 | 0,50 | 0,82 | 0,08 | 0,47 | 0,06 |
| Kidney | 128,90 | 10,74 | 148,44 | 13,61 | 185,84 | 34,57 |
| Adrenal gland | 6,25 | 2,59 | 5,23 | 1,20 | 33,51 | 20,62 |
| Muscle | 0,18 | 0,07 | 0,22 | 0,02 | 0,29 | 0,08 |
| Bone | 0,14 | 0,04 | 0,43 | 0,11 | 0,24 | 0,06 |
| Brain | 0,03 | 0,01 | 0,04 | 0,00 | 0,05 | 0,01 |
| Tumor | 4,69 | 0,95 | 5,24 | 0,48 | 10,08 | 4,32 |

**Figure 12**

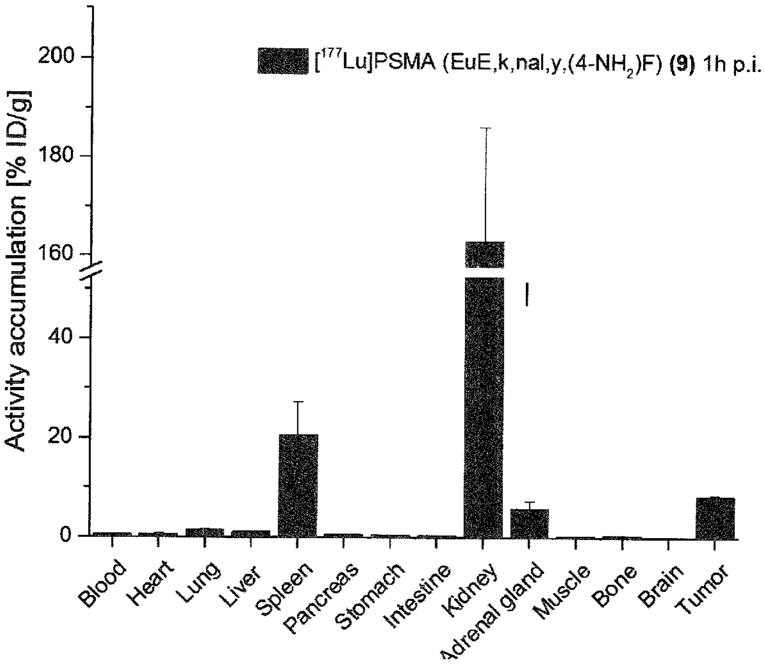


| | $[^{177}Lu]$PSMA (EuE,k,nal,y,(4-$NH_2$)F) (9) | |
|---|---|---|
| Organ | [% ID/g] | SD |
| Blood | 0,46 | 0,06 |
| Heart | 0,57 | 0,11 |
| Lung | 1,47 | 0,25 |
| Liver | 0,99 | 0,19 |
| Spleen | 20,53 | 6,79 |
| Pancreas | 0,56 | 0,07 |
| Stomach | 0,35 | 0,10 |
| Intestine | 0,30 | 0,08 |
| Kidney | 162,96 | 23,20 |
| Adrenal gland | 5,66 | 1,66 |
| Muscle | 0,17 | 0,02 |
| Bone | 0,29 | 0,14 |
| Brain | 0,03 | 0,00 |
| Tumor | 8,21 | 0,23 |

Figure 13

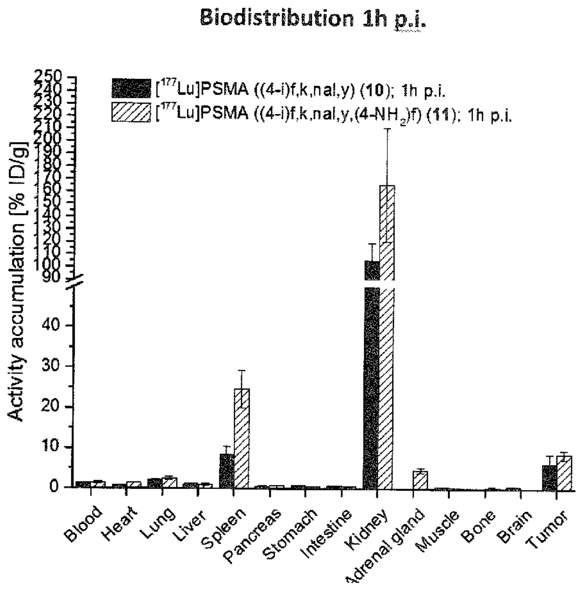
Biodistribution 1h p.i.
[177Lu]PSMA ((4-i)f,k,nal,y) (10); 1h p.i.
[177Lu]PSMA ((4-i)f,k,nal,y,(4-NH2)f) (11); 1h p.i.
Activity accumulation [% ID/g]
250
240
230
220
210
200
190
180
170
160
150
140
130
120
110
100
90
40
30
20
10
0
Blood
Heart
Lung
Liver
Spleen
Pancreas
Stomach
Intestine
Kidney
Adrenal gland
Muscle
Bone
Brain
Tumor

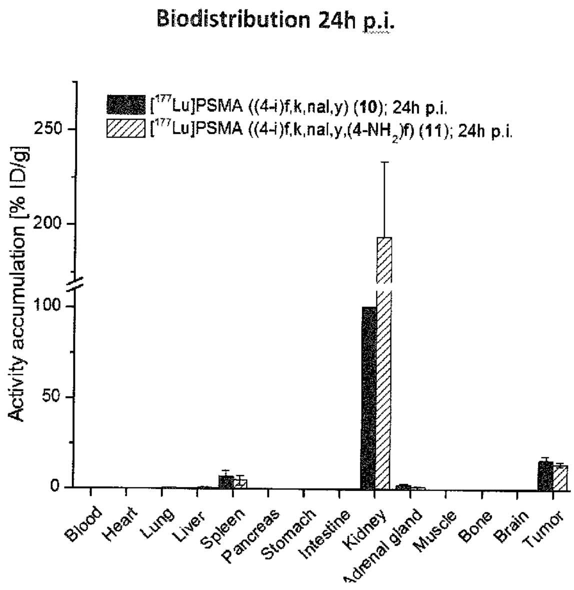
Biodistribution 24h p.i.
[177Lu]PSMA ((4-i)f,k,nal,y) (10); 24h p.i.
[177Lu]PSMA ((4-i)f,k,nal,y,(4-NH2)f) (11); 24h p.i.
Activity accumulation [% ID/g]
250
200
100
50
0
Blood
Heart
Lung
Liver
Spleen
Pancreas
Stomach
Intestine
Kidney
Adrenal gland
Muscle
Bone
Brain
Tumor

**Figure 14**

# Biodistribution 1h p.i.

| | $[^{177}Lu]$PSMA I&T (1) | | $[^{177}Lu]$PSMA PSMA (EuK,(3-i)f,k,nal,y) (10) | | $[^{177}Lu]$ PSMA (EuK,(3-i)f,k,nal,(4-$NH_2$)f) (11) | |
|---|---|---|---|---|---|---|
| Organ | [% ID/g] | SD | [% ID/g] | SD | [% ID/g] | SD |
| Blood | 0,37 | 0,10 | 1,29 | 0,14 | 1,34 | 0,12 |
| Heart | 0,58 | 0,15 | 0,65 | 0,13 | 1,30 | 0,04 |
| Lung | 1,32 | 0,45 | 2,06 | 0,23 | 2,46 | 0,41 |
| Liver | 0,37 | 0,10 | 1,08 | 0,13 | 0,97 | 0,20 |
| Spleen | 13,80 | 4,59 | 8,40 | 2,15 | 24,66 | 4,74 |
| Pancreas | 1,30 | 1,39 | 0,60 | 0,13 | 0,75 | 0,05 |
| Stomach | 0,29 | 0,06 | 0,62 | 0,06 | 0,51 | 0,10 |
| Intestine | 0,59 | 0,50 | 0,63 | 0,13 | 0,40 | 0,06 |
| Kidney | 128,90 | 10,74 | 104,82 | 14,34 | 165,86 | 45,49 |
| Adrenal gland | 6,25 | 2,59 | - | - | 4,54 | 0,70 |
| Muscle | 0,18 | 0,07 | 0,42 | 0,12 | 0,26 | 0,07 |
| Bone | 0,14 | 0,04 | 0,05 | 0,06 | 0,33 | 0,17 |
| Brain | 0,03 | 0,01 | 0,44 | 0,01 | 0,03 | 0,02 |
| Tumor | 4,69 | 0,95 | 6,29 | 2,22 | 8,51 | 1,10 |

**Figure 14 continued**

# Biodistribution 24h p.i.

| | [$^{177}$Lu]PSMA I&T (1) | | [$^{177}$Lu]PSMA PSMA (EuK,(3-i)f,k,nal,y) (10) | | [$^{177}$Lu] PSMA (EuK,(3-i)f,k,nal,(4-$NH_2$)f) (11) | |
|---|---|---|---|---|---|---|
| Organ | [% ID/g] | SD | [% ID/g] | SD | [% ID/g] | SD |
| Blood | 0,01 | 0,01 | 0,05 | 0,02 | 0,17 | 0,03 |
| Heart | 0,05 | 0,03 | 0,1 | 0,03 | 0,25 | 0,1 |
| Lung | 0,16 | 0,03 | 0,26 | 0,10 | 0,51 | 0,16 |
| Liver | 0,05 | 0,01 | 0,16 | 0,06 | 0,62 | 0,41 |
| Spleen | 1,94 | 1,01 | 6,63 | 3,25 | 4,71 | 2,45 |
| Pancreas | 0,05 | 0,03 | 0,20 | 0,11 | 0,16 | 0,06 |
| Stomach | 0,05 | 0,02 | 0,13 | 0,04 | 0,12 | 0,03 |
| Intestine | 0,12 | 0,07 | 0,14 | 0,06 | 0,12 | 0,03 |
| Kidney | 34,66 | 17,20 | 100,92 | 45,43 | 194,6 | 39,8 |
| Adrenal gland | 1,06 | 0,24 | 2,37 | 0,41 | 1,27 | 0,16 |
| Muscle | 0,01 | 0,00 | 0,07 | 0,04 | 0,06 | 0,02 |
| Bone | 0,01 | 0,00 | 0,03 | 0,01 | 0,06 | 0,02 |
| Brain | 0,02 | 0,00 | 0,11 | 0,03 | 0,03 | 0,02 |
| Tumor | 4,06 | 1,12 | 16,05 | 2,51 | 14,01 | 1,25 |

Europäisches Patentamt
European Patent Office
Office européen des brevets

# EUROPEAN SEARCH REPORT

Application Number
EP 17 20 6516

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/165473 A1 (UNIV JOHNS HOPKINS [US]) 28 September 2017 (2017-09-28)<br>* page 1, line 24 - page 3, line 10 *<br>----- | 1-15 | INV.<br>A61K51/04 |
| A | CINDY J CHOY ET AL: "177Lu-Labeled Phosphoramidate-Based PSMA Inhibitors: The Effect of an Albumin Binder on Biodistribution and Therapeutic Efficacy in Prostate Tumor-Bearing Mice", THERANOSTICS, IVYSPRING INTERNATIONAL PUBLISHER, AU, vol. 7, no. 7, 1 January 2017 (2017-01-01), pages 1928-1939, XP002778825, ISSN: 1838-7640, DOI: 10.7150/THNO.18719 [retrieved on 2017-04-27]<br>Scheme 1.;<br>* abstract *<br>----- | 1-15 | |

TECHNICAL FIELDS SEARCHED (IPC)

A61K

The present search report has been drawn up for all claims

2

| Place of search | Date of completion of the search | Examiner |
|---|---|---|
| The Hague | 5 June 2018 | Siebum, Bastiaan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 17 20 6516

This annex lists the patent family members relating to the patent documents cited in the above-mentioned European search report.
The members are as contained in the European Patent Office EDP file on
The European Patent Office is in no way liable for these particulars which are merely given for the purpose of information.

05-06-2018

| Patent document cited in search report | Publication date | Patent family member(s) | Publication date |
|---|---|---|---|
| WO 2017165473 A1 | 28-09-2017 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20070072831 A

### Non-patent literature cited in the description


- **D.A. et al.** Prostate-specific membrane antigen expression in normal and malignant human tissues. *Clinical Cancer Research,* 1997, vol. 3 (1), 81-85
- **AFSHAR-OROMIEH, A. et al.** The diagnostic value of PET/CT imaging with the 68Ga-labelled PSMA ligand HBED-CC in the diagnosis of recurrent prostate cancer. *European journal of nuclear medicine and molecular imaging,* 2015, vol. 42 (2), 197-209
- **BENEŠOVÁ, M. et al.** Preclinical Evaluation of a Tailor-Made DOTA-Conjugated PSMA Inhibitor with Optimized Linker Moiety for Imaging and Endoradiotherapy of Prostate Cancer. *Journal of Nuclear Medicine,* 2015, vol. 56 (6), 914-920
- **ROBU, S. et al.** Preclinical evaluation and first patient application of Tc-PSMA-I&S for SPECT imaging and radioguided surgery in prostate cancer. *Journal of Nuclear Medicine,* 2016, 116
- **WEINEISEN, M. et al.** Development and first in human evaluation of PSMA I&T-A ligand for diagnostic imaging and endoradiotherapy of prostate cancer. *Journal of Nuclear Medicine,* 2014, vol. 55 (1), 1083-1083
- **ROWE, S. et al.** PET imaging of prostate-specific membrane antigen in prostate cancer: current state of the art and future challenges. *Prostate cancer and prostatic diseases,* 2016
- **MAURER, T. et al.** Current use of PSMA-PET in prostate cancer management. *Nature Reviews Urology,* 2016
- **ZHOU, J. et al.** NAAG peptidase inhibitors and their potential for diagnosis and therapy. *Nature Reviews Drug Discovery,* 2005, vol. 4 (12), 1015-1026
- **MACHULKIN, A.E. et al.** Small-molecule PSMA ligands. Current state, SAR and perspectives. *Journal of drug targeting,* 2016, 1-15
- **BARINKA, C. et al.** Interactions between Human Glutamate Carboxypeptidase // and Urea-Based Inhibitors: Structural Characterization. *Journal of medicinal chemistry,* 2008, vol. 51 (24), 7737-7743
- **ZHANG, A.X. et al.** A remote arene-binding site on prostate specific membrane antigen revealed by antibody-recruiting small molecules. *Journal of the American Chemical Society,* 2010, vol. 132 (36), 12711-12716
- **LIU, T. et al.** Spacer length effects on in vitro imaging and surface accessibility of fluorescent inhibitors of prostate specific membrane antigen. *Bioorganic & medicinal chemistry letters,* 2011, vol. 21 (23), 7013-7016
- **KIESS, A.P. et al.** Prostate-specific membrane antigen as a target for cancer imaging and therapy. The quarterly journal of nuclear medicine and molecular imaging: official publication. *Italian Association of Nuclear Medicine (AIMN)[and] the International Association of Radiopharmacology,* 2015, vol. 59 (3), 241
- **EDER, M. et al.** Ga-complex lipophilicity and the targeting property of a urea-based PSMA inhibitor for PET imaging. *Bioconjugate chemistry,* 2012, vol. 23 (4), 688-697
- **ROWE, S.P. et al.** PSMA-Based [18F] DCFPyL PET/CT Is Superior to Conventional Imaging for Lesion Detection in Patients with Metastatic Prostate Cancer. *Molecular Imaging and Biology,* 2016, 1-9
- **DIETLEIN, M. et al.** Comparison of [18F] DCFPyL and [68Ga] Ga-PSMA-HBED-CC for PSMA-PET imaging in patients with relapsed prostate cancer. *Molecular Imaging and Biology,* 2015, vol. 17 (4), 575-584
- **BECKER, A. et al.** Nephro-and hepatotoxicity after radioligand therapy of metastatic castrate-resistant prostate cancer with Lu-PSMA-617. *Journal of Nuclear Medicine,* 2016, vol. 57 (2), 1430-1430
- **RAHBAR, K. et al.** Response and tolerability of a single dose of Lu-PSMA-617 in patients with metastatic castration-resistant prostate cancer: a multicenter retrospective analysis. *Journal of Nuclear Medicine,* 2016, 116
- **EIBER, M. et al.** Systemic radioligand therapy with Lu-PSMA I&T in patients with metastatic castration-resistant prostate cancer. *Journal of Nuclear Medicine,* 2016, vol. 57 (2), 61-61

- **SCHOTTELIUS, M. et al.** PSMA-I&T: expanding the spectrum of PSMA-I&T applications towards SPECT and radioguided surgery. *EJNMMI research,* 2015, vol. 5 (1), 1
- **ROBU, S. et al.** Preclinical evaluation and first patient application of mTc-PSMA-I&S for SPECT imaging and radioguided surgery in prostate cancer. *Journal of Nuclear Medicine,* 2016, 116
- **DELKER, A. et al.** Dosimetry for Lu-DKFZ-PSMA-617: a new radiopharmaceutical for the treatment of metastatic prostate cancer. *European journal of nuclear medicine and molecular imaging,* 2016, vol. 43 (1), 42-51
- **KABASAKAL, L. et al.** Pre-therapeutic dosimetry of normal organs and tissues of Lu-PSMA-617 prostate-specific membrane antigen (PSMA) inhibitor in patients with castration-resistant prostate cancer. *European journal of nuclear medicine and molecular imaging,* 2015, vol. 42 (13), 1976-1983
- **YADAV, M.P. et al.** Lu-DKFZ-PSMA-617 therapy in metastatic castration resistant prostate cancer: safety, efficacy, and quality of life assessment. *European Journal of Nuclear Medicine and Molecular Imaging,* 2016, 1-11
- **SILVER, D.A. et al.** Prostate-specific membrane antigen expression in normal and malignant human tissues. *Clinical Cancer Research,* 1997, vol. 3 (1), 81-85
- **S. M. BERGE et al.** Pharmaceutical Salts. *J. Pharm. Sci.,* 1977, vol. 66, 1-19
- **N. DAS et al.** *European Journal of Pharmaceutical Sciences,* 2010, vol. 41, 571-588
- **NING SHAN et al.** *Drug Discovery Today,* 2008, vol. 13 (9/10), 440-446
- **D. J. GOOD et al.** *Cryst. Growth Des.,* 2009, vol. 9 (5), 2252-2264
- **VAN ESSEN M ; KRENNING EP ; KAM BL ; DE HERDER WW ; VAN AKEN MO ; KWEKKEBOOM DJ.** Report on short-term side effects of treatments with 177Lu-octreotate in combination with capecitabine in seven patients with gastroenteropancreatic neuroendocrine tumours. *Eur J Nucl Med Mol Imaging,* 2008, vol. 35, 743-748
- **KONG G ; CALLAHAN J ; HOFMAN MS et al.** High clinical and morphologic response using 90Y-DOTA-octreotate sequenced with 177Lu-DOTA-octreotate induction peptide receptor chemoradionuclide therapy (PRCRT) for bulky neuroendocrine tumours. *Eur J Nucl Med Mol Imaging,* 2017, vol. 44, 476-489
- **ŠIMEČEK, J. et al.** A Monoreactive Bifunctional Triazacyclononane Phosphinate Chelator with High Selectivity for Gallium-68. *ChemMedChem,* 2012, vol. 7 (8), 1375-1378
- **WEINEISEN, M. et al.** Synthesis and preclinical evaluation of DOTAGA-conjugated PSMA ligands for functional imaging and endoradiotherapy of prostate cancer. *EJNMMI research,* 2014, vol. 4 (1), 1
- **WEINEISEN, M. et al.** 68Ga- and Lu-Labeled PSMA I&T: Optimization of a PSMA-Targeted Theranostic Concept and First Proof-of-Concept Human Studies. *Journal of Nuclear Medicine,* 2015, vol. 56 (8), 1169-1176
- **EDER, M. et al.** 68Ga-complex lipophilicity and the targeting property of a urea-based PSMA inhibitor for PET imaging. *Bioconjugate chemistry,* 2012, vol. 23 (4), 688-697
- **SOSABOWSKI, J.K. ; S.J. MATHER.** Conjugation of DOTA-like chelating agents to peptides and radiolabeling with trivalent metallic isotopes. *Nat. Protocols,* 2006, vol. 1 (2), 972-976
- **YAMAZAKI, K. ; M. KANAOKA.** Computational prediction of the plasma protein-binding percent of diverse pharmaceutical compounds. *Journal of pharmaceutical sciences,* 2004, vol. 93 (6), 1480-1494